# EUROPÄISCHE PATENTSCHRIFT

(11) **EP 1 697 372 B1**
(45) Veröffentlichungstag und Bekanntmachung des Hinweises auf die Patenterteilung: **19.12.2007**
(21) Anmeldenummer: 04820058.8
(22) Anmeldetag: 08.12.2004
(51) Int. Cl.: C07D 487/04, A01N 43/90

(54) **PYRAZOLOPYRIMIDINE**
PYRAZOLOPYRIMIDINES
PYRAZOLOPYRIMIDINES

(30) Priorität: 10.12.2003 DE 10357566
(43) Veröffentlichungstag der Anmeldung: 06.09.2006
(73) Patentinhaber: Bayer CropScience AG, 40789 Monheim (DE)
(72) Erfinder: GEBAUER, Olaf, 51377 Leverkusen (DE); HEINEMANN, Ulrich, 42799 Leichlingen (DE); HERRMANN, Stefan, 40764 Langenfeld (DE); GAYER, Herbert, 40789 Monheim am Rhein (DE); HILLEBRAND, Stefan, 41462 Neuss (DE); ELBE, Hans-Ludwig, 42329 Wuppertal (DE); EBBERT, Ronald, 90475 Nürnberg (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2004/013930
(87) Internationale Veröffentlichungsnummer: WO 2005/056555

(56) Entgegenhaltungen:
- FR-A- 2 794 745
- US-A- 5 965 561
- US-A1- 2002 049 318
- NOVINSON T ET AL: "Synthesis and antifungal properties of certain 7-alkylaminopyrazolo(1,5-a)pyrimidines" JOURNAL OF MEDICINAL CHEMISTRY, AMERICAN CHEMICAL SOCIETY, US, Bd. 20, Nr. 2, 1977, Seiten 296-299, XP002970049 ISSN: 0022-2623

## Beschreibung

Die Erfindung betrifft Pyrazolopyrimidine, mehrere Verfahren zu deren Herstellung und deren Verwendung zur Bekämpfung von unerwünschten Mikroorganismen.

Es ist bereits bekannt geworden, dass bestimmte Pyrazolopyrimidine fungizide Eigenschaften besitzen (vergleiche DE-A 3 130 633 oder FR-A 2 794 745).

Da sich aber die ökologischen und ökonomischen Anforderungen an moderne Fungizide laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen Vorteile gegenüber den bekannten aufweisen.

Es wurden nun neue Pyrazolopyrimidine der Formel 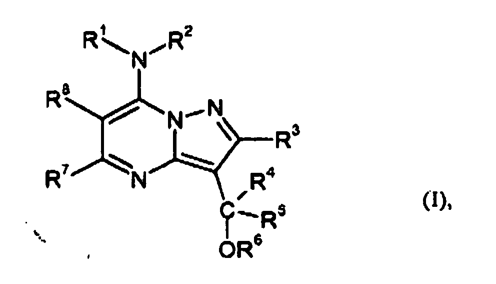 in welcher
- R¹: für Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
- R¹: für Alkenyl mit 2 bis 6 Kohlenstoffatomen steht, das einfach bis dreifach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
- R¹: für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und/oder Cycloalkyl mit 3 bis 6 Kohlenstoffatomen, oder
- R¹: für Cycloalkyl mit 3 bis 6 Kohlenstoffatomen steht, das einfach bis dreifach, gleichartig oder verschieden substituiert sein kann durch Halogen und/oder Alkyl mit 1 bis 4 Kohlenstoffatomen, oder
- R¹: für gesättigtes oder ungesättigtes Heterocyclyl mit 5 oder 6 Ringgliedern und 1 bis 3 Heteroatomen, wie Stickstoff, Sauerstoff und/oder Schwefel, steht, wobei das Heterocyclyl einfach oder zweifach substituiert sein kann durch Halogen, Alkyl mit 1 bis 4 Kohlenstoffatomen, Cyano, Nitro und/oder Cycloalkyl mit 3 bis 6 Kohlenstoffatomen,
- R²: für Wasserstoff oder Alkyl mit 1 bis 4 Kohlenstoffatomen steht, oder
- R¹ und R²: gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
- R³: für Wasserstoff, Fluor, Chlor, Brom, Iod, Alkyl mit 1 bis 4 Kohlenstoffatomen, Halogenalkyl mit 1 bis 4 Kohlenstoffatomen und 1 bis 9 Halogenatomen oder für Cycloalkyl mit 3 bis 6 Kohlenstoffatomen steht,
- R⁴: für Wasserstoff, Alkyl mit 1 bis 4 Kohlenstoffatomen, Halogenalkyl mit 1 bis 4 Kohlenstoffatomen im Alkylteil, Cycloalkyl mit 3 bis 6 Kohlenstoffatomen, Alkoxyalkyl mit 1 oder 2 Kohlenstofthtomen im Alkoxyteil und 1 bis 4 Kohlenstoffatomen im Alkylteil, Alkenyl mit 2 bis 5 Kohlenstoffatomen, Alkinyl mit 2 bis 5 Kohlenstoffatomen oder Benzyl steht,
- R⁵: für Wasserstoff, Alkyl mit 1 bis 4 Kohlenstoffatomen, Halogenalkyl mit 1 bis 4 Kohlenstoffatomen im Alkylteil, Cycloalkyl mit 3 bis 6 Kohlenstoffatomen, Alkoxyalkyl mit 1 oder 2 Kohlenstoffatomen im Alkoxyteil und 1 bis 4 Kohlenstoffatomen im Alkylteil. Alkenyl mit 2 bis 5 Kohlenstoffatomen, Alkinyl mit 2 bis 5 Kohlenstoffatomen oder Benzyl steht,

- R⁶: für Wasserstoff, Alkyl mit 1 bis 4 Kohlenstoffatomen, Alkoxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen, Alkinyl mit 2 bis 5 Kohlenstoffatomen oder Benzyl steht, oder
- R⁵ und -OR⁶: gemeinsam für einen Rest der Formel $- O - {\left({CH}_{2}\right)}_{p} - O -$ stehen,
worin
- p: für 2, 3 oder 4 steht und
1 oder 2 Wasserstoffatome ersetzt sein können durch Methyl, Ethyl, Hydroxy, Methoxy, Ethoxy, Hydroxymethyl, Methoxymethyl oder Ethoxymethyl.
- R⁷: für Fluor, Chlor, Brom, CN, Methyl, Alkoxy mit 1 bis 4 Kohlenstoffatomen, Alkylthio mit 1 bis 4 Kohlenstoffatomen, Alkylsulfinyl mit 1 bis 4 Kohlenstoftätomen oder Alkylsulfonyl mit 1 bis 4 Kohlenstoffatomen steht, und
- R⁸: für Phenyl steht, das einfach bis vierfach, gleichartig oder verschieden substituiert sein kann durch Halogen, Cyclo, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyl mit jeweils 2 bis 6 Kohlenstoffatomen;
jeweils geradkettiges oder verzweigtes Halogenalkyl, Halogenalkoxy, Halogenalkylthio, I-Ialogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkytsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach bis mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen,
gefunden.
Weiterhin wurde gefunden, dass sich Pyrazolopyrimidine der Formel (I) herstellen lassen, indem man
a) Pyrazolopyrimidine der Formel 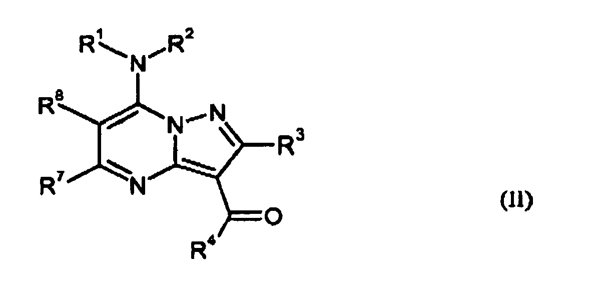 in welcher
   R¹, R², R³, R⁴, R⁷ und R⁸ die oben angegebenen Bedeutungen haben,
   entweder
   α) mit Diisobutyl-aluminiumhydrid in Gegenwart von wässriger Ammoniumchlorid-Lösung sowie in Gegenwart eines organischen Verdünnungsmittels umsetzt,
      oder mit Natriumborhydrid in Gegenwart eines Verdünnungsmittels umsetzt,
      oder
   β) mit Grignard-Verbindungen der Formel

      R⁹- Mg-X (III)

      in welcher
      - R⁹: für Alkyl, Alkoxyalkyl, Alkenyl, Alkinyl oder Benzyl steht und
      - X: für Chlor, Brom oder lod steht,
      in Gegenwart eines Katalysators und in Gegenwart eines Verdünnungsmittels umsetzt,
      und gegebenenfalls die nach der Variante (α) oder (β) erhaltenen Pyrazolopyrimidine der Formel 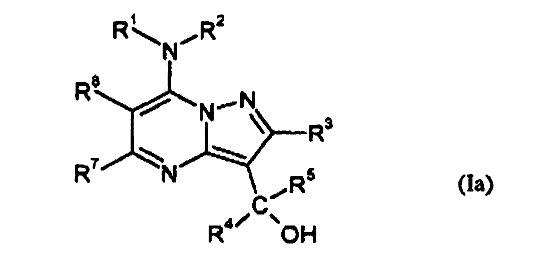
   in welcher
   R¹, R², R³, R⁴, R⁵, R⁷ und R⁸ die oben angegebenen Bedeutungen haben,
   mit Verbindungen der Formel

   R¹⁰-X¹ (IV)

   in welcher
   - R¹⁰: für Alkyl, Cycloalkyl, Alkoxyalkyl, Alkenyl, Alkinyl oder Benzyl, jeweils gegebenenfalls substituiert, steht und
   - X¹: für Chlor, Brom, lod oder den Rest R¹⁰O-SO₂-O- steht,
   gegebenenfalls in Gegenwart einer Base und gegebenenfalls in Gegenwart eines Verdünnungsmittels umsetzt,
   oder
b) Pyrazolopyrimidine der Formel 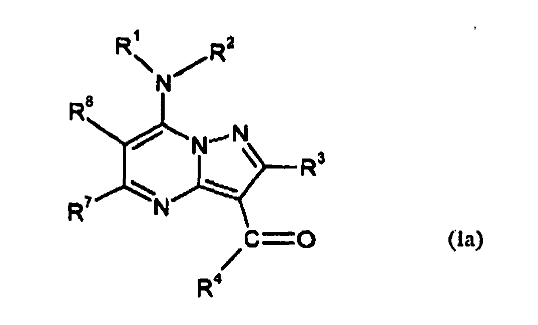 in welcher
   R¹, R², R³, R⁴, R⁷ und R⁸ die oben angegebenen Bedeutungen haben,
   mit Diolen der Formel

   HO-(CH₂)ₚ-OH (V)

   in welcher
   - p: für ganzen Zahlen von 1 bis 5 steht und
   - 1 bis 3: Wasserstoffatome ersetzt sein können durch Methyl, Ethyl, Hydroxy, Methoxy, Ethoxy, Hydroxymethyl, Methoxymethyl oder Ethoxymethyl,
   in Gegenwart eines Katalysators und gegebenenfalls in Gegenwart eines Verdünnungsmittels umsetzt.

Schließlich wurde gefunden, dass sich die Pyrazolopyrimidine der Formel (I) sehr gut zur Bekämpfung von unerwünschten Mikroorganismen eignen. Sie zeigen vor allem eine starke fungizide Wirksamkeit und lassen sich sowohl im Pflanzenschutz als auch im Materialschutz verwenden.

Die erfindungsgemäßen Verbindungen können je nach Substitutionsmuster gegebenenfalls als Mischungen verschiedener möglicher isomerer Formen, insbesondere von Stereoisomeren, wie E- und Z-, threo- und erythro-, sowie optischen Isomeren, gegebenenfalls aber auch in Form von Tautomeren vorliegen. Ist R⁸ an beiden Atomen, die der Bindungsstelle benachbart sind, ungleich substituiert, so können die betreffenden Verbindungen in einer besonderen Form der Stereoisomerie vorliegen, und zwar als Atropisomere.

Die erfindungsgemäßen Pyrazolopyrimidine sind durch die Formel (1) allgemein definiert. Bevorzugt sind diejenigen Pyrazolopyrimidine der Formel (I), in denen
- R¹: für einen Rest der Formel 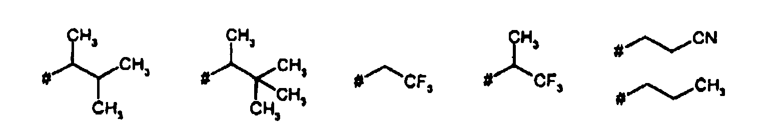 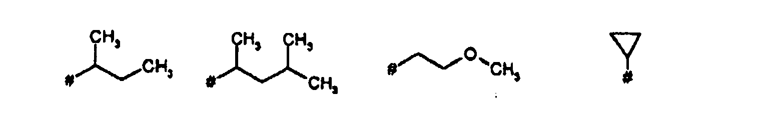 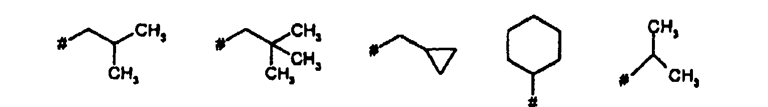 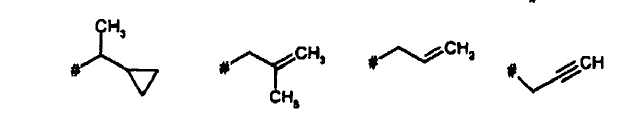 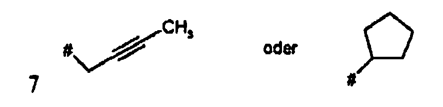 steht,
wobei # die Anknüpfungsstelle markiert,
R² für Wasserstoff, Methyl, Ethyl oder Propyl steht, oder
R¹ und R² gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl oder Tetrahydro-1 (2H)-pyridazinyl stehen, wobei diese Reste durch 1 bis 3 Fluoratome, 1 bis 3 Methylgruppen und/oder Trifluormethyl substituiert sein können,
oder
R¹ und R² gemeinsam mit dem Stickstoffatom, an das sie gebunden sind, für einen Rest der Formel 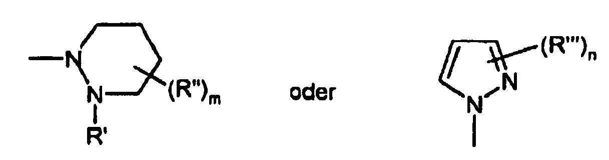 stehen
worin
R' für Wasserstoff oder Methyl steht,
R" für Methyl, Ethyl, Fluor, Chlor oder Trifluormethyl steht,
m für die Zahlen 0, 1, 2 oder 3 steht, wobei R" für gleiche oder verschiedene Reste steht, wenn m für 2 oder 3 steht,
R'" für Methyl, Ethyl, Fluor, Chlor oder Trifluormethyl steht
und
n für die Zahlen 0, 1, 2 oder 3 steht, wobei R'" für gleiche oder verschiedene Reste steht, wenn n für 2 oder 3 steht,
- R³: für Wasserstoff, Fluor, Chlor, Brom, Iod, Methyl, Ethyl, Isopropyl, Cyclopropyl, Cyclobutyl, Cyclopentyl, Cyclohexyl, Trifluormethyl, 1-Trifluormethyl-2,2,2-trifluorethyl oder Heptafluorisopropyl steht,
- R⁴: für Wasserstoff, Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder Benzyl steht,
- R⁵: für Wasserstoff, Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder Benzyl steht,
- R⁶: Für Wasserstoff, Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder Benzyl steht, oder
- R⁵ und -OR⁶: gemeinsam für einen Rest der Formel -O-CH₂-CH₂-O- stehen, worin 1 oder 2 Wasserstoffatome ersetzt sein können durch Methyl, Ethyl, Hydroxy, Methoxy, Ethoxy, Hydroxymethyl, Methoxymethyl oder Ethoxymethyl.
- R⁷: für Fluor, Chlor, Brom, CN, Methyl, Methoxy, Ethoxy, Methylthio, Methylsulfinyl oder Methylsulfonyl steht, und
- R⁸: für Phenyl steht, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t- Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino,
Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (O-CH₂-CH₂-O), wobei diese Reste einfach oder mehrfach, gleichartig oder verschieden substituiert sein können durch Fluor, Chlor, Methyl, Ethyl, n-Propyl, i-Propyl und/oder Trifluormethyl.

Eine besonders bevorzugte Gruppe erfindungsgemäßer Verbindungen sind Pyrazolopyrimidine der Formel (1), in denen
- R¹, R², R³, R⁴, R⁵ und R⁶: die zuvor angegebenen besonders bevorzugten Bedeutungen haben,
- R⁷: für Fluor, Chlor, Brom, CN, Methyl, Methoxy oder Methylthio steht und
- R⁸: für 2,4-, 2,5- oder 2,6-disubstituiertes Phenyl, oder 2-substituiertes Phenyl oder für 2,4,6-trisubstituiertes Phenyl steht, wobei als Substituenten diejenigen Reste in Frage kommen, die im Rahmen der Aufzählung der besonders bevorzugten Definitionen genannt wurden.

Die zuvor genannten Reste-Definitionen können untereinander in beliebiger Weise kombiniert werden. Außerdem können einzelne Definitionen entfallen.

Verwendet man 3-Formyl-5-chlor-6-(2-chlor-4-fluor-phenyl)-7-(4-methyl-piperidino)-pyrazolo-[1,5-a]pyrimidin als Ausgangsstoff und Natrium-borhydirid als Reaktionskomponente, so kann der Verlauf des erfindungsgemäßen Verfahrens (a, Variante α) durch das folgende Formelschema veranschaulicht werden.

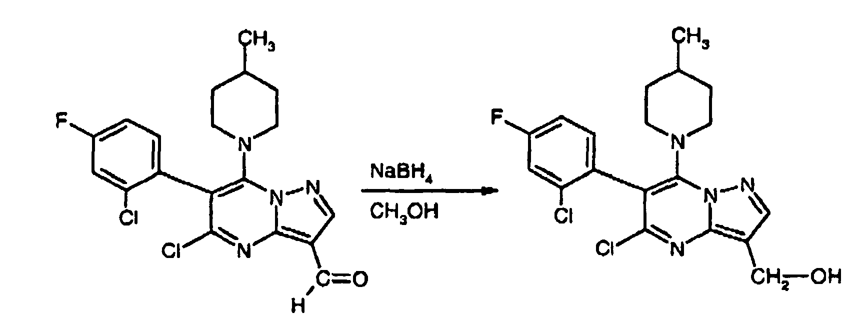

Verwendet man 3-Methylcarbonyl-5-chlor-6-(2-chlor-fluor-phenyl)-7-(4-methyl-piperidino)-pyrazolo[1,5-a]pyrimidin als Ausgangsstoff und Methyl-magnesium-bromid als Reaktionskomponente, so kann der Verlauf des erfindungsgemäßen Verfahrens (a, Variante β) durch das folgende Formelschema veranschaulicht werden.

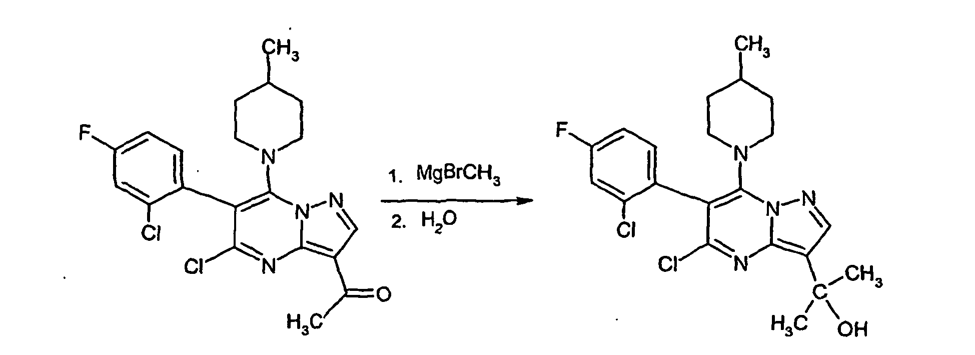

Verwendet man 3-Hydroxymethyl-5-chlor-6-(2-chlor-4-fluor-phenyl)-7-(2,2,2-trifluor-isopropylamino)-pyrazolo[1,5-a]pyrimidin als Ausgangsstoff und Methyliodid als Reaktionskomponente, so kann der Verlauf der zweiten Stufe des erfindungsgemäßen Verfahrens (a) durch das folgende Formelschema veranschaulicht werden.

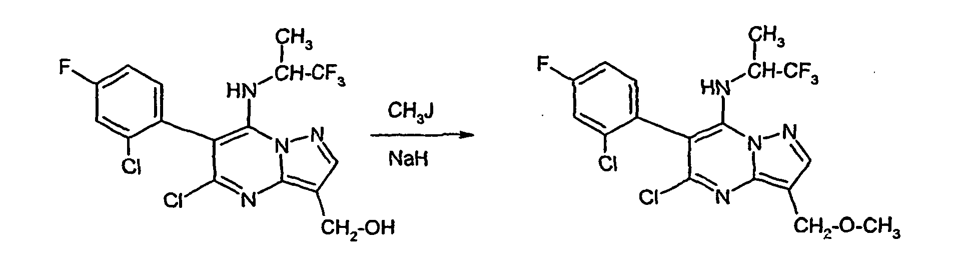

Verwendet man 3-Formyl-5-chlor-6-(2-chlor-4-fluor-phenyl)-7-(3,3-dimethyl-but-2-yl-amino)-pyrazolo[1,5-a]pyrimidin als Ausgangsstoff und Butan-1,2-diol als Reaktionskomponente, so kann der Verlauf des erfindungsgemäßen Verfahrens (b) durch das folgende Formelschema veranschaulicht werden.

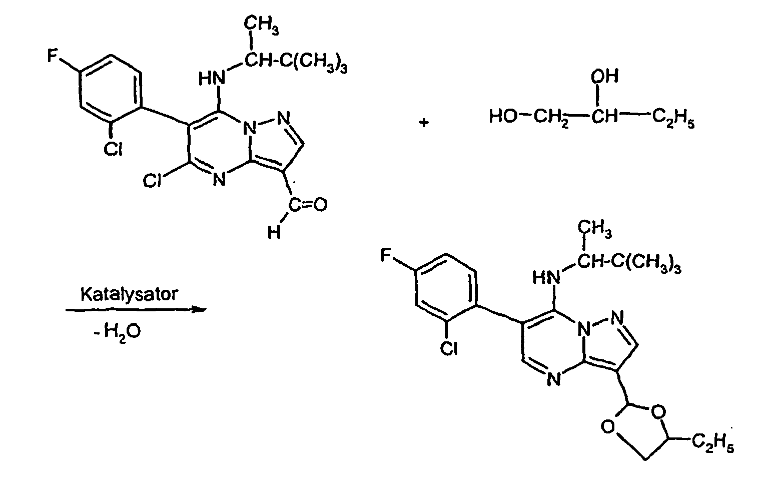

Die zur Durchführung des erfindungsgemäßen Verfahrens (a) als Ausgangsstoffe benötigten Pyrazolopyrimidine sind durch die Formel (II) allgemein definiert. In dieser Formel haben R¹, R², R³, R⁴, R⁷ und R⁸ vorzugsweise diejenigen Bedeutungen, die bereits im Zusammenhang mit der Beschreibung der erfindungsgemäßen Verbindungen der Formel (I) für diese Reste als bevorzugt genannt wurden.

Die Pyrazolopyrimidine der Formel (II) lassen sich herstellen, indem man
c) Pyrazolopyrimidin-Derivate der Formel 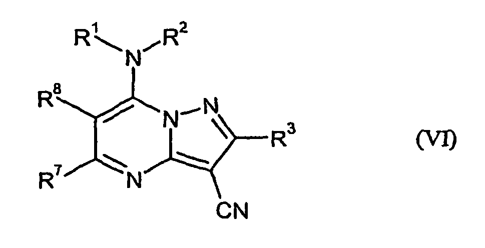 in welcher
   R¹, R², R³, R⁷ und R⁸ die oben angegebenen Bedeutungen haben,
   entweder
   α) mit Diisobutyl-aluminiumhydrid in Gegenwart von wässriger Ammonium-Lösung sowie in Gegenwart eines organischen Verdünnungsmittels umsetzt,
      oder
   β) mit Grignard-Verbindungen der Formel

      R⁹-Mg-X (III)

      in welcher
      R⁹ und X die oben angegebenen Bedeutungen haben,
      in Gegenwart eines Verdünnungsmittels und gegebenenfalls in Gegenwart eines Katalysators umsetzt,
      oder
   d) Pyrazolopyrimidine der Formel 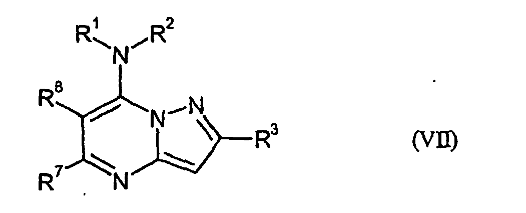 in welcher
      R¹, R², R³, R⁷ und R⁸ die oben angegebenen Bedeutungen haben,
      mit. Säurebalogeniden der Formel 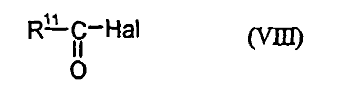 in welcher
      - R¹¹: für Alkyl, Alkoxyalkyl, Alkenyl, Alkiny oder Benzyl steht und
      - Hal: für Chlor oder Brom steht,
      mit Säureanhydriden oder anderen aktivierten Carbonsäureserivaten der Formel

      R¹² - COX¹ (IX)

      in welcher
      - R¹²: für Alkyl, Alkoxyalkyl, Alkenyl, Alkiny oder Benzyl steht und
      - X¹: für O-CO-R¹² oder einen Rest der Formel 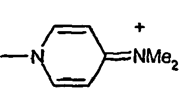 steht,
      jeweils in Gegenwart eines Katalysators und in Gegenwart eines Verdünnungsmittels umsetzt,
      oder
   e) Hydroxy-pyrazolopyrimidine der Formel 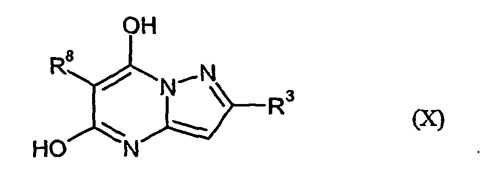 in welcher
      R³ und R⁸ die oben angegebenen Bedeutungen haben,
      mit Phosphoroxychlorid in Gegenwart von Dimethylformamid umsetzt und gegebenenfalls unter Zugabe von Phosphorpentachlorid nachreagieren lässt, und die dabei entstehenden Halogeno-pyrazolopyrimidine der Formel 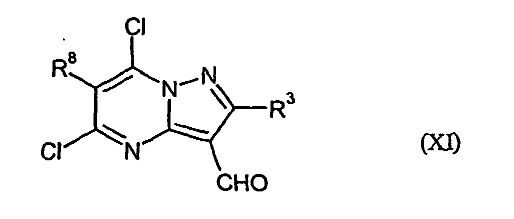 in welcher
      R³ und R⁸ die oben angegebenen Bedeutungen haben,
      mit Aminen der Formel 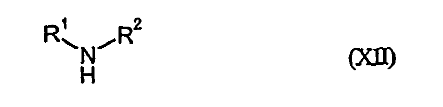 in welcher
      R¹ und R² die oben angegebenen Bedeutungen haben,
      gegebenenfalls in Gegenwart eines Katalysators, gegebenenfalls in Gegenwart eines Säurebindemittels und gegebenenfalls in Gegenwart eines Verdünnungsmittels.

Die bei der Durchführung des Verfahrens (c) als Ausgangsstoffe benötigten Pyrazolopyrimidin-Derivate der Formel (VI) lassen sich herstellen, indem man
f) Halogen-pyrazolopyrimidine der Formel 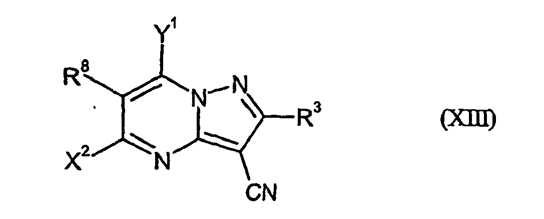 in welcher
   - R³ und R⁸: die oben angegebenen Bedeutungen haben,
   - X²: für Halogen steht und
   - Y¹: für Halogen steht,
   mit Aminen der Formel 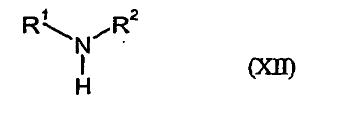 in welcher
   R¹ und R² die oben angegebenen Bedeutungen haben,
   gegebenenfalls in Gegenwart eines Verdünnungsmittels, gegebenenfalls in Gegenwart eines Katalysators und gegebenenfalls in Gegenwart eines Säureakzeptors, umsetzt,
   und gegebenenfalls in einem zweiten Schritt die so erhaltenen Cyano-Verbindungen der Formel 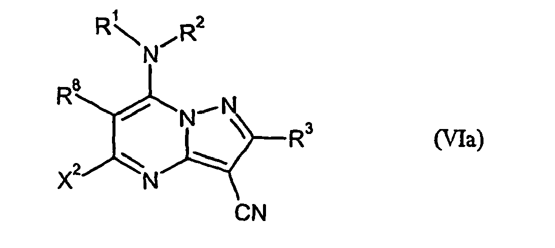 in welcher
   R¹, R², R³, R⁸ und X² die oben angegebenen Bedeutungen haben,
   mit Verbindungen der Formel

   R¹³-Me (XIV)

   in welcher
   - R¹³: für gegebenenfalls substituiertes Alkoxy, gegebenenfalls substituiertes Alkylthio, gegebenenfalls substituiertes Alkylsulfinyl oder gegebenenfalls substituiertes Alkylsulfonyl steht und
   - Me: für Natrium oder Kalium steht,
   gegebenenfalls in Gegenwart eines Verdünnungsmittels umsetzt.

Die Halogen-pyrazolopyrimidine der Formel (XIII) sind bekannt oder lassen sich nach bekannten Methoden herstellen (vgl. DE-A 103 28 996 und PCT/EP 03/05159).

So erhält man Halogen-pyrazolo-pyrimidine der Formel (XIII), indem man
g) Dihydroxy-pyrazolopyrimidine der Formel 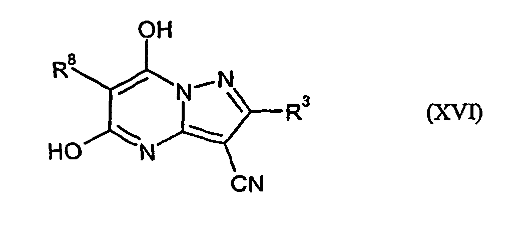 in welcher
   R³ und R⁸ die oben angegebenen Bedeutungen haben,
   mit Halogenierungsmitteln gegebenenfalls in Gegenwart eines Verdünnungsmittels umsetzt.

Die Dihydroxy-pyrazolopyrimidine der Formel (XV) lassen sich herstellen, indem man
(h) Aryl-malonester der Formel 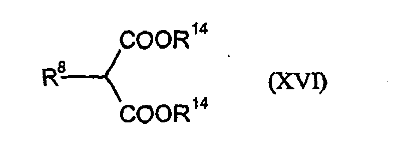 in welcher
   - R⁸: die oben angegebene Bedeutung hat und
   - R¹⁴: für Alkyl steht,
   mit Aminopyrazolen der Formel 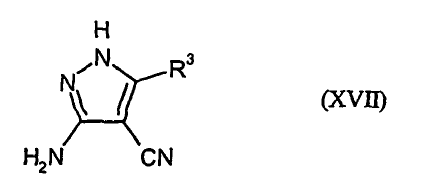 in welcher
   - R³: die oben angegebenen Bedeutungen hat,
   gegebenenfalls in Gegenwart eines Verdünnungsmittels und gegebenenfalls in Gegenwart einer Base umsetzt.

Die bei der Durchführung des Verfahrens (h) als Ausgangsstoffe benötigten Aryl-malonester sind durch die Formel (XVI) allgemein definiert. In dieser Formel hat R⁸ vorzugsweise diejenigen Bedeutungen, die bereits im Zusammenhang mit der Beschreibung der erfindungsgemäßen Stoffe der Formel (I) für diesen Rest als bevorzugt genannt wurden. R¹⁴ steht vorzugsweise für Alkyl mit 1 bis 4 Kohlenstoffen, besonders bevorzugt für Methyl oder Ethyl.

Die Aryl-malonester der Formel (XVI) sind bekannt oder lassen sich nach bekannten Methoden herstellen (vgl. US-A 6 156 925).

Die Aminopyrazole der Formel (XVII) sind ebenfalls bekannt oder lassen sich nach bekannten Methoden herstellen.

Als Verdünnungsmittel kommen bei der Durchführung des Verfahrens (h) alle üblichen, inerten organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; Amine, wie Tri-n-butylamin oder Carbonsäuren, wie Essigsäure.

Als starke Basen kommen bei der Durchführung des Verfahrens (h) vorzugsweise Erdalkalimetall- oder Alkalimetall-hydride oder -alkoholate sowie Alkalimetallamide in Frage. Beispielhaft genannt seien Natriumhydrid, Natriumamid, Natrium-methylat, Natrium-ethylat und Kalium-tert.-butylat.

Bei der Durchführung des Verfahrens (h) sowie auch bei der Durchführung der anderen in dieser Patentanmeldung beschriebenen Verfahren arbeitet man im Allgemeinen unter Atmosphärendruck. Es ist aber auch möglich, unter erhöhtem Druck oder, -sofern keine leicht flüchtigen Reaktionskomponenten enthalten sind-, unter vermindertem Druck zu arbeiten.

Die Reaktionstemperaturen können bei der Durchführung des Verfahrens (h) jeweils innerhalb eines größeren Bereiches variiert werden. Bei Abwesenheit von Basen arbeitet man im Allgemeinen bei Temperaturen zwischen 100°C und 250°C, vorzugsweise zwischen 120°C und 200°C. Bei Anwesenheit von Basen arbeitet man im Allgemeinen bei Temperaturen zwischen 20°C und 120°C, vorzugsweise zwischen 20°C und 80°C.

Bei der Durchführung des Verfahrens (h) setzt man auf 1 Mol an Aryl-malonester der Formel (XVI) im Allgemeinen 1 bis 15 Mol, vorzugsweise 1 bis 8 Mol an Aminopyrazol der Formel (XVII) ein. Die Aufarbeitung erfolgt nach üblichen Methoden.

Als Halogenierungsmittel kommen bei der Durchführung des Verfahrens (g) alle üblichen Reagenzien in Betracht, die für einen Austausch von an Kohlenstoff gebundene Hydroxygruppen gegen Halogen geeignet sind. Vorzugsweise verwendbar sind Phosphortrichlorid, Phosphortribromid, Phosphorpentachlorid, Phosphoroxychlorid, Phosgen, Thionylchlorid, Thionylbromid oder deren Gemische. Die entsprechenden Fluor-Verbindungen der Formel (XIII) lassen sich aus den Chlor- oder Brom-Verbindungen durch Umsetzung mit Kaliumfluorid herstellen.

Als Verdünnungsmittel kommen bei der Durchführung des Verfahrens (g) alle für derartige Halogenierungen üblichen organischen Solventien in Frage. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan.

Als Verdünnungsmittel kann aber auch das Halogenierungsmittel selbst oder ein Gemisch aus Halogenierungsmittel und einem der genannten Verdünnungsmittel dienen.

Die Reaktionstemperaturen können bei der Durchführung des Verfahrens (g) innerhalb eines größeren Bereiches variiert werden. Im Allgemeinen arbeitet man bei Temperaturen zwischen 20°C und 150°C, vorzugsweise zwischen 40°C und 120°C.

Bei der Durchführung des Verfahrens (g) setzt man auf 1 Mol an Dihydroxy-pyrazolopyrimidin der Formel (XV) jeweils einen Überschuss an Halogenierungsmittel ein. Die Aufarbeitung erfolgt nach üblichen Methoden.

Die bei der Durchführung des Verfahrens (f) als Ausgangsstoffe benötigten Halogen-pyrazolopyrimidine sind durch die Formel (XIII) allgemein definiert. In dieser Formel haben R³ und R⁸ vorzugsweise diejenigen Bedeutungen, die bereits im Zusammenhang mit der Beschreibung der erfindungsgemäßen Stoffe der Formel (I) für diese Reste als bevorzugt genannt wurden. X² und Y¹ stehen jeweils vorzugsweise für Fluor, Chlor oder Brom, besonders bevorzugt für Fluor oder Chlor.

Die bei der Durchführung des Verfahrens (f) als Reaktionskomponenten benötigten Amine sind durch die Formel (XII) allgemein definiert. In dieser Formel haben R¹ und R² vorzugsweise diejenigen Bedeutungen, die bereits im Zusammenhang mit der Beschreibung der erfindungsgemäßen Stoffe der Formel (I) für diese Reste als bevorzugt genannt wurden.

Die im zweiten Schritt des Verfahrens (f) als Reaktionskomponenten benötigten Verbindungen sind durch die Formel (XIV) allgemein definiert. In dieser Formel steht R¹³ vorzugsweise für Alkoxy mit 1 bis 4 Kohlenstoffatomen, Alkylthio mit 1 bis 4 Kohlenstoffatomen, Alkylsulfinyl mit 1 bis 4 Kohlenstoffatomen oder Alkylsulfonyl mit 1 bis 4 Kohlenstoffatomen. Me steht auch vorzugsweise für Natrium oder Kalium.

Besonders bevorzugt sind Verbindungen der Formel (XIV), in denen R¹³ für Methoxy, Ethoxy, Methylthio, Methylsulfinyl oder Methylsulfonyl steht und Me für Natrium oder Kalium steht.

Die Amine der Formel (XII) und auch die Verbindungen der Formel (XIV) sind bekannt oder lassen sich nach bekannten Methoden herstellen.

Als Verdünnungsmittel kommen bei der Durchführung der ersten Stufe des Verfahrens (f) alle üblichen inerten organischen Solventien in Betracht. Vorzugsweise verwendbar sind halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Als Säureakzeptoren kommen bei der Durchführung der ersten Stufe des Verfahrens (f) alle für derartige Umsetzungen üblichen anorganischen oder organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithium-diisopropylamid, Natrium methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem AmmoniumVerbindungen wie Ammoniumhydroxid, Ammoniumacetat und Ammoniumcarbonat, sowie tertiäre Amine, wie Trimethylamin, Triethylamin, Tributylamin, N,N-Dimlethylamilin, N,N-Dimethyl-benzylamin, Pyridin, N-Methylpiperidin, N-Methylmorpholin, N,N Dimethylaminopyridin, Diazabicyclooctan (DABCO), Diazabicyclononen (DBN) oder Diazabicycloundecen (DBU).

Als Katalysatoren kommen bei der Durchführung der ersten Stufe des Verfahrens (f) alle für derartige Umsetzungen üblichen Reaktionsbeschleuniger in Betracht. Vorzugsweise verwendbar sind Fluoride wie Natriumfluorid, Kaliumfluorid oder Ammoniumfluorid.

Die Reaktionstemperaturen können bei der Durchführung der ersten Stufe des Verfahrens (f) in einem größeren Bereich variiert werden. Im Allgemeinen arbeitet man bei Temperaturen zwischen 0°C und 150°C, vorzugsweise bei Temperaturen zwischen 0°C und 80°C.

Bei der Durchführung der ersten Stufe des Verfahrens (f) setzt man auf 1 Mol an Halogenpyrazolopyrimidin der Formel (XIII) im Allgemeinen 0,5 bis 10 Mol, vorzugsweise 0,8 bis 2 Mol an Amin der Formel (XII) ein. Die Aufarbeitung erfolgt nach üblichen Methoden.

Die bei der Durchführung der zweiten Stufe des Verfahrens (f) kommen als Verdünnungsmittel alle üblichen, inerten organischen Solventien in Betracht. Vorzugsweise verwendbar sind halogenierte Kohlenwasserstoffe, wie beispielsweise Chlorbenzol, Dichlorbenzol, Dichlormethan, -Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Auch bei der Durchführung der zweiten Stufe des Verfahrens (f) können die Reaktionstemperaturen in einem größeren Bereich variiert werden. Im Allgemeinen arbeitet man bei Temperaturen zwischen 0°C und 150°C, vorzugsweise zwischen 20°C und 100°C.

Bei der Durchführung der zweiten Stufe des Verfahrens (f) setzt man die jeweilige Cyano-Verbindung der Formel (Via) mit einer äquivalenten Menge oder mit einem Überschuss an einer Verbindung der Formel (XIV) um. Die Aufarbeitung erfolgt nach üblichen Methoden.

Bei der Durchführung des erfindungsgemäßen Verfahrens (a, Variante β) und des Verfahrens (c, Variante β) als Reaktionskomponenten benötigten Grignard-Verbindungen sind durch die Formel (III) allgemein definiert. In dieser Formel steht R⁹ vorzugsweise für Alkyl mit 1 bis 4 Kohlenstoffatomen, Alkoxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen, Alkinyl mit 2 bis 5 Kohlenstoffatomen oder Benzyl. X steht auch vorzugsweise für Chlor, Brom oder Iod.

Besonders bevorzugt sind diejenigen Verbindungen der Formel (III), in denen
- R⁹: für Methyl, Ethyl, Propyl, Methoxymethyl, Methox-yethyl, Alkenyl mit 3 oder 4 Kohlenstoffatomen, Alkinyl mit 3 oder 4 Kohlenstoffatomen oder Benzyl steht und
- X: für Chlor, Brom oder Iod steht.

Die Grignard-Verbindungen der Formel (III) sind bekannt oder lassen sich nach bekannten Methoden herstellen.

Als Verdünnungsmittel kommen bei der Durchführung des Verfahrens (c, Variante α) alle üblichen inerten, organischen Solventien in Frage. Vorzugsweise verwendbar sind aliphatische oder aromatische, gegebenenfalls halogenierte Kohlenwasserstoffe, wie Toluol, Dichlormethan, Chloroform oder Tetrachlorkohlenstoff.

Die Reaktionstemperaturen können bei der Durchführung des Verfahrens (c, Variante α) innerhalb eines bestimmten Bereiches variiert werden. Im Allgemeinen arbeitet man bei Temperaturen zwischen -80°C und +20°C, vorzugsweise zwischen -60°C und +10°C.

Bei der Durchführung des Verfahrens (c, Variante α) setzt man auf 1 Mol an Pyrazolopyrimidin der Formel (VI) im Allgemeinen eine äquivalente Menge oder auch einen Überschuss, vorzugsweise 1,1 bis 1,2 Mol an Di-isobutyl-aluminiumhydrid ein und fügt anschließend einen Überschuss an wässriger Ammoniumchlorid-Lösung hinzu. Die Aufarbeitung erfolgt nach üblichen Methoden. Im Allgemeinen verfährt man in der Weise, dass man das Reaktionsgemisch ansäuert, die organische Phase abtrennt, die wässrige Phase mit einem mit Wasser wenig mischbaren organischen Solvens extrahiert, die vereinigten organischen Phasen wäscht, trocknet und unter vermindertem Druck einengt.

Als Katalysatoren kommen bei der Durchführung des Verfahrens (c, Variante β) alle für Grignard-Reaktionen üblichen Reaktionsbeschleuniger in Betracht. Beispielsweise genannt seien Kaliumiodid und Iod.

Als Verdünnungsmittel kommen bei der Durchführung des Verfahrens (c, Variante β) alle für derartige Umsetzungen üblichen, inerten organischen Solventien in Frage. Vorzugsweise verwendbar sind Ether, wie Diethylether, Dioxan oder Tetrahydrofuran, außerdem aromatische Kohlenwasserstoffe, wie Toluol, und auch Gemische aus Ethern und aromatischen Kohlenwasserstoffen, wie Toluol/Tetrahydrofuran.

Die Reaktionstemperaturen können bei der Durchführung des Verfahrens (c, Variante β) in einem bestimmten Bereich variiert werden. Im Allgemeinen arbeitet man bei Temperaturen zwischen -20°C und +100°C, vorzugsweise zwischen 0°C und 80°C.

Bei der Durchführung des Verfahrens (c, Variante β) setzt man auf 1 Mol an Pyrazolopyrimidin-Derivate der Formel (VI) im Allgemeinen 2 bis 3 Mol an Grignard-Verbindung der Formel (III) ein. Anschließend erfolgt eine wässrige Aufarbeitung nach üblichen Methoden.

Pyrazolopyrimidine der Formel (II) lassen sich auch nach den Verfahren (d) und (e) herstellen.

Die bei der Durchführung des Verfahrens (d) als Ausgangsstoffe benötigte Pyrazolopyrimidine sind durch die Formel (VII) allgemein definiert. In dieser Formel haben R¹, R², R³, R⁷ und R⁸ vorzugsweise diejenigen Bedeutungen, die bereits im Zusammenhang mit der Beschreibung der erfindungsgemäßen Stoffe der Formel (I) für diese Reste als bevorzugt genannt wurden.

Die Pyrazolopyrimidine der Formel (VII) sind bekannt oder lassen sich nach bekannten Methoden herstellen.

Die bei der Durchführung des Verfahrens (d) als Reaktionskomponenten benötigten aktivierten Carbonsäurederivate, wie Säurehalogenide und Säureanyhdride sind durch die Formeln (VIII) und (IX) allgemein definiert. In der Formel (VIII) steht R¹¹ vorzugsweise für Alkyl mit 1 bis 4 Kohlenstoffatomen, Alkoxyalkyl mit 1 oder 2 Kohlenstoffatomen im Alkoxyteil und 1 bis 4 Kohlenstoffatomen in Alkylteil, Alkenyl mit 2 bis 5 Kohlenstoffatomen, Alkinyl mit 2 bis 5 Kohlenstoffatomen oder für Benzyl. Hal steht auch vorzugsweise für Chlor oder Brom.

Besonders bevorzugt sind Säurehalogenide der Formel (VIII), in denen
- R¹¹: für Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder für Benzyl steht
und
- Hal: für Chlor oder Brom steht.

Als aktiviertes Carbonsäurederivat der Formel (IX) ist bevorzugt beispielsweise das kommerziell erhältliche 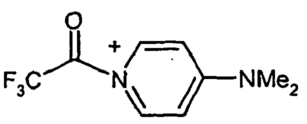 (Synthesis 1996 (9), 1093).

In der Formel (IX) steht R¹² vorzugsweise für Alkyl mit 1 bis 4 Kohlenstoffatomen, besonders bevorzugt für Methyl, Ethyl oder Propyl.

Sowohl die Säurehalogenide der Formel (VIII) als auch die Säureanhydride der Formel (IX) sind bekannt oder lassen sich nach bekannten Methoden herstellen.

Als Katalysatoren kommen bei der Durchführung des Verfahrens (d) alle für Friedel-Crafts-Reaktionen üblicherweise verwendbaren Reaktionsbeschleuniger in Betracht. Vorzugsweise verwendbar sind Lewis-Säuren, wie Aluminium-trichlorid, Aluminium-tribromid und Eisen(III) chlorid.

Als Verdünnungsmittel kommen bei der Durchführung des Verfahrens (d) alle für derartige Friedel-Crafts-Reaktionen üblichen, inerten organischen Solventien in Frage. Vorzugsweise verwendar sind Ether, wie Diethylether, Methyl-tert-butylether, Dioxan und Tetrahydrofuran, sowie auch Schwefelkohlenstoff.

Die Reaktionstemperaturen können bei der Durchführung des Verfahrens (d) in einem bestimmten Bereich variiert werden. Im Allgemeinen arbeitet man bei Temperaturen zwischen -10°C und +100°C, vorzugsweise zwischen 0°C und 60°C.

Bei der Durchführung des Verfahrens (d) setzt man auf 1 mol an Pyrazolopyrimidin der Formel (VII) im Allgemeinen 1 bis 5 mol, vorzugsweise 1 bis 2 mol an Säurehalogenid der Formel (VIII) und 1,1 bis 5 mol, vorzugsweise 1,1 bis 3 mol an Katalysator, beziehungsweise 1 bis 5 mol, vorzugsweise 1 bis 2 mol an Säureanhydrid der Formel (IX) und 2,1 bis 6 mol, vorzugsweise 2,1 bis 4 mol an Katalysator ein. Man verfährt im Allgemeinen in der Weise, dass man die Reaktionskomponenten zunächst bei niedriger Temperatur zusammengibt und nach dem Abklingen der anfangs heftigen Reaktion allmählich bis auf Rückflusstemperatur erhitzt. Die Aufarbeitung erfolgt nach üblichen Methoden.

Die bei der Durchführung des Verfahrens (e) als Ausgangsstoffe benötigten Hydroay-pyrazolopyrimidine sind durch die Formel (X) allgemein definiert. In diese Formel haben R³ und R⁸ vorzugsweise diejenigen Bedeutungen, die bereits im Zusammenhang mit der Beschreibung der erfindungsgemäßen Stoffe der Formel (1) für diese Reste als bevorzugt genannt wurden.

Die Hydroxy-pyrazolopyrimidine der Formel (X) lassen sich nach dem Verfahren (h) herstellen, wenn man Aminopyrazole der Formel (XVII) einsetzt, die statt der CN-Gruppe ein Wasserstoffatom tragen.

Die erste Stufe des Verfahrens (e) wird unter den Bedingungen der Vilsmeier-Formylierung mit Hilfe von Phosphoroxychlorid in Gegenwart von Dimethylformamid durchgeführt. Dabei kann auch Phosphorpentachlorid als Chlorierungsmittel hinzugefügt werden.

Die Reaktionstemperaturen können bei der Durchführung der ersten Stufe des Verfahrens (e) in einem größeren Bereich variiert werden. Im Allgemeinen arbeitet man bei Temperaturen zwischen -10°C und +150°C, vorzugsweise zwischen 0°C und 120°C.

Bei der Durchführung der ersten Stufe des Verfahrens (e) setzt man auf 1 mol an Hydroxypyrazolopyrimidin der Formel (X) im Allgemeinen 2 bis 5 mol an Dimethylformamid, 5 bis 15 mol Phosphoroxychlorid und gegebenenfalls 0 bis 2 mol Phosphorpentachlorid ein. Die Aufarbeitung erfolgt nach üblichen Methoden.

Bei der Durchführung der zweiten Stufe des Verfahrens (e) kommen die Amine der Formel (XII) sowie diejenigen Katalysatoren, Säurebindemittel und Verdünnungsmittel in Betracht, die bereits im Zusammenhang mit der Beschreibung des Verfahrens (f) genannt wurden. Auch die Reaktionstemperaturen und die übrigen Umsetzungsbedingungen entsprechen denjenigen, die im Falle des Verfahrens (f) angewandt werden.

Die bei der Durchführung des erfindungsgemäßen Verfahrens (a) weiterhin als Reaktionskomponenten benötigten Verbindungen sind durch die Formel (IV) allgemein definiert. In dieser Formel steht R¹⁰ vorzugsweise für Alkyl mit 1 bis 4 Kohlenstoffatomen, Alkoxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen, Alkinyl mit 2 bis 5 Kohlenstoffatomen oder Benzyl. X¹ steht vorzugsweise für Chlor, Brom, Iod oder den Rest der Formel ${R}^{10} ⁢ O - {SO}_{2} - O ,$ worin R¹⁰ die zuvor als bevorzugt angegebenen Bedeutungen hat.

Besonders bevorzugt sind diejenigen Verbindungen der Formel (IV), in denen
- R¹⁰: für Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder für Benzyl steht und
- X¹: für Chlor, Brom, Iod oder den Rest der Formel ${R}^{10} ⁢ O - {SO}_{2} - O$steht, worin R¹⁰ die zuvor als besonders bevorzugt angegebenen Bedeutungen hat.

Die Verbindungen der Formel (IV) sind bekannt oder lassen sich nach bekannten Methoden herstellen.

Verwendet man bei der Durchführung des erfindungsgemäßen Verfahrens (a, Variante α) in der ersten Stufe Di-isobutyl-aluminiumhydrid als Reduhrtionsmittel, so arbeitet man zweckmäßigerweise unter den Bedingungen, die bereits im Zusammenhang mit der Beschreibung des Verfahrens (c, Variante α) erwähnt wurden.

Verwendet man bei der Durchführung des erfindungsgemäßen Verfahrens (a, Variante α) in der ersten Stufe Natriumborhydrid als Reduktionsmittel, so verwendet man als Verdünnungsmittel im Allgemeinen Alkohole, vorzugsweise Methanol, Ethanol oder Isopropanol.

Bei der Reduktion mit Natriumborhydrid können die Reaktionstemperaturen innerhalb eines bestimmten Bereiches variiert werden. Im Allgemeinen arbeitet man bei Temperaturen zwischen 0°C und 70°C, vorzugsweise zwischen 0°C und 50°C.

Bei der Durchführung der Reduktion mit Natriumborhydrid setzt man auf 1 Mol an Pyrazolopyrimidin der Formel (II) eine äquivalente Menge oder auch einen Überschuss an Natriumborhydrid ein. Die Aufarbeitung erfolgt wiederum nach üblichen Methoden.

Bei der Durchführung des erfindungsgemäßen Verfahrens (a, Variante β) arbeitet man unter den Bedingungen, die bereits im Zusammenhang mit der Beschreibung des Verfahrens (c, Variante β) erwähnt wurden.

Als Verdünnungsmittel kommen bei der Durchführung der zweiten Stufe des erfindungsgemäßen Verfahrens (a) alle üblichen, inerten organischen Solventien in Betracht. Vorzugsweise verwendbar sind Ether, wie Dioxan oder Tetrahydrofuran, und außerdem Nitrile, wie Acetonitril.

Die Temperaturen können bei der Durchführung der zweiten Stufe des erfindungsgemäßen Verfahrens (a) innerhalb eines größeren Bereiches variiert werden. Im Allgemeinen arbeitet man bei Temperaturen zwischen 0°C und 100°C, vorzugsweise zwischen 20°C und 80°C.

Bei der Durchführung der zweiten Stufe des erfindungsgemäßen Verfahrens (a) setzt man auf 1 Mol an Pyrazolopyrimidin der Formel (Ia) im Allgemeinen 1 bis 2 Mol, vorzugsweise 1 bis 1,5 Mol an Verbindung der Formel (IV) ein. Die Aufarbeitung erfolgt wiederum nach üblichen Methoden.

Die bei der Durchführung des erfindungsgemäßen Verfahrens (b) als Ausgangssubstanzen benötigten Pyrazolopyrimidine sind durch die Formel (Ia) allgemein definiert. In dieser Formel haben R¹, R², R³, R⁴, R⁷ und R⁸ vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Pyrazolopyrimidinen der Formel (Ia) handelt es sich um erfindungsgemäße Substanzen, die sich nach dem erfindungsgemäßen Verfahren (a) herstellen lassen.

Die bei der Durchführung des erfindungsgemäßen Verfahrens (b) als Reaktionskomponenten benötigten Diole sind durch die Formel (V) definiert. Bevorzugt sind Diole der Formel (V), in denen
- p: für 2, 3 oder 4 steht und
1 oder 2 Wasserstoffatome durch Methyl, Ethyl, Hydroxy, Methoxy, Ethoxy, Hydroxymethyl, Methoxymethyl oder Ethoxymethyl ersetzt sein können.

Besonders bevorzugt sind Diole der Formel (V), in denen
- p: für 2 steht und
1 oder 2 Wasserstoffatome durch Methyl, Ethyl, Hydroxy, Methoxy, Ethoxy, Hydroxymethyl, Methoxymethyl oder Ethoxymethyl ersetzt sein können.

Als Katalysatoren kommen bei der Durchführung des erfindungsgemäßen Verfahrens (b) alle für derartige Umsetzungen üblichen Reah-tionsbeschleuniger in Frage. Vorzugsweise verwendbar sind saure Katalysatoren, wie verdünnte Salzsäure oder verdünnte Schwefelsäure, außerdem p-Toluolsulfonsäure.

Als Verdünnungsmittel können bei der Durchführung des erfindungsgemäßen Verfahrens (b) alle üblichen, inerten, organischen Solventien verwendet werden. Vorzugsweise verwendbar sind Ether, wie Diethylether, Tetrahydrofuran oder Dioxan, Nitrile, wie Acetonitril, oder aromatische Kohlenwasserstoffe, wie Toluol. Außerdem können auch die Diole selbst als Lösungsmittel fungieren.

Die Temperaturen können bei der Durchführung des erfindungsgemäßen Verfahrens (b) innerhalb eines bestimmten Bereiches variiert werden. Im Allgemeinen arbeitet man bei Temperaturen zwischen 0°C und 150°C, vorzugsweise zwischen 20°C und 120°C.

Bei der Durchführung des erfindungsgemäßen Verfahrens (b) setzt man auf 1 Mol an Pyrazolopyrimidin der Formel (Ia) im Allgemeinen einen Überschuss an Diol der Formel (V) ein. Die Aufarbeitung erfolgt nach üblichen Methoden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, von Krankheiten im Wein-, Obst- und Gemüseanbau, wie beispielsweise gegen Botrytis-, Venturia-, Sphaerotheca- und Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimienmgsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Ihokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoaystrobin; Benalaxyl; Benalaxyl-M; Benodanil; Benomyl; Benthiavalicarb-isopropyl; Benzamacril; Benzamacril-isobutyl; Bilanafos; Binapacryl; Biphenyl; Bitertanol; Blasticidin-S; Boscalid; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinarn; Flubenzimine; Fludioxonil; Flumetover; Flumorph; Fluoromide; Fluoxastrobin; Fluquinconazole; Flurprimidol; Flusilazole; Flusulfamide; Fluto-lanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Inunamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2 propnyl]oxy]-3-methoxyphenyl]ethyl]-3 methyl 2-[(methylsulfonyl)amino]-butanamid; 1-(1-Naphthalinyl)-1H-pyrrol-2,5-diom, 2,3,5,6-Tetrachlor-4-(methylsulfonyl)-pyridin; 2-Amino-4-methyl-N phenyl-S-thiazolcarboxamid; 2-Chlor-N-(2,3-dihydro-1,1,3-trimethyl-1H-inden-4-yl)-3-pyridincarboxamid; 3,4,5-Trichlor 2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyndinyl)-cyclopropancarboxamid; N-Butyl-8-(1,1-dimethylethyl)-1-oxaspiro[4.5]decan-3-amin; Natriumtetracarbonat;
sowie Kupfersalze und -zubereitungen, wie Bordeaux Mischung; Kupferhydroxid, Kupfernaphthenat; Kupferoxychlorid; Kupfersulfat; Cufraneb; Kupferoxid; Mancopper; Kupferoxin.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophosethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* / *Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Gis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (IR-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (IR-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonisten-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Aceylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid Kanal-Aktivatoren*
   6.1 Mectine (z.B. Abamectin, Avermectin, Emamectin, Emamectin-benzoate, Ivermectin, Milbemectin, Milbemycin)
*7. Juvenilhormon-Mimetika*
   (z.B. Diofenolan, Epofenonane, Fenoxycarb, Hydroprene, Kinoprene, Methoprene, Pyriproxifen, Triprene)
*8. Ecdysonagoniste*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradiente*n
   11.1 Pyrrole (z.B. Chlorfenapyr)
   11.2 Dinitrophenole (z.B. Binapacyrl, Dinobuton, Dinocap, DNOC)
*12. Site-I-Elektronentransportinhibitoren*
   12.1 METI's (z.B. Fenazaquin, Fenpyroximate, Pyrimidifen, Pyridaben, Tebufenpyrad, Tolfenpyrad)
   12.2 Hydramethylnone
   12.3 Dicofol
*13. Site-ll-Elektronentransportinhibitoren*
   13.1 Rotenone
*14. Site-III Elektronentransportinhibitoren*
   14.1 Acequinocyl, Fluacrypyrim
*15. Mikrobielle Disruptoren der Insektendannmembran*
   Bacillus thuringiensis-Stämme
*16. Inhibitoren der Fettsynthese*
   16.1 Tetronsänren (z.B. Spirodiclofen, Spiromesifen)
   16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg: No.: 382608-10-8) and Carbonic acid, cis-3-(2,5-dimethylphenyl)-8-methoxy-2-oxo-1-azaspiro[4.5]dec-3-en--yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Phthalamide*
   (z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon,Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits", die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), K.nockOui® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (1) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeftihrten Verbindungen bzw. Mischungen.

Weiterhin eignen sich die erfindungsgemäßen Verbindungen der Formel (I) zur Unterdrückung des Wachstums von Tumorzellen in Menschen und Säugetieren. Dies basiert auf einer Wechselwirkung der erfindungsgemäßen Verbindungen mit Tubulin und Mikrotubuli und durch Förderung der Mikrotubuli-Polymerisation.

Zu diesem Zweck kann man eine wirksame Menge an einer oder mehreren Verbindungen der Formel (I) oder pharmazeutisch verträglicher Salze davon verabreichen.

Die Herstellung und die Verwendung der erfindungsgemäßen Wirkstoffe geht aus den folgenden Beispielen hervor.

### Herstellungbeispiele

### Beispiel 1

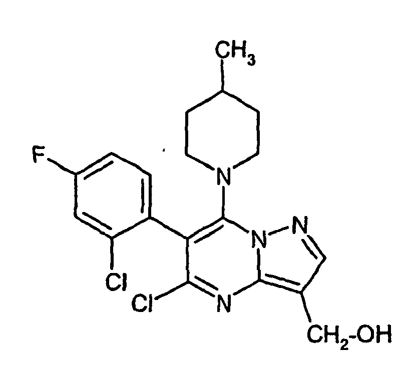

### Verfahren (a, Variante β):

In ein Gemisch aus 1,0 g (2,455 mmol) 3-Formyl-5-chlor-6-(2-chlor-4-fluor-phenyl)-7-(4-methylpiperidino)-pyrazolo[1,5-a]pynmidm und 50 ml Methanol werden bei Raumtemperatur unter Rühren 0,093 g (2,455 mmol) Natriumborhydrid portionsweise gegeben. Nach beendeter Gasentwicklung wird noch 2 Stunden bei Raumtemperatur gerührt und dann unter vermindertem Druck eingeengt. Der verbleibende Rückstand wird mit Wasser verrührt, dann abgesaugt und getrocknet. Man erhält auf diese Weise 0,7 g (64,03 % der Theorie) an 3-Hydroxymethyl-5-chlor-6-(2-chlor-4-fluor-phenyl)-7-(4-methyl-piperidino)-pyrazolo[1,5-a]pyrimidin in Form eines farblosen Feststoffes.
HPLC: logP = 3,89

### Beispiel 2

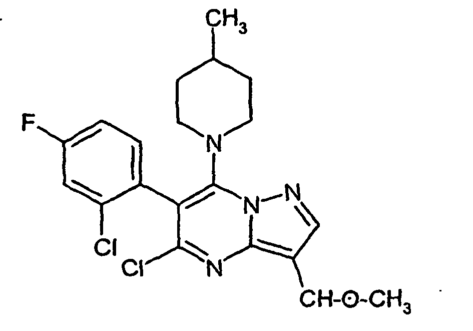

### Verfahren (a), zweite Stufe:

In eine Lösung von 0,5 g (1,222 mmol) 3-Hydroxymethyl-5-chlor-6-(2-chlor-4-fluor-phenyl)-7-(4-methyl-piperidino)-pyrazolo[1,5-a]pyrimidin und 50 ml Tetrahydrofuran werden bei Raumtemperatur unter Rühren 0,098 g (2,443 mmol) Natriumhydrid gegeben. Man lässt 15 Minuten bei Raumtemperatur nachrühren und versetzt dann mit 0,191 g (1,344 mmol) Iodmethan. Das Reaktionsgemisch wird 16 Stunden bei Raumtemperatur gerührt und dann 5 Stunden auf 80°C erhitzt. Es werden erneut 0,05 g Natriumhydrid und 0,1 g Iodmethan hinzugefügt und es wird weitere 2 Stunden unter Rückfluss erhitzt. Danach wird unter vermindertem Druck eingeengt, und der verbleibende Rückstand wird mit Essigsäureethylester extrahiert. Die vereinigten organischen Phasen werden über Natriumsulfat getrocknet und anschließend unter vermindertem Druck eingeengt. Der verbleibende Rückstand wird mit einem Gemisch aus 4 Teilen Cyclohexan und 1 Teil Essigsäureethylester an Kieselgel chromatographiert. Man erhält auf diese Weise 0,7 g (92,6 % der Theorie) an 3-Methoxymethyl-5-chlor-6-(2-chlor-4-fluor-phenyl)-7-(4-methylpiperidno)-pyrazolo[1,5-a]pyrimidin.
HPLC: logP = 5,09

### Beispiel 23

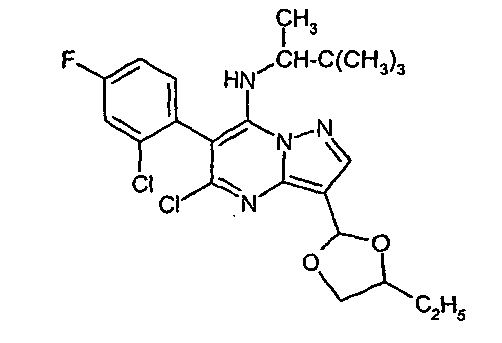

### Verfahren (b):

Ein Gemisch aus 1,22 mmol 3-Formyl-5-chlor-6-(2-chlor-4-fluor-phenyl)-7-(3,3-dimethyl-but-2-yl-amino)-pyrazolo-[1,5-a]pyrimidin, 1,46 mmol Butan-1,2-diol und 6 mmol 4-Toluolsulfonsäure in 80 ml Toluol wird 24 Stunden am Wasserabscheider gekocht. Nach dem Abkühlen auf Raumtemperatur wird die organische Phase mit Wasser gewaschen, dann über Natriumsulfat getrocknet und unter vermindertem Druck eingeengt. Der verbleibende Rückstand wird an Kieselgel chromatographiert. Man erhält auf diese Weise die Substanz der oben angegebenen Formel.
HPLC: log P = 5,70

Die Bestimmung der LogP-Werte erfolgte gemäß EEC-Directive 79/831 Annex V. A8 durch HPLC (Gradientmethode, Acetonitril/0,1 % wässrige Phosphorsäure)

Analog zu den zuvor angegebenen Methoden werden bzw. wurden auch die in den nachstehenden Tabellen 1- 6 aufgeführten Verbindungen der Formel (1) erhalten:

**Tabelle 1**

| | | | |
|---|---|---|---|
| 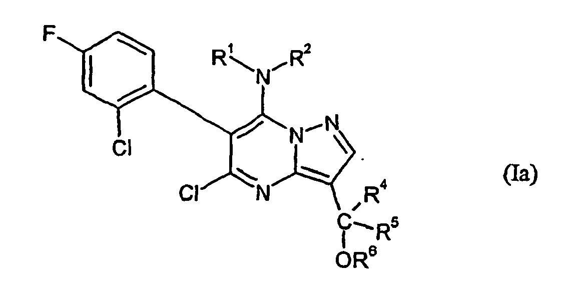 | | | |

| **Bsp. Nr.** | **N(R¹R²)** | **-C(R⁴R⁵(OR⁶))** | **logP** |
|---|---|---|---|
| 1 | 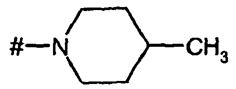 | -CH₂OH | 3,89 |
| 2 | 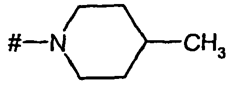 | -CH₂-O-CH₃ | 5,09 |
| 3 | 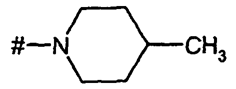 | Allyloxymethyl | 5,73 |
| 4 | 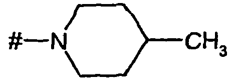 | -CH₂-O-C₂H₅ | 5,53 |
| 5 | 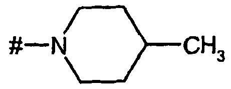 | 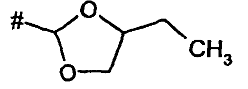 | |
| 6 | 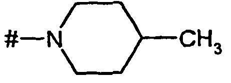 | 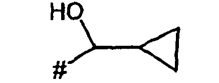 | 4,76 |
| 7 | 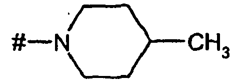 | 1-Hydroxyethyl | 4,24 |
| 8 | 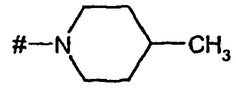 | 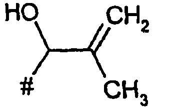 | 4,74 |
| 9 | 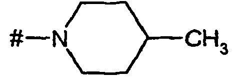 | 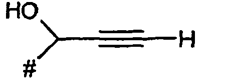 | 4,38 |
| 10 | 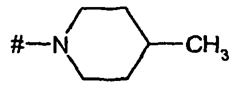 | 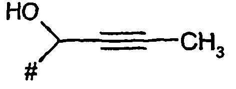 | |
| 11 | 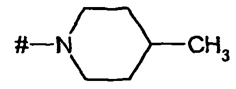 | 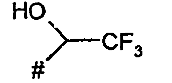 | 4,99 |
| 12 | 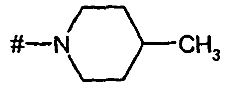 | -CH(OH)-CH₂Cl | |
| 13 | 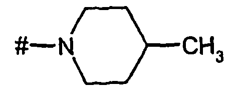 | -CH(OH)-CH(CH₃)₂ | |
| 14 | 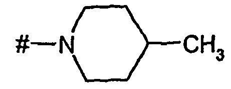 | -CH(OH)-C(CH₃)₃ | |
| 15 | 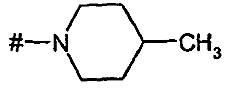 | -C(CH₃)(OH)-CH(CH₃)₂ | |
| 16 | 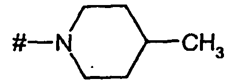 | -CH(OCH₃)-CH(CH₃)₂ | |
| 17 | 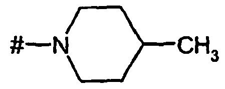 | -CH(CH₃)(OCH(CH₃)₂) | |
| 18 | 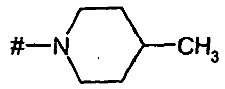 | -C(CH₃)(CF₃)-(OCH₃) | |
| 19 | 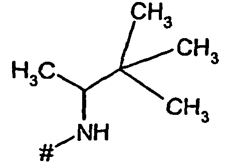 | -CH₂OH | 3,89 |
| 20 | 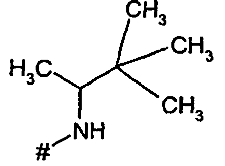 | -CH₂-O-CH₃ | |
| 21 | 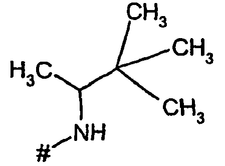 | Allyloxymethyl | |
| 22 | 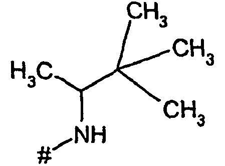 | -CH₂-O-C₂H₅ | |
| 23 | 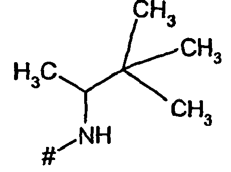 | 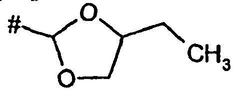 | 5,70 |
| 24 | 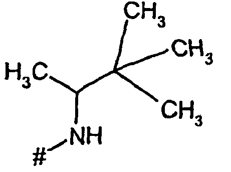 | 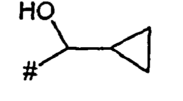 | |
| 25 | 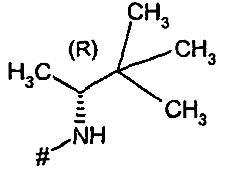 | 1-Hydroxyethyl | |
| 26 | 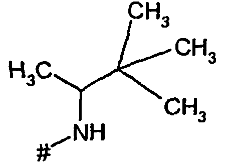 | 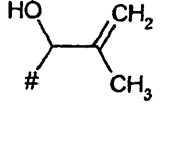 | |
| 27 | 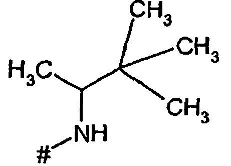 | 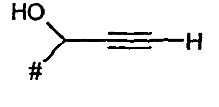 | |
| 28 | 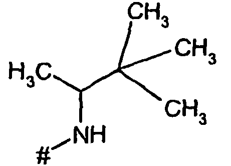 | 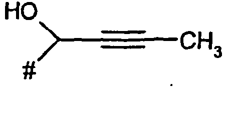 | 4.54 |
| 29 | 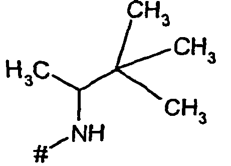 | 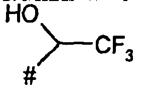 | |
| 30 | 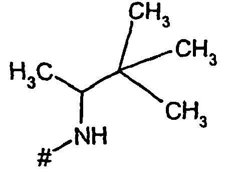 | -CH(OH)-CH₂Cl | |
| 31 | 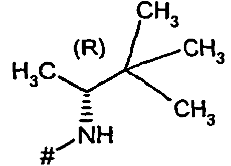 | -CH(OH)-CH(CH₃)₂ | |
| 32 | 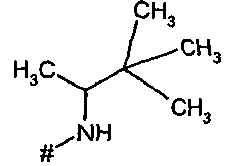 | -CH(OH)-C(CH₃)₃ | |
| 33 | 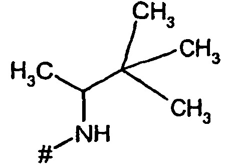 | -C(CH₃)(OH)-CH(CH₃)₂ | |
| 34 | 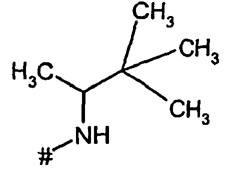 | -CH(OCH₃)-CH(CH₃)₂ | |
| 35 | 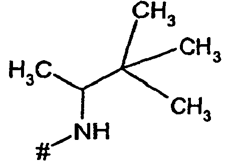 | -CH(CH₃)(OCH(CH₃)₂) | |
| 36 | 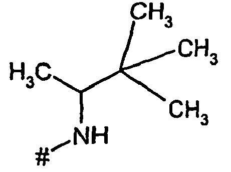 | -C(CH₃)(CF₃)-(OCH₃) | |
| 37 | 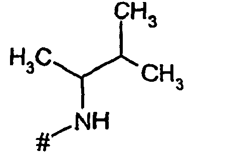 | -CH₂OH | 3,56 |
| 38 | 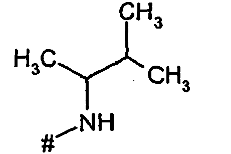 | -CH₂-O-CH₃ | 4,63 |
| 39 | 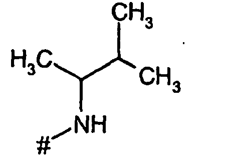 | Allyloxymethyl | |
| 40 | 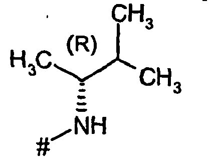 | -CH₂-O-C₂H₅ | |
| 41 | 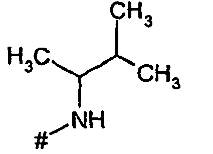 | 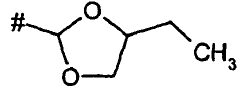 | 5,70 |
| 42 | 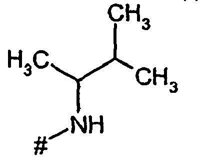 | 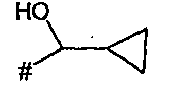 | |
| 43 | 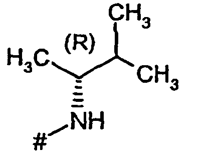 | 1-Hydroxyethyl | |
| 44 | 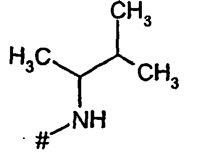 | 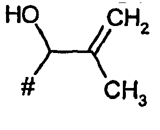 | |
| 45 | 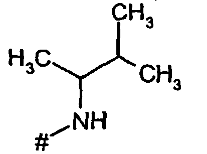 | 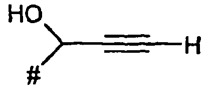 | |
| 46 | 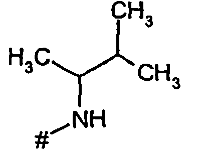 | 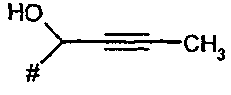 | 4.54 |
| 47 | 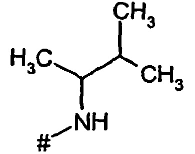 | 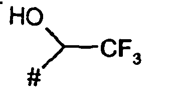 | |
| 48 | 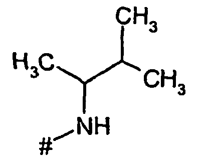 | -CH(OH)-CH₂Cl | |
| 49 | 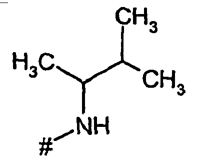 | -CH(OH)-CH(CH₃)₂ | |
| 50 | 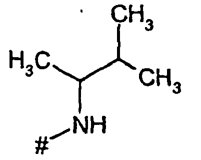 | -CH(OH)-C(CH₃)₃ | |
| 51 | 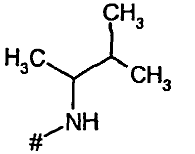 | -C(CH₃)(OH)-CH(CH₃)₂ | |
| 52 | 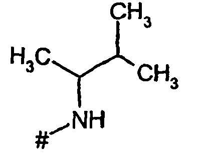 | -CH(OCH₃)-CH(CH₃)₂ | |
| 53 | 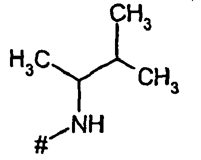 | -CH(CH₃)(OCH(CH₃)₂) | |
| 54 | 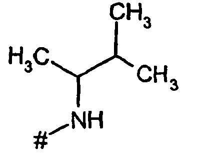 | -C(CH₃)(CF₃)-(OCH₃) | |
| 55 | 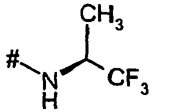 | -CH₂OH | 3,13 |
| 56 | 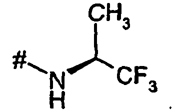 | -CH₂-O-CH₃ | 4,08 |
| 57 | 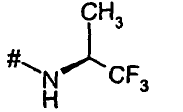 | Allyloxymethyl | |
| 58 | 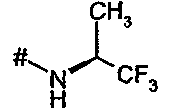 | -CH₂-O-C₂H₅ | |
| 59 | 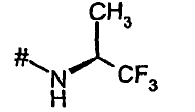 | 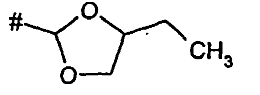 | 5,70 |
| 60 | 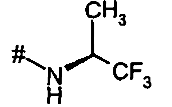 | 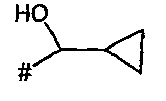 | |
| 61 | 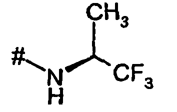 | 1-Hydroxyethyl | |
| 62 | 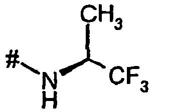 | 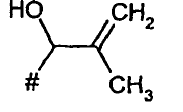 | |
| 63 | 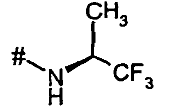 | 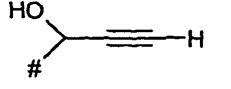 | |
| 64 | 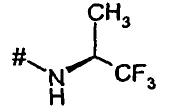 | 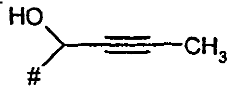 | 4.54 |
| 65 | 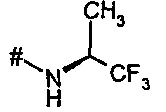 | 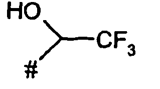 | |
| 66 | 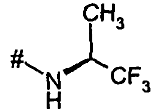 | -CH(OH)-CH₂Cl | |
| 67 | 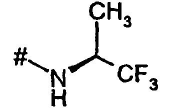 | -CH(OH)-CH(CH₃)₂ | |
| 68 | 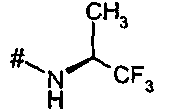 | -CH(OH)-C(CH₃)₃ | |
| 69 | 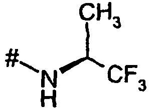 | -C(CH₃)(OH)-CH(CH₃)₂ | |
| 70 | 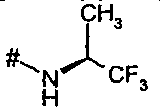 | -CH(OCH₃)-CH(CH₃)₂ | |
| 71 | 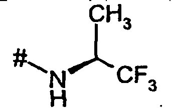 | -CH(CH₃)(OCH(CH₃)₂) | |
| 72 | 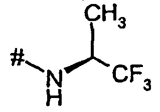 | -C(CH₃)(CF₃)-(OCH₃) | |
| 73 | 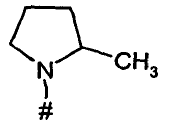 | -CH₂OH | 3,34 |
| 74 | 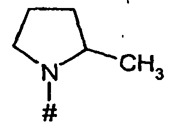 | -CH₂-O-CH₃ | |
| 75 | 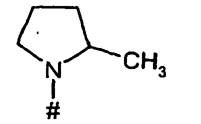 | Allyloxymethyl | |
| 76 | 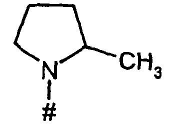 | -CH₂-O-C₂H₅ | |
| 77 | 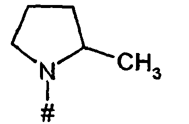 | 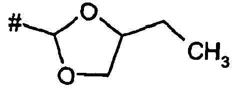 | 5,70 |
| 78 | 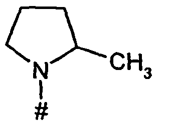 | 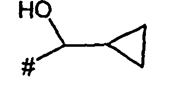 | |
| 79 | 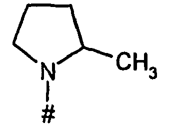 | 1-Hydroxyethyl | |
| 80 | 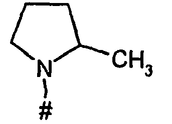 | 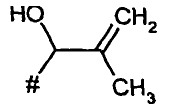 | |
| 81 | 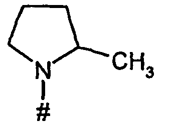 | 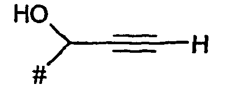 | |
| 82 | 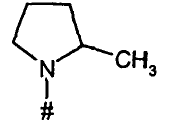 | 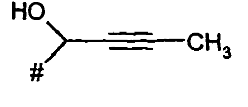 | 4.54 |
| 83 | 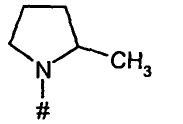 | 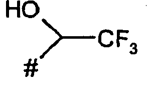 | |
| 84 | 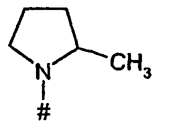 | -CH(OH)-CH₂Cl | |
| 85 | 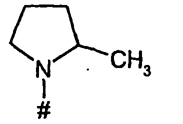 | -CH(OH)-CH(CH₃)₂ | |
| 86 | 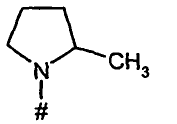 | -CH(OH)-C(CH₃)₃ | |
| 87 | 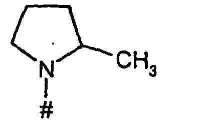 | -C(CH₃)(OH)-CH(CH₃)₂ | |
| 88 | 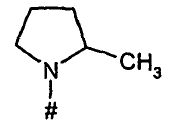 | -CH(OCH₃)-CH(CH₃)₂ | |
| 89 | 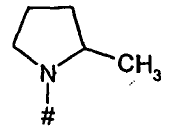 | -CH(CH₃)(OCH(CH₃)₂) | |
| 90 | 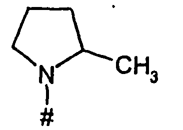 | -C(CH₃)(CF₃)-(OCH₃) | |

**Tabelle 2**

| | | | |
|---|---|---|---|
| 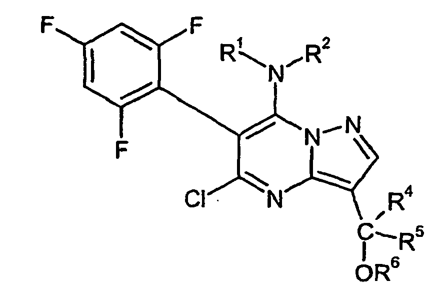 | | | |

| **Bsp. Nr.** | **N(R¹R²)** | **-C(R⁴R⁵(OR⁶)** | **logP** |
|---|---|---|---|
| 91 | 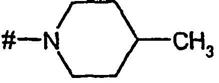 | -CH₂OH | |
| 92 | 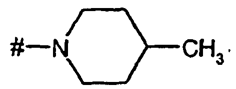 | -CH₂-O-CH₃ | |
| 93 | 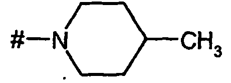 | Allyloxymethyl | |
| 94 | 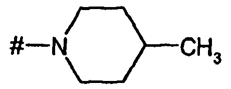 | -CH₂O-C₂H₅ | |
| 95 | 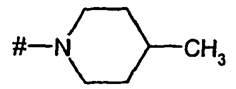 | 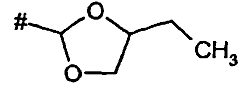 | |
| 96 | 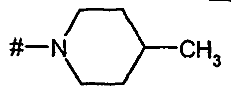 | 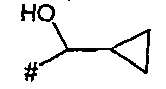 | |
| 97 | 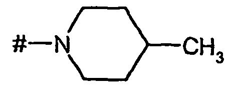 | 1-Hydroxyethyl | |
| 98 | 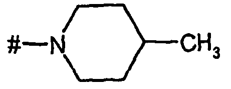 | 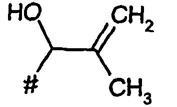 | |
| 99 | 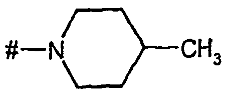 | 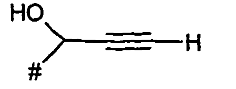 | |
| 100 | 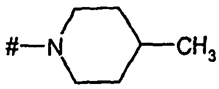 | 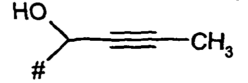 | |
| 101 | 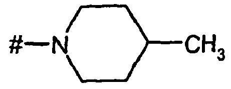 | 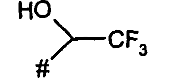 | |
| 102 | 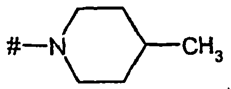 | -CH(OH)-CH₂Cl | |
| 103 | 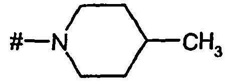 | -CH(OH)-CH(CH₃)₂ | |
| 104 | 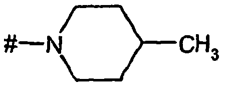 | -CH(OH)-C(CH₃)₃ | |
| 105 | 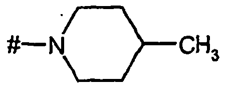 | C(CH₃)(O_{H})-CH(CH₃)₂ | |
| 106 | 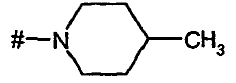 | -CH(OCH₃)-CH(CH₃)₂ | |
| 107 | 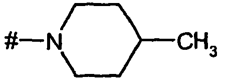 | -CH(CH₃)(OCH(CH₃)₂) | |
| 108 | 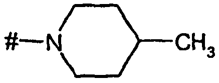 | -C(CH₃)(CF₃)-(OCH₃) | |
| 109 | 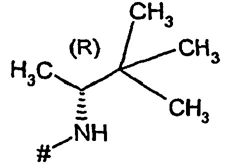 | -CH₂OH | |
| 110 | 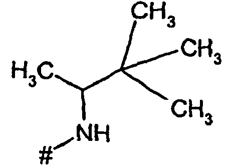 | -CH₂-O-CH₃ | |
| 111 | 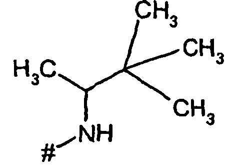 | Allyloxymethyl | |
| 112 | 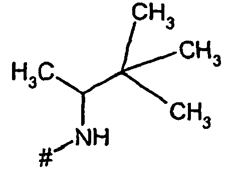 | -CH₂-O-C₂H₅ | |
| 113 | 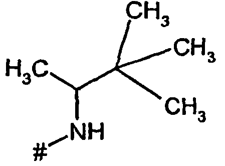 | 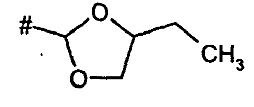 | |
| 114 | 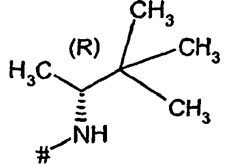 | 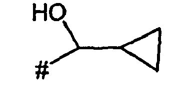 | |
| 115 | 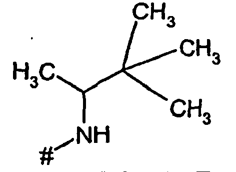 | 1-Hydroxyethyl | |
| 116 | 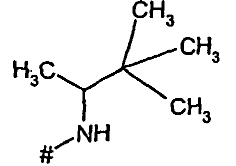 | 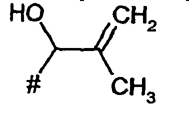 | |
| 117 | 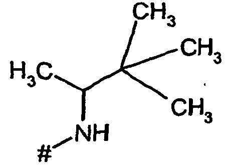 | 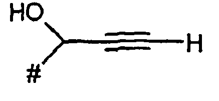 | |
| 118 | 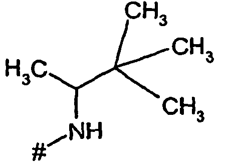 | 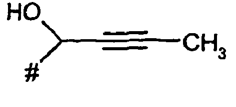 | |
| 119 | 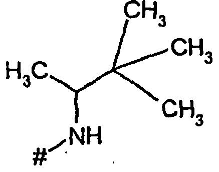 | 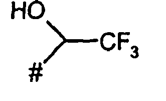 | |
| 120 | 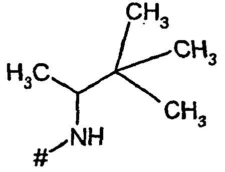 | -CH(OH)-CH₂Cl | |
| 121 | 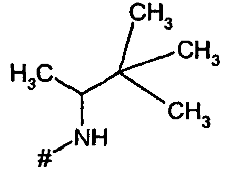 | -CH(OH)-CH(CH₃)₂ | |
| 122 | 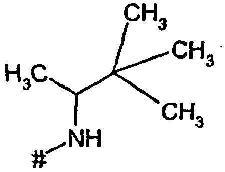 | -CH(OH)-C(CH₃)₃ | |
| 123 | 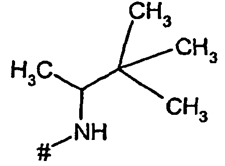 | -C(CH₃)(OH)-CH(CH₃)₂ | |
| 124 | 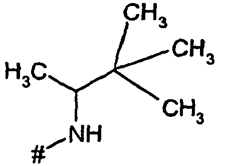 | -CH(OCH₃)-CH(CH₃)₂ | |
| 125 | 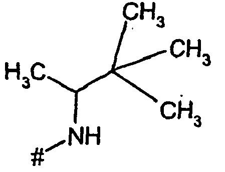 | -CH(CH₃)(OCH(CH₃)₂) | |
| 126 | 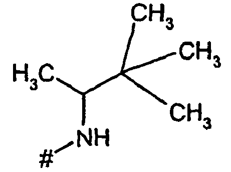 | -C(CH₃)(CF₃)-(OCH₃) | |
| 127 | 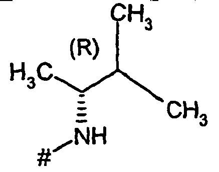 | -CH₂OH | |
| 128 | 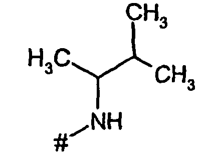 | -CH₂-O-CH₃ | |
| 129 | 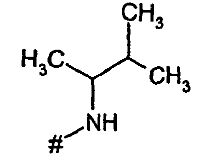 | Allyloxymethyl | |
| 130 | 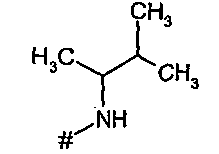 | -CH₂-O-C₂H₅ | |
| 131 | 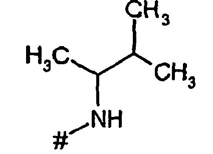 | 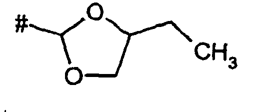 | |
| 132 | 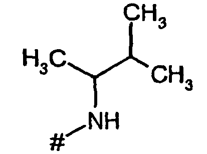 | 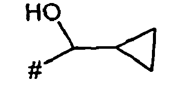 | |
| 133 | 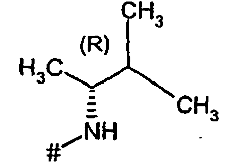 | 1-Hydroxyethyl | |
| 134 | 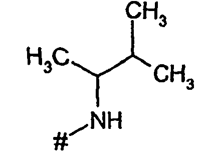 | 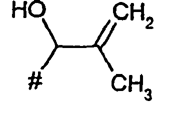 | |
| 135 | 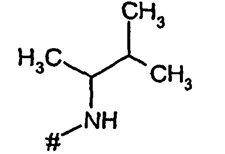 | 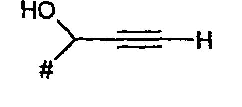 | |
| 136 | 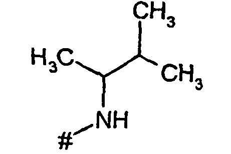 | 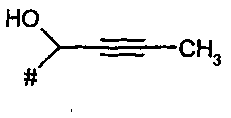 | |
| 137 | 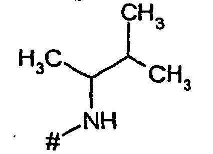 | 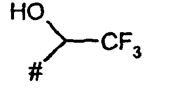 | |
| 138 | 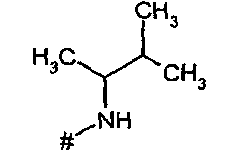 | -CH(OH)-CH₂Cl | |
| 139 | 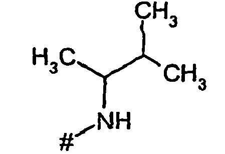 | -CH(OH)-CH(CH₃)₂ | |
| 140 | 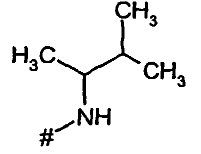 | -CH(OH)-C(CH₃)₃ | |
| 141 | 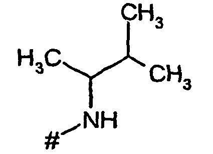 | -C(CH₃)(OH)-CH(CH₃)₂ | |
| 142 | 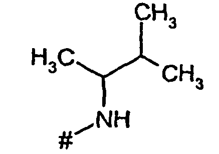 | -CH(OCH₃)-CH(CH₃)₂ | |
| 143 | 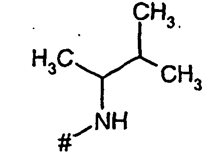 | -CH(CH₃)(OCH(CH₃)₂) | |
| 144 | 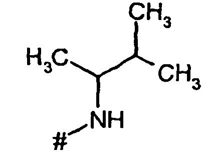 | -C(CH₃)(CF₃)-(OCH₃) | |
| 145 | 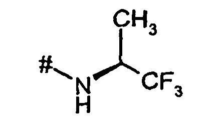 | -CH₂OH | |
| 146 | 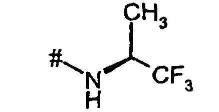 | -CH₂-O-CH₃ | |
| 147 | 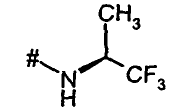 | Allyloxymethyl | |
| 148 | 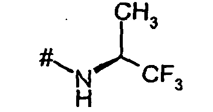 | -CH₂-O-C₂H₅ | |
| 149 | 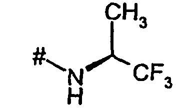 | 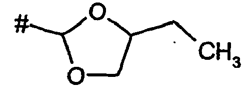 | |
| 150 | 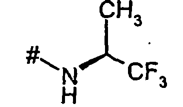 | 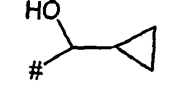 | |
| 151 | 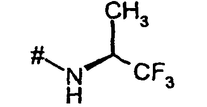 | 1-Hydroxyethyl | |
| 152 | 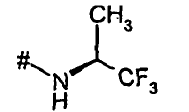 | 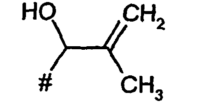 | |
| 153 | 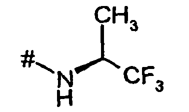 | 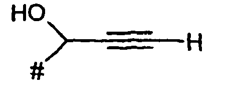 | |
| 154 | 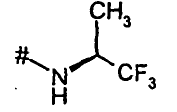 | 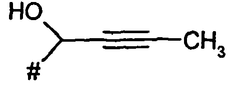 | |
| 155 | 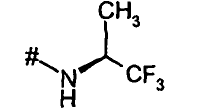 | 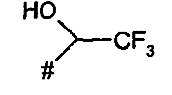 | |
| 156 | 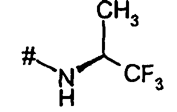 | -CH(OH)-CH₂Cl | |
| 157 | 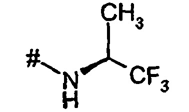 | -CH(OH)-CH(CH₃)₂ | |
| 158 | 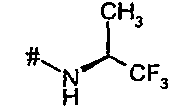 | -CH(OH)-C(CH₃)₃ | |
| 159 | 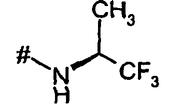 | -C(CH₃)(OH)-CH(CH₃)₂ | |
| 160 | 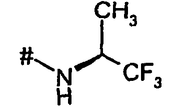 | -CH(OCH₃)-CH(CH₃)₂ | |
| 161 | 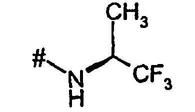 | -CH(CH₃)(OCH(CH₃)₂) | |
| 162 | 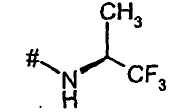 | -C(CH₃)(CF₃)-(OCH₃) | |
| 163 | 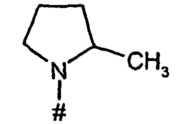 | -CH₂OH | |
| 164 | 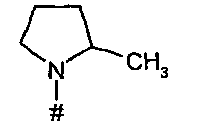 | -CH₂O-CH₃ | |
| 165 | 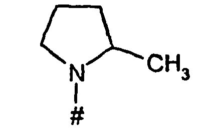 | Allyloxymethyl | |
| 166 | 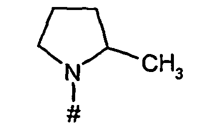 | -CH₂-O-C₂H₅ | |
| 167 | 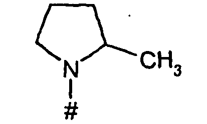 | 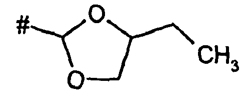 | |
| 168 | 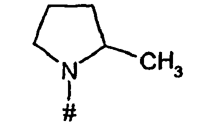 | 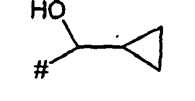 | |
| 169 | 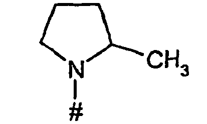 | 1-Hydroxyethyl | |
| 170 | 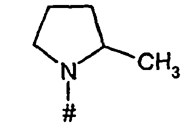 | 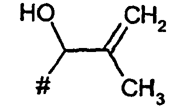 | |
| 171 | 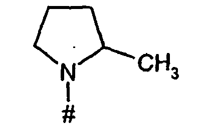 | 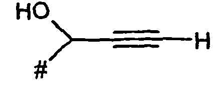 | |
| 172 | 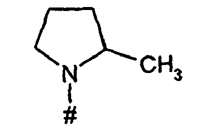 | 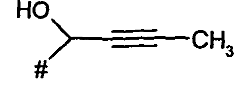 | |
| 173 | 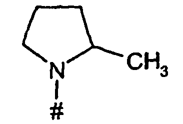 | 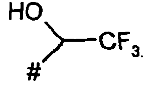 | |
| 174 | 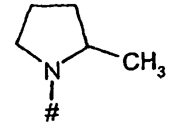 | -CH(OH)-CH₂Cl | |
| 175 | 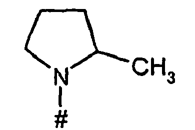 | -CH(OH)-CH(CH₃)₂ | |
| 176 | 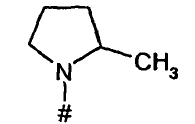 | -CH(OH)-C(CH₃)₃ | |
| 177 | 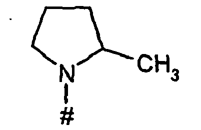 | -C(CH₃)(OH)-CH(CH₃)₂ | |
| 178 | 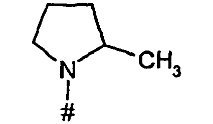 | -CH(OCH₃)-CH(CH₃)₂ | |
| 179 | 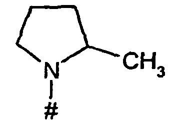 | -CH(CH₃)(OCH(CH₃)₂) | |
| 180 | 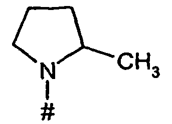 | -C(CH₃)(CF₃)-(OCH₃) | |

**Tabelle 3**

| | | | |
|---|---|---|---|
| 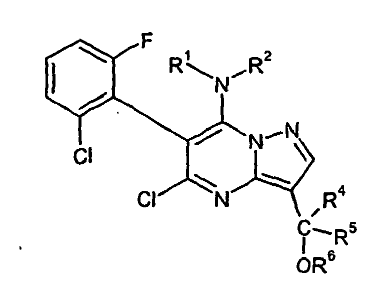 | | | |

| **Bsp. Nr.** | **N(R¹R²)** | **-C(R⁴R⁵(OR⁶)** | **logP** |
|---|---|---|---|
| 181 | 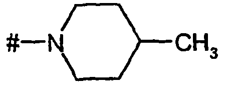 | -CH₂OH | |
| 182 | 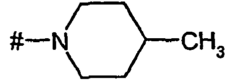 | -CH₂-O-CH₃ | |
| 183 | 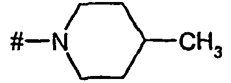 | Allyloxymethyl | |
| 184 | 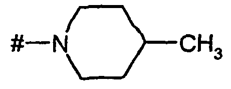 | -CH₂-O-C₂H₅ | |
| 185 | 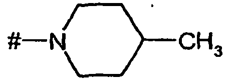 | 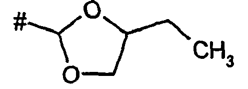 | |
| 186 | 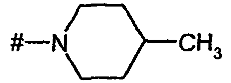 | 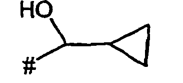 | |
| 187 | 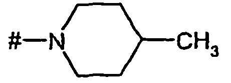 | 1-Hydroxyethyl | |
| 188 | 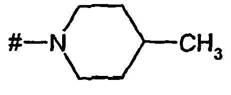 | 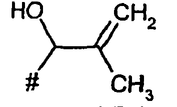 | |
| 189 | 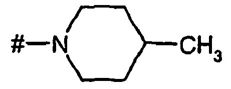 | 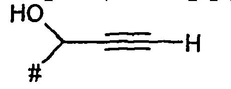 | |
| 190 | 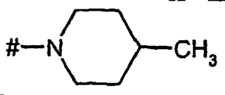 | 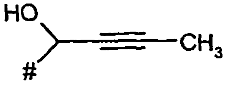 | |
| 191 | 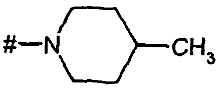 | 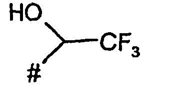 | |
| 192 | 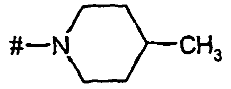 | -CH(OH)-CH₂Cl | |
| 193 | 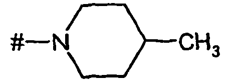 | -CH(OH)-CH(CH₃)₂ | |
| 194 | 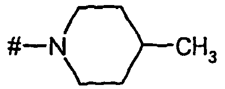 | -CH(OH)-C(CH₃)₃ | |
| 195 | 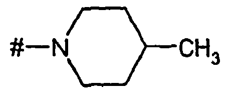 | -C(CH₃)(OH)-CH(CH₃)₂ | |
| 196 | 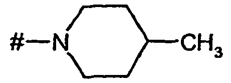 | -CH(OCH₃)-CH(CH₃)₂ | |
| 197 | 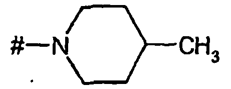 | -CH(CH₃)(OCH(CH₃)₂) | |
| 198 | 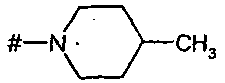 | -C(CH₃)(CF₃)-(OCH₃) | |
| 199 | 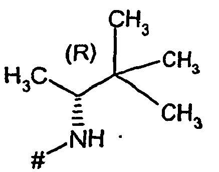 | -CH₂OH | |
| 200 | 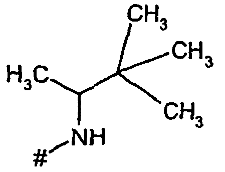 | -CH₂-O-CH₃ | |
| 201 | 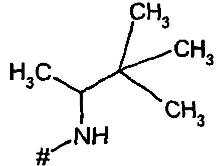 | Allyloxymethyl | |
| 202 | 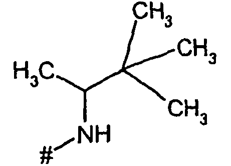 | -CH₂-O-C₂H₅ | |
| 203 | 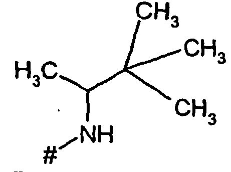 | 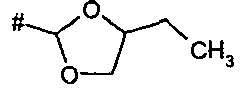 | |
| 204 | 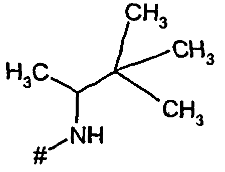 | 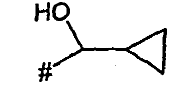 | |
| 205 | 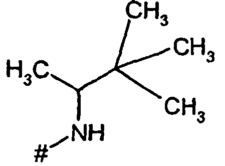 | 1-Hydroxyethyl | |
| 206 | 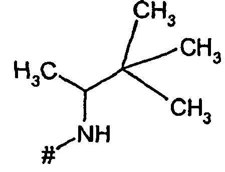 | 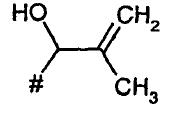 | |
| 207 | 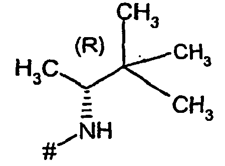 | 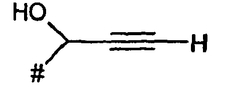 | |
| 208 | 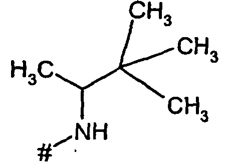 | 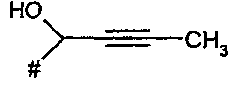 | |
| 209 | 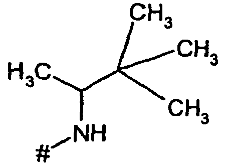 | 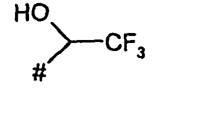 | |
| 210 | 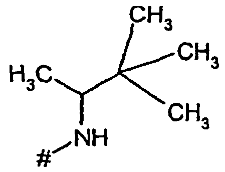 | -CH(OH)-CH₂Cl | |
| 211 | 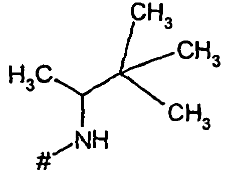 | -CH(OH)-CH(CH₃)₂ | |
| 212 | 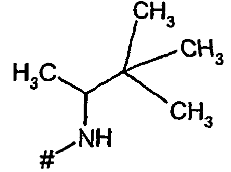 | -CH(OH)-C(CH₃)₃ | |
| 213 | 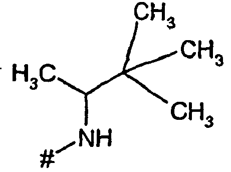 | -C(CH₃)(OH)-CH(CH₃)₂ | |
| 214 | 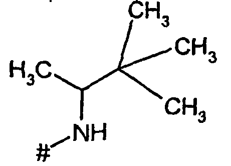 | -CH(OCH₃)-CH(CH₃)₂ | |
| 215 | 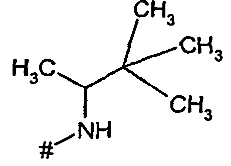 | -CH(CH₃)(OCH(CH₃)₂) | |
| 216 | 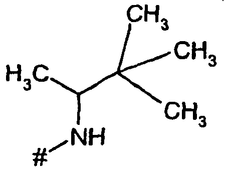 | -C(CH₃)(CF₃)-(OCH₃) | |
| 217 | 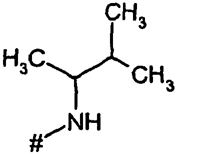 | -CH₂OH | |
| 218 | 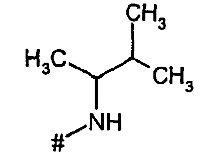 | -CH₂-O-CH₃ | |
| 219 | 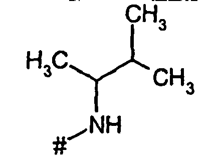 | Allyloxymethyl | |
| 220 | 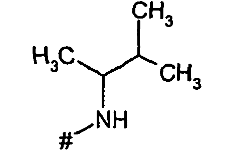 | -CH₂-O-C₂H₅ | |
| 221 | 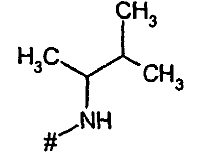 | 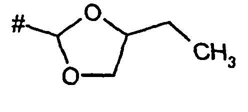 | |
| 222 | 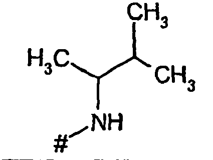 | 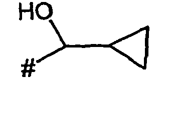 | |
| 223 | 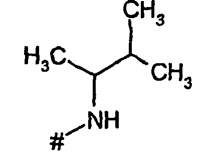 | 1-Hydroxyethyl | |
| 224 | 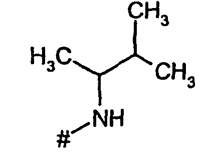 | 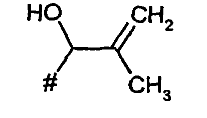 | |
| 225 | 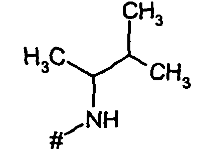 | 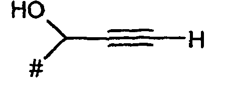 | |
| 226 | 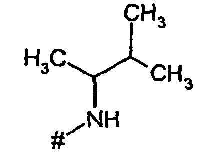 | 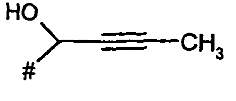 | |
| 227 | 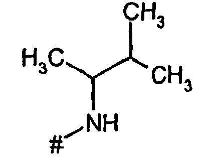 | 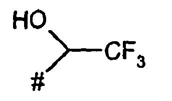 | |
| 228 | 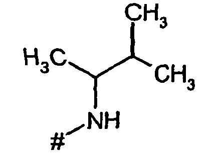 | -CH(OH)-CH₂Cl | |
| 229 | 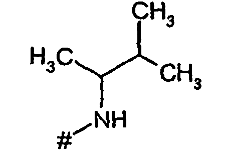 | -CH(OH)-CH(CH₃)₂ | |
| 230 | 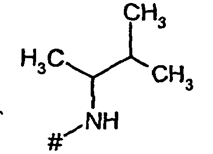 | -CH(OH)-C(CH₃)₃ | |
| 231 | 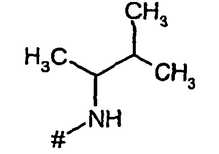 | -C(CH₃)(OH)-CH(CH₃)₂ | |
| 232 | 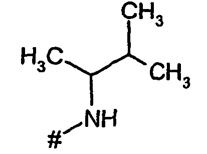 | -CH(OCH₃)-CH(CH₃)₂ | |
| 233 | 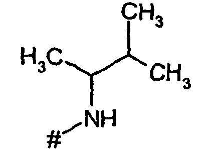 | -CH(CH₃)(OCH(CH₃)₂) | |
| 234 | 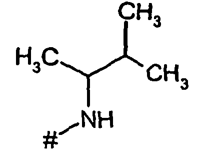 | -C(CH₃)(CF₃)-(OCH₃) | |
| 235 | 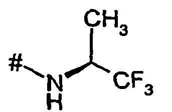 | -CH₂OH | |
| 236 | 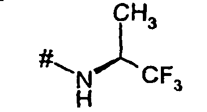 | -CH₂-O-CH₃ | |
| 237 | 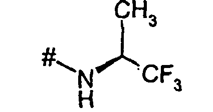 | Allyloxymethyl | |
| 238 | 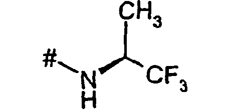 | -CH₂-O-C₂H₅ | |
| 239 | 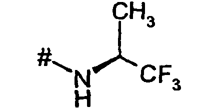 | 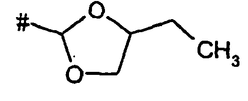 | |
| 240 | 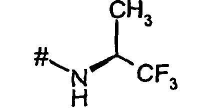 | 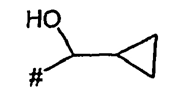 | |
| 241 | 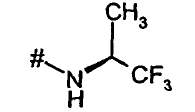 | 1-Hydroxyethyl | |
| 242 | 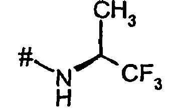 | 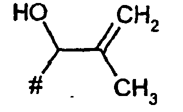 | |
| 243 | 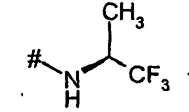 | 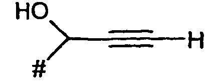 | |
| 244 | 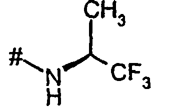 | 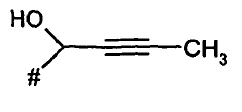 | |
| 245 | 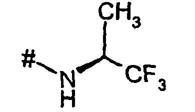 | 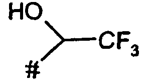 | |
| 246 | 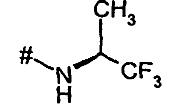 | -CH(OH)-CH₂Cl | |
| 247 | 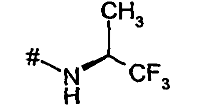 | -CH(OH)-CH(CH₃)₂ | |
| 248 | 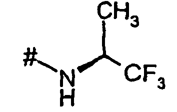 | -CH(OH)-C(CH₃)₃ | |
| 249 | 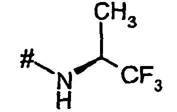 | -C(CH₃)(OH)-CH(CH₃)₂ | |
| 250 | 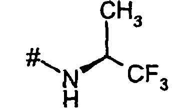 | -CH(OCH₃)-CH(CH₃)₂ | |
| 251 | 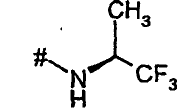 | -CH(CH₃)(OCH(CH₃)₂) | |
| 252 | 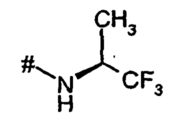 | -C(CH₃)(CF₃)-(OCH₃) | |
| 253 | 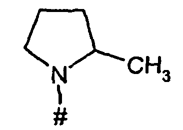 | -CH₂OH | |
| 254 | 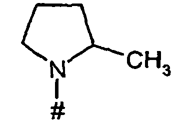 | -CH₂-O-CH₃ | |
| 255 | 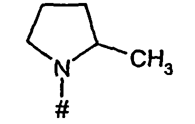 | Allyloxymethyl | |
| 256 | 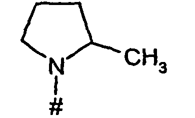 | -CH₂-O-C₂H₅ | |
| 257 | 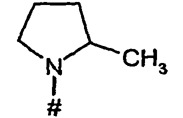 | 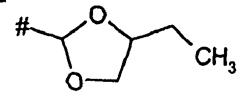 | |
| 258 | 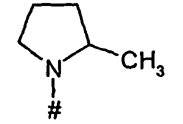 | 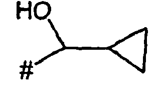 | |
| 259 | 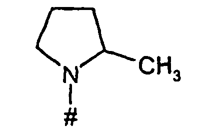 | 1-Hydroxyethyl | |
| 260 | 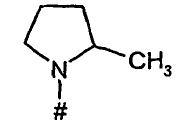 | 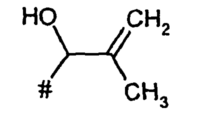 | |
| 261 | 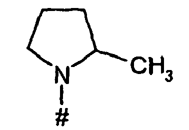 | 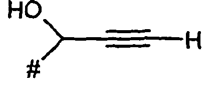 | |
| 262 | 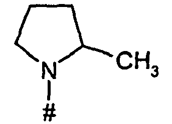 | 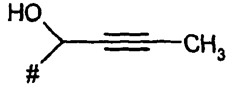 | |
| 263 | 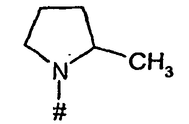 | 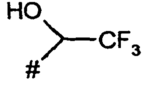 | |
| 264 | 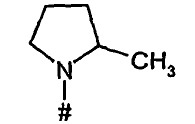 | -CH(OH)-CH₂Cl | |
| 265 | 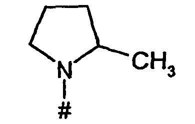 | -CH(OH)-CH(CH₃)₂ | |
| 266 | 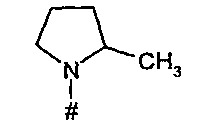 | -CH(OH)-C(CH₃)₃ | |
| 267 | 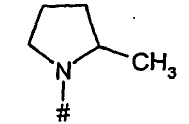 | -C(CH₃)(OH)-CH(CH₃)₂ | |
| 268 | 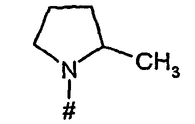 | -CH(OCH₃)-CH(CH₃)₂ | |
| 269 | 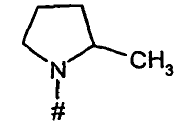 | -CH(CH₃)(OCH(CH₃)₂) | |
| 270 | 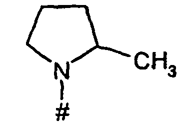 | -C(CH₃)(CF₃)-(OCH₃) | |

**Tabelle 4**

| | | | |
|---|---|---|---|
| 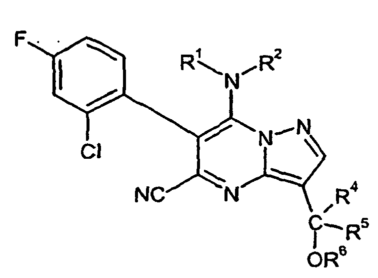 | | | |

| **Bsp. Nr.** | **N(R¹R²)** | **-C(R⁴R⁵(OR⁶)** | **logP** |
|---|---|---|---|
| 271 | 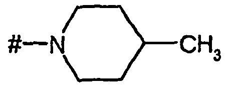 | -CH₂OH | |
| 272 | 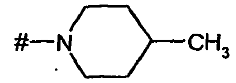 | -CH₂-O-CH₃ | |
| 273 | 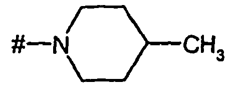 | Allyloxymethyl | |
| 274 | 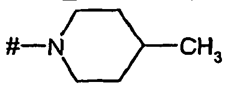 | -CH₂-O-C₂H₅ | |
| 275 | 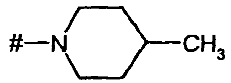 | 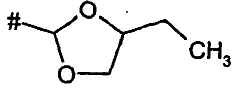 | |
| 276 | 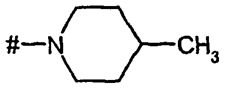 | 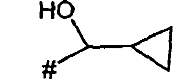 | |
| 277 | 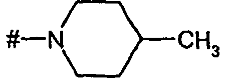 | 1-Hydroxyethyl | |
| 278 | 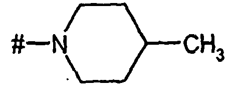 | 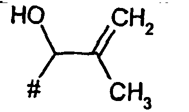 | |
| 279 | 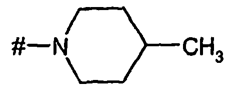 | 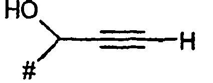 | |
| 280 | 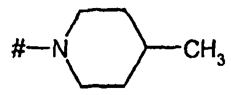 | 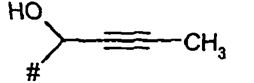 | |
| 281 | 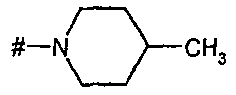 | 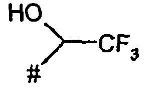 | |
| 282 | 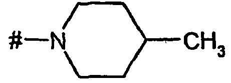 | -(CH(OH)-CH₂Cl | |
| 283 | 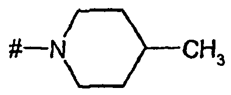 | -CH(OH)-CH(CH₃)₂ | |
| 284 | 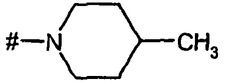 | -CH(O_{H})-C(CH₃)₃ | |
| 285 | 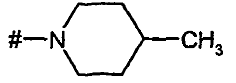 | -C(CH₃)(OH)-CH(CH₃)₂ | |
| 286 | 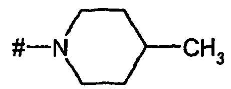 | -CH(OCH₃)-CH(CH₃)₂ | |
| 287 | 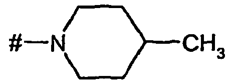 | -CH(CH₃)(OCH(CH₃)₂) | |
| 288 | 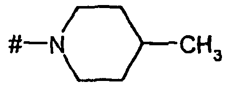 | -C(CH₃)(CF₃)-(OCH₃) | |
| 289 | 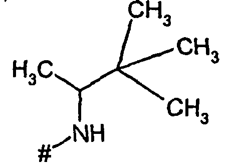 | -CH₂OH | |
| 290 | 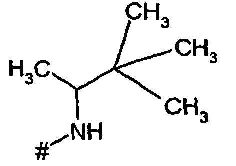 | -CH₂-O-CH₃ | |
| 291 | 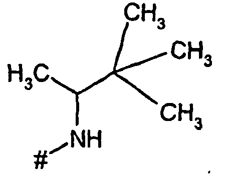 | Allyloxymethyl | |
| 292 | 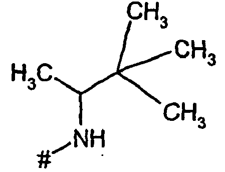 | -CH₂-O-C₂H₅ | |
| 293 | 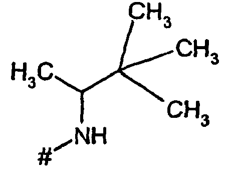 | 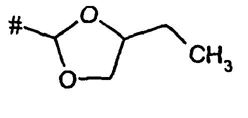 | |
| 294 | 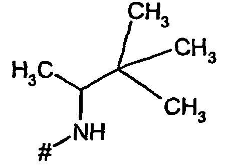 | 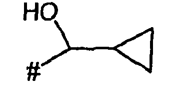 | |
| 295 | 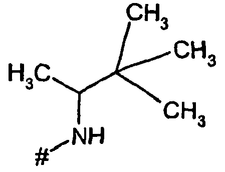 | 1-Hydroxyethyl | |
| 296 | 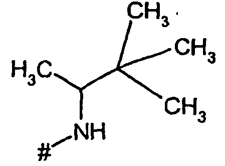 | 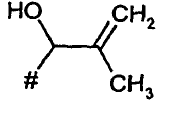 | |
| 297 | 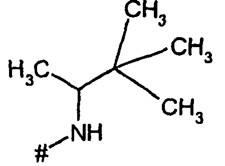 | 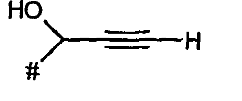 | |
| 298 | 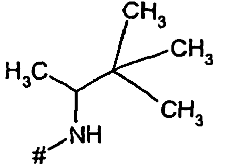 | 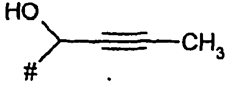 | |
| 299 | 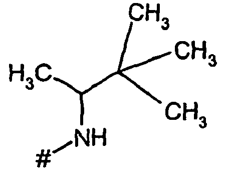 | 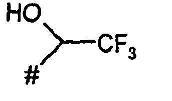 | |
| 300 | 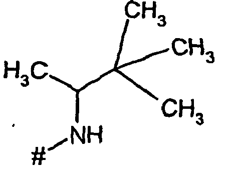 | -(CH(OH)-CH₂Cl | |
| 301 | 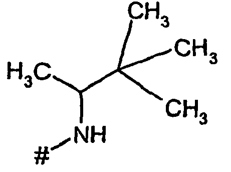 | -CH(OH)-CH(CH₃)₂ | |
| 302 | 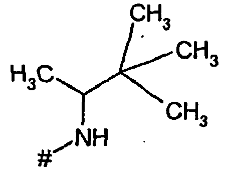 | -CH(OH)-C(CH₃)₃ | |
| 303 | 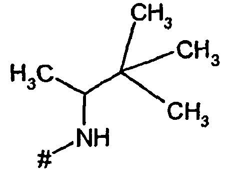 | -C(CH₃)(OH)-CH(CH₃)₂ | |
| 304 | 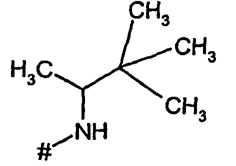 | -CH(OCH₃)-CH(CH₃)₂ | |
| 305 | 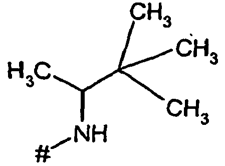 | -CH(CH₃)(OCH(CH₃)₂) | |
| 306 | 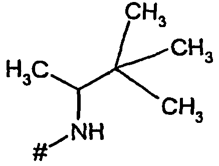 | -C(CH₃)(CF₃)-(OCH₃) | |
| 307 | 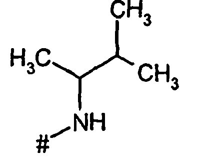 | -CH₂OH | |
| 308 | 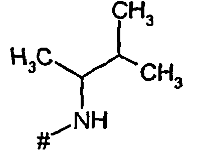 | -CH₂-O-CH₃ | |
| 309 | 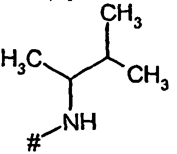 | Allyloxymethyl | |
| 310 | 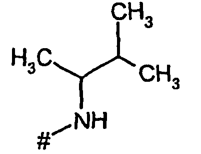 | -CH₂-O-C₂H₅ | |
| 311 | 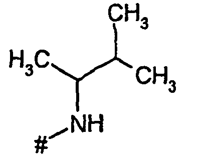 | 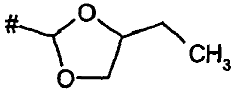 | |
| 312 | 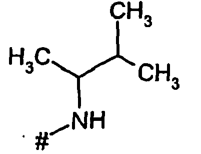 | 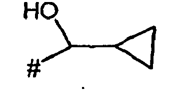 | |
| 313 | 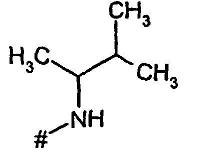 | 1 -Hydroxyethyl | |
| 314 | 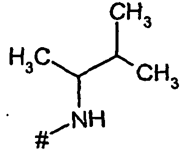 | 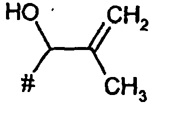 | |
| 315 | 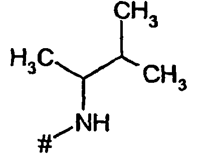 | 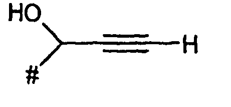 | |
| 316 | 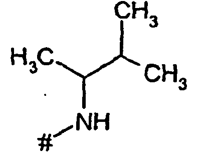 | 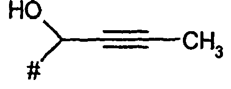 | |
| 317 | 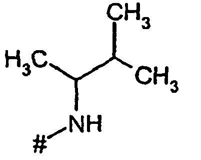 | 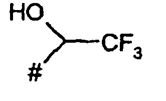 | |
| 318 | 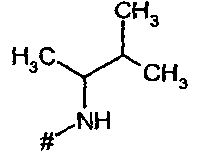 | -(CH(OH)-CH₂Cl | |
| 319 | 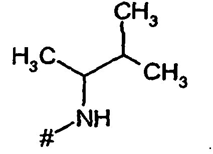 | -CH(OH)-CH(CH₃)₂ | |
| 320 | 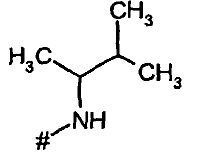 | -CH(OH)-C(CH₃)₃ | |
| 321 | 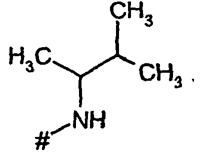 | -C(CH₃)(OH)-CH(CH₃)₂ | |
| 322 | 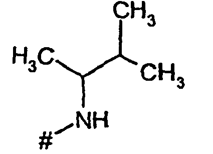 | -CH(OCH₃)-CH(CH₃)₂ | |
| 323 | 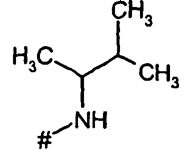 | -CH(CH₃)(OCH(CH₃)₂) | |
| 324 | 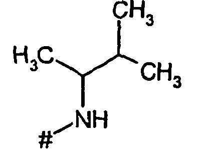 | -C(CH₃)(CF₃)-(OCH₃) | |
| 325 | 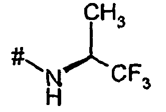 | -CH₂OH | |
| 326 | 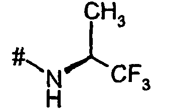 | -CH₂-O-CH₃ | |
| 327 | 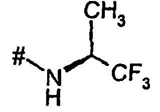 | Allyloxymethyl | |
| 328 | 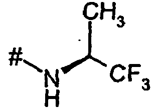 | -CH₂-O-C₂H₅ | |
| 329 | 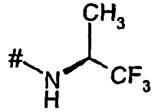 | 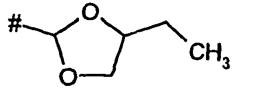 | |
| 330 | 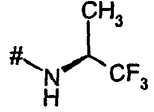 | 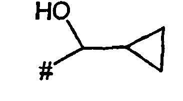 | |
| 331 | 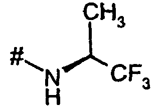 | 1-Hydroxyethyl | |
| 332 | 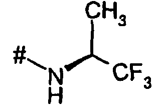 | 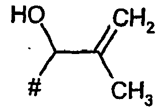 | |
| 333 | 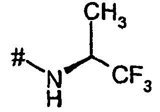 | 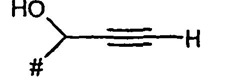 | |
| 334 | 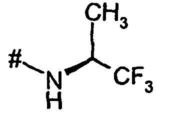 | 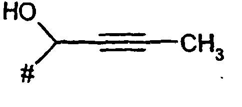 | |
| 335 | 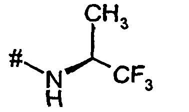 | 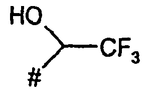 | |
| 336 | 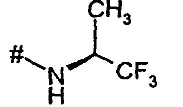 | -(CH(OH)-CH₂Cl | |
| 337 | 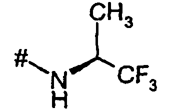 | -CH(OH)-CH(CH₃)₂ | |
| 338 | 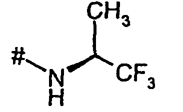 | -CH(OH)-C(CH₃)₃ | |
| 339 | 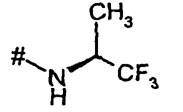 | -C(CH₃)(OH)-CH(CH₃)₂ | |
| 340 | 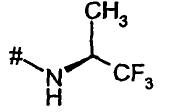 | -CH(OCH₃)-CH(CH₃)₂ | |
| 341 | 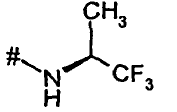 | -CH(CH₃)(OCH(CH₃)₂) | |
| 342 | 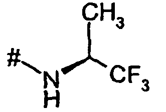 | -C(CH₃)(CF₃)-(OCH₃) | |
| 343 | 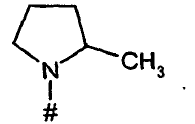 | -CH₂OH | |
| 344 | 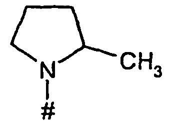 | -CH₂-O-CH₃ | |
| 345 | 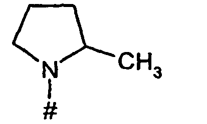 | Allyloxymethyl | |
| 346 | 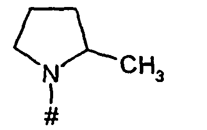 | -CH₂-O-C₂H₅ | |
| 347 | 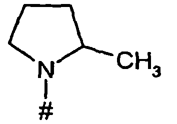 | 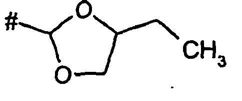 | |
| 348 | 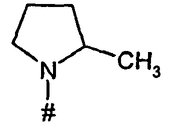 | 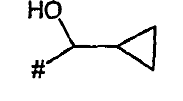 | |
| 349 | 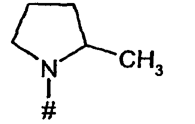 | 1-Hydroxyethyl | |
| 350 | 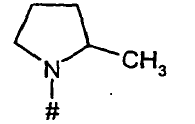 | 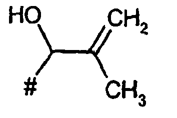 | |
| 351 | 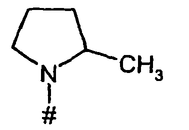 | 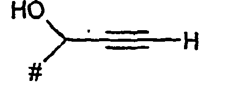 | |
| 352 | 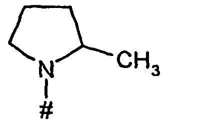 | 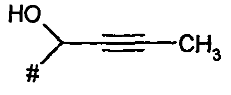 | |
| 353 | 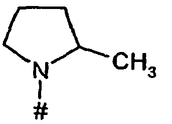 | 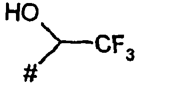 | |
| 354 | 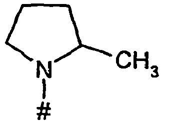 | -(CH(OH)-CH₂Cl | |
| 355 | 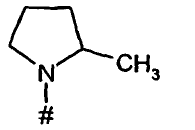 | -CH(OH)-CH(CH₃)₂ | |
| 356 | 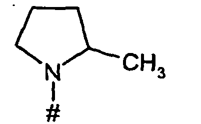 | -CH(OH)-C(CH₃)₃ | |
| 357 | 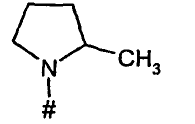 | -C(CH₃)(OH)-CµH(CH₃)₂ | |
| 358 | 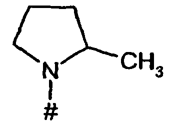 | -CH(OCH₃)-CH(CH₃)₂ | |
| 359 | 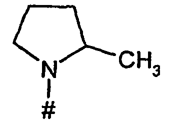 | -CH(CH₃)(OCH(CH₃)₂) | |
| 360 | 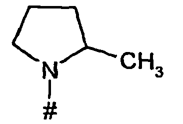 | -C(CH₃)(CF₃)-(OCH₃) | |

**Tabelle 5**

| | | | |
|---|---|---|---|
| 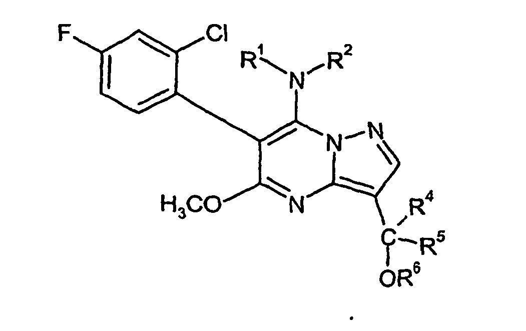 | | | |

| **Bsp. Nr.** | **N(R¹R²)** | **-C(R⁴R⁵(OR⁶)** | **logP** |
|---|---|---|---|
| 361 | 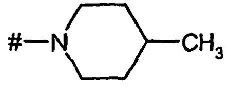 | -CH₂OH | |
| 362 | 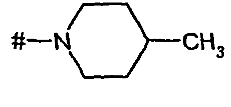 | -CH₂-O-CH₃ | |
| 363 | 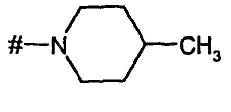 | Allyloxymethyl | |
| 364 | 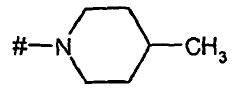 | -CH₂-O-C₂H₅ | |
| 365 | 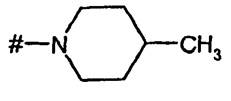 | 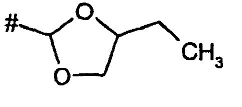 | |
| 366 | 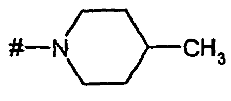 | 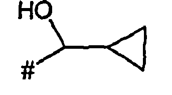 | |
| 367 | 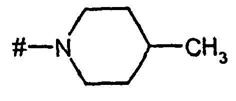 | 1-Hydroxyethyl | |
| 368 | 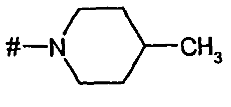 | 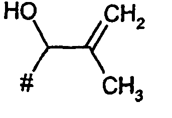 | |
| 369 | 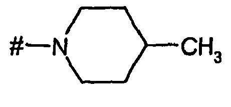 | 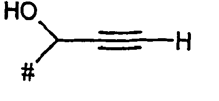 | |
| 370 | 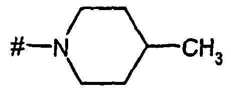 | 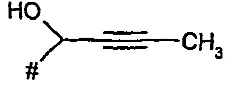 | |
| 371 | 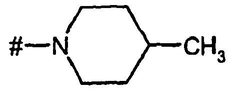 | 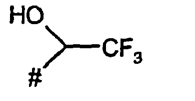 | |
| 372 | 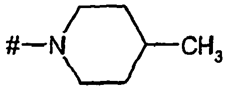 | -(CH(OH)-CH₂Cl | |
| 373 | 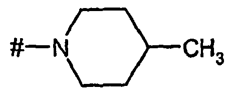 | -CH(OH)-CH(CH₃)₂ | |
| 374 | 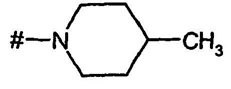 | -CH(OH)-C(CH₃)₃ | |
| 375 | 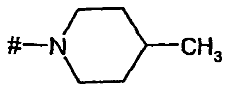 | -C(CH₃)(OH)-CH(CH₃)₂ | |
| 376 | 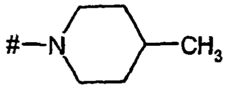 | -CH(OCH₃)-CH(CH₃)₂ | |
| 377 | 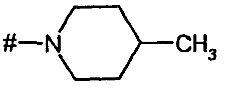 | -CH(CH₃)(OCH(CH₃)₂) | |
| 378 | 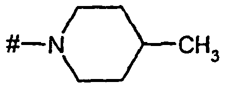 | -C(CH₃)(CF₃)-(OCH₃) | |
| 379 | 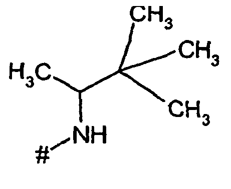 | -CH₂OH | 4.14 |
| 380 | 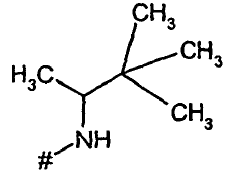 | -CH₂-O-CH₃ | |
| 381 | 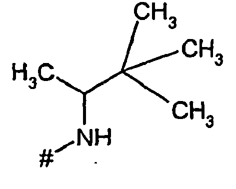 | Allyloxymethyl | |
| 382 | 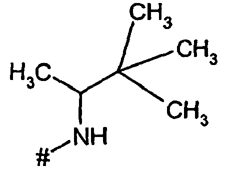 | -CH₂-O-C₂H₅ | |
| 383 | 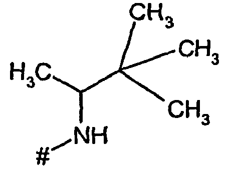 | 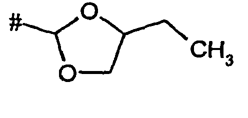 | |
| 384 | 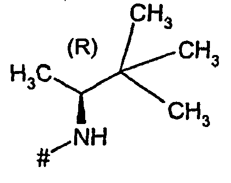 | 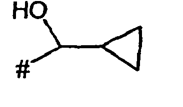 | |
| 385 | 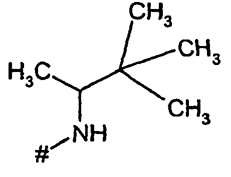 | 1-Hydroxyethyl | |
| 386 | 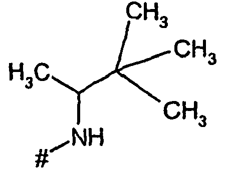 | 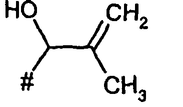 | |
| 387 | 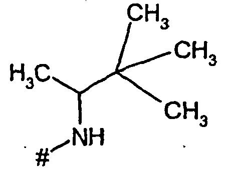 | 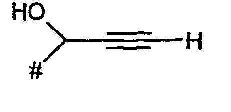 | |
| 388 | 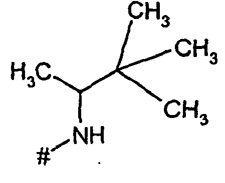 | 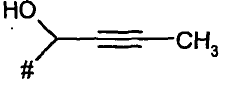 | |
| 389 | 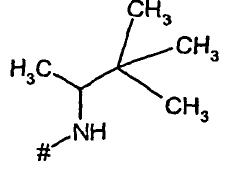 | 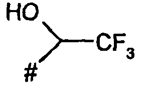 | |
| 390 | 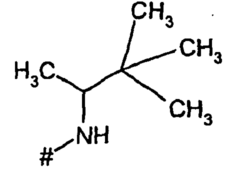 | -(CH(OH)-CH₂Cl | |
| 391 | 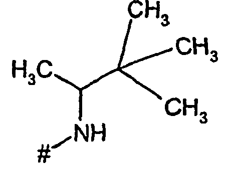 | -CH(OH)-CH(CH₃)₂ | |
| 392 | 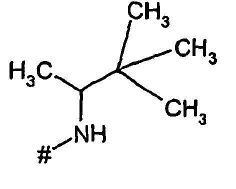 | -CH(OH)-C(CH₃)₃ | |
| 393 | 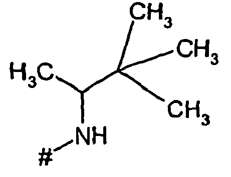 | -C(CH₃)(OH)-CH(CH₃)₂ | |
| 394 | 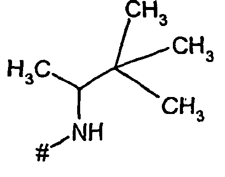 | -CH(OCH₃)-CH(CH₃)₂ | |
| 395 | 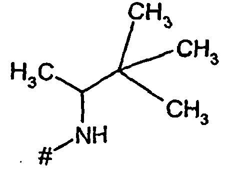 | -CH(CH₃)(OCH(CH₃)₂ | |
| 396 | 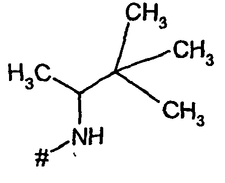 | -C(CH₃)(CF₃)-(OCH₃) | |
| 397 | 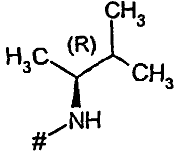 | -CH₂OH | |
| 398 | 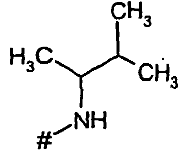 | -CH₂-O-CH₃ | |
| 399 | 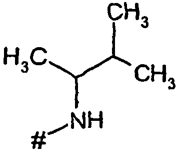 | Allyloxymethyl | |
| 400 | 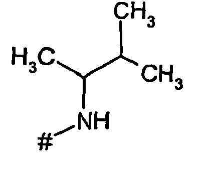 | -CH₂-O-C₂H₅ | |
| 401 | 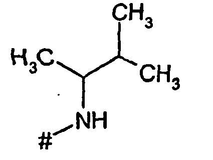 | 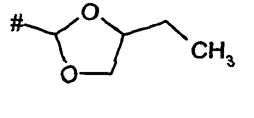 | |
| 402 | 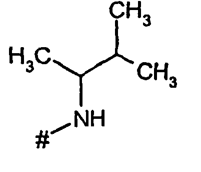 | 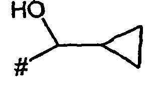 | |
| 403 | 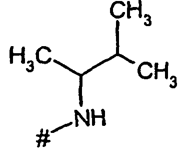 | 1-Hydroxyethyl | |
| 404 | 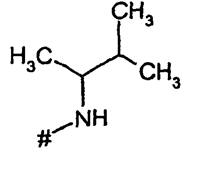 | 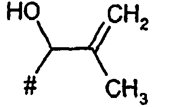 | |
| 405 | 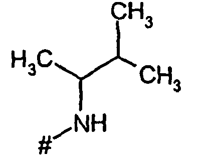 | 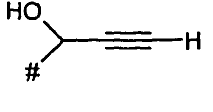 | |
| 406 | 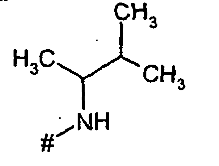 | 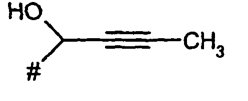 | |
| 407 | 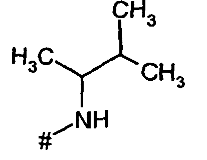 | 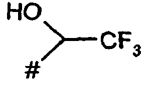 | |
| 408 | 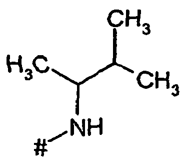 | -(CH(OH)-CH₂Cl | |
| 409 | 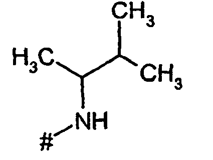 | -CH(OH)-CH(CH₃)₂ | |
| 410 | 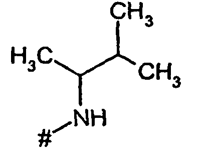 | -CH(O_{H})-C(CH₃)₃ | |
| 411 | 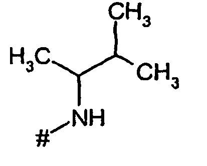 | -C(CH₃)(OH)-CH(CH₃)₂ | |
| 412 | 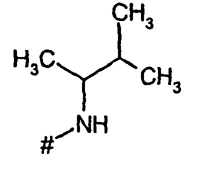 | -CH(OCH₃)-CH(CH₃)₂ | |
| 413 | 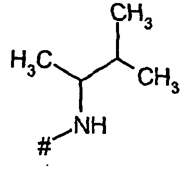 | -CH(CH₃)(OCH(CH₃)₂) | |
| 414 | 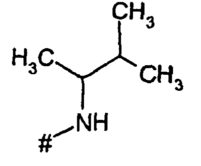 | -C(CH₃)(CF₃)-(OCH₃) | |
| 415 | 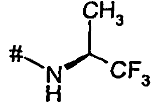 | -CH₂OH | |
| 416 | 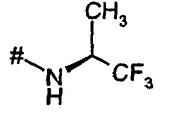 | -CH₂-O-CH₃ | |
| 417 | 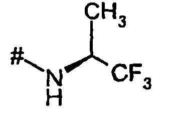 | Allyloxymethyl | |
| 418 | 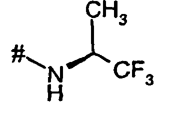 | -CH₂-O-C₂H₅ | |
| 419 | 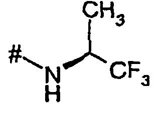 | 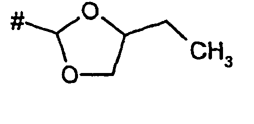 | |
| 420 | 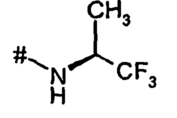 | 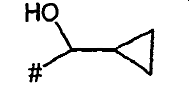 | |
| 421 | 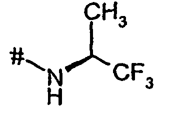 | 1-Hydroxyethyl | |
| 422 | 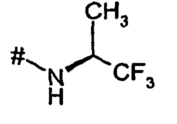 | 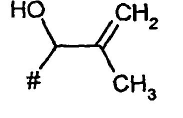 | |
| 423 | 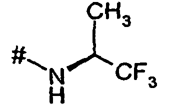 | 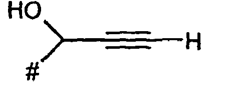 | |
| 424 | 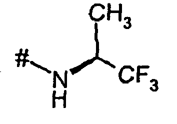 | 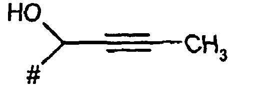 | |
| 425 | 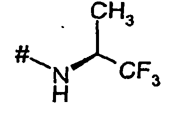 | 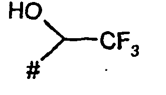 | |
| 426 | 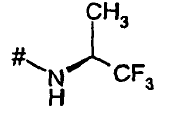 | -(CH(OH)-CH₂Cl | |
| 427 | 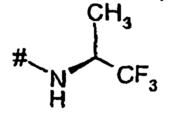 | -CH(OH)-CH(CH₃)₂ | |
| 428 | 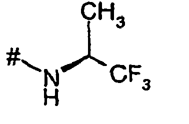 | -CH(C)H)-C(CH₃)₃ | |
| 429 | 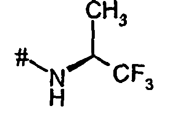 | -C(CH₃)(OH)-CH(CH₃)₂ | |
| 430 | 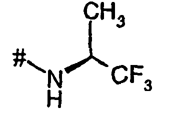 | -CH(OCH₃)-CH(CH₃)₂ | |
| 431 | 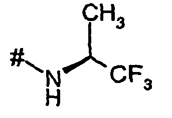 | -CH(CH₃)(OCH(CH₃)₂) | |
| 432 | 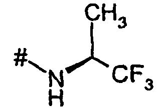 | -C(CH₃)(CF₃)-(OCH₃) | |
| 433 | 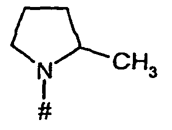 | -CH₂OH | |
| 434 | 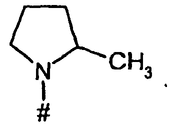 | -CH₂-O-CH₃ | |
| 435 | 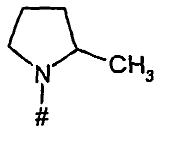 | Allyloxymethyl | |
| 436 | 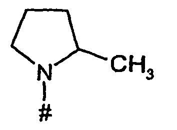 | -CH₂-O-C₂H₅ | |
| 437 | 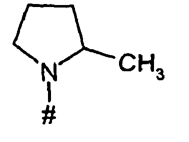 | 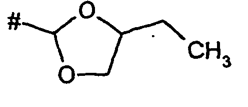 | |
| 438 | 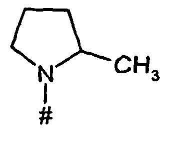 | 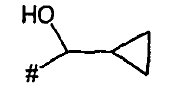 | |
| 439 | 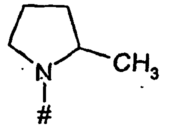 | 1-Hydroxyethyl | |
| 440 | 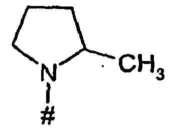 | 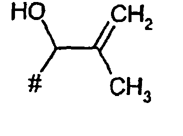 | |
| 441 | 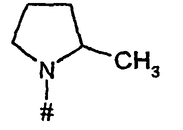 | 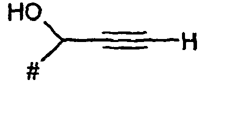 | |
| 442 | 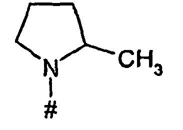 | 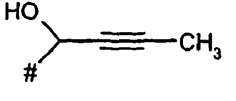 | |
| 443 | 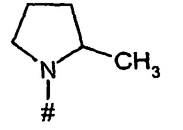 | 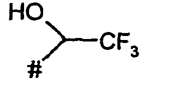 | |
| 444 | 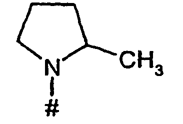 | -(CH(OH)-CH₂Cl | |
| 445 | 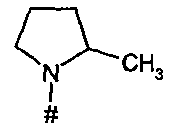 | -CH(OH)-CH(CH₃)₂ | |
| 446 | 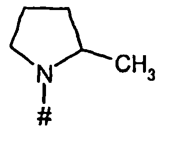 | -CH(OH)-C(CH₃)₃ | |
| 447 | 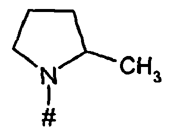 | -C(CH₃)(OH)-CH(CH₃)₂ | |
| 448 | 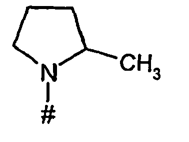 | -CH(OCH₃)-CH(CH₃)2 | |
| 449 | 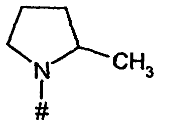 | -CH(CH₃)(OCH(CH₃)₂) | |
| 450 | 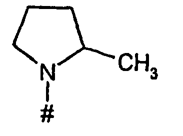 | -C(CH₃)(CF₃)-(OCH₃) | |

**Tabelle 6**

| | | | |
|---|---|---|---|
| 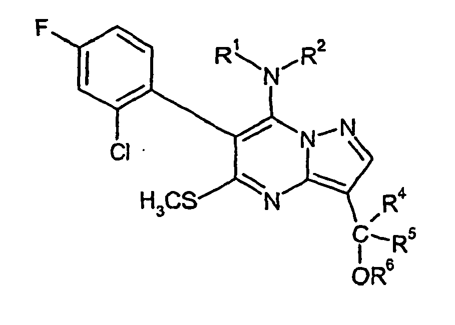 | | | |

| **Bsp. Nr.** | **N(R¹R²)** | **-C(R⁴R⁵(OR⁶))** | **logP** |
|---|---|---|---|
| 451 | 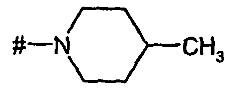 | -CH₂OH | |
| 452 | 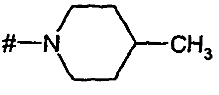 | -CH₂-O-CH₃ | |
| 453 | 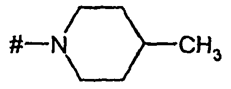 | Allyloxymethyl | |
| 454 | 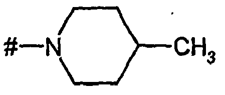 | -CH₂-O-C₂H₅ | |
| 455 | 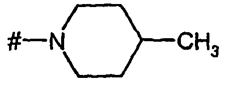 | 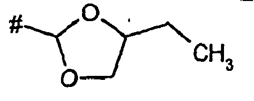 | |
| 456 | 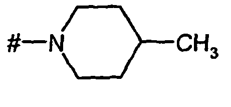 | 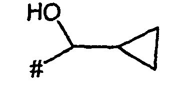 | |
| 457 | 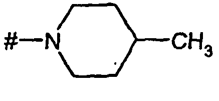 | 1-Hydroxyethyl | |
| 458 | 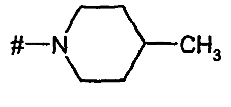 | 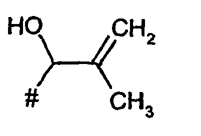 | |
| 459 | 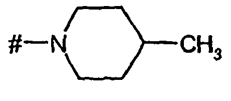 | 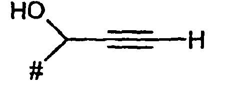 | |
| 460 | 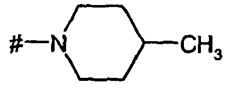 | 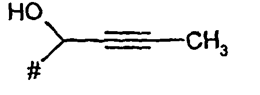 | |
| 461 | 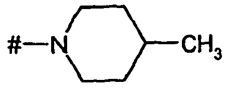 | 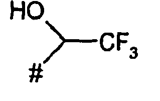 | |
| 462 | 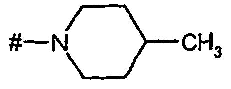 | -(CH(OH)-CH₂Cl | |
| 463 | 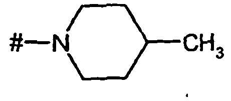 | -CH(OH)-CH(CH₃)₂ | |
| 464 | 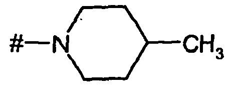 | -CH(OH)-C(CH₃)₃ | |
| 465 | 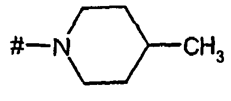 | -C(CH₃)(OH)-CH(CH₃)₂ | |
| 466 | 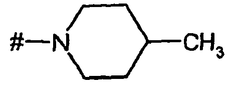 | -CH(OCH₃)-CH(CH₃)₂ | |
| 467 | 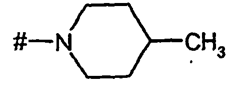 | -CH(CH₃)(OCH(CH₃)₂) | |
| 468 | 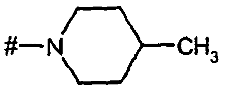 | -C(CH₃)(CF₃)-(OCH₃) | |
| 469 | 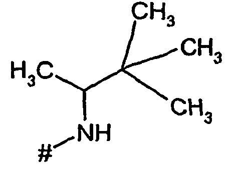 | -CH₂OH | 4.38 |
| 470 | 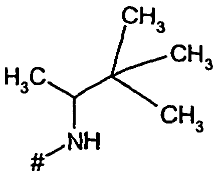 | -CH₂-O-CH₃ | |
| 471 | 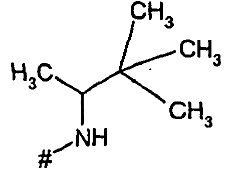 | Allyloxymethyl | |
| 472 | 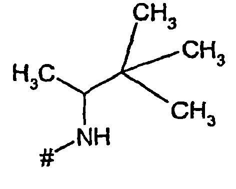 | -CH₂O-C₂H₅ | |
| 473 | 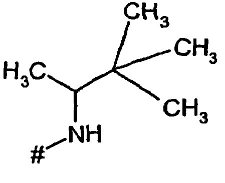 | 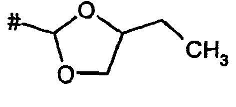 | |
| 474 | 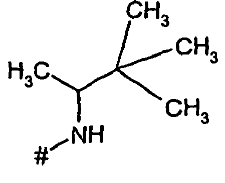 | 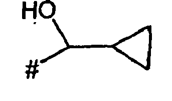 | |
| 475 | 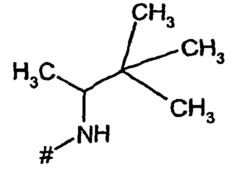 | 1-Hydroxyethyl | |
| 476 | 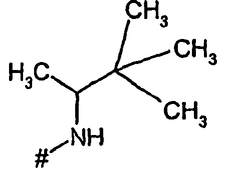 | 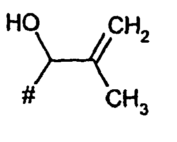 | |
| 477 | 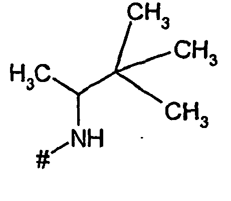 | 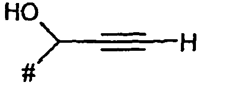 | |
| 478 | 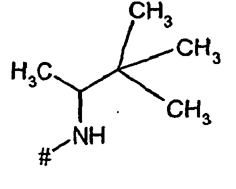 | 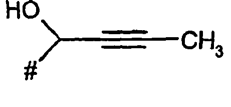 | |
| 479 | 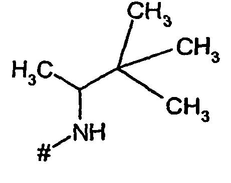 | 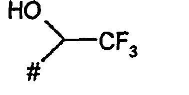 | |
| 480 | 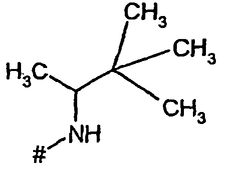 | -(CH(OH)-CH₂Cl | |
| 481 | 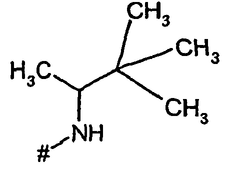 | -CH(OH)-CH(CH₃)₂ | |
| 482 | 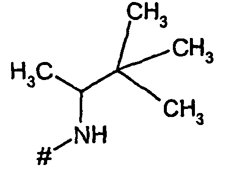 | -CH(OH)-C(CH₃)₃ | |
| 483 | 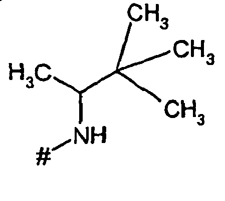 | -C(CH₃)(OH)-CH(CH₃)₂ | |
| 484 | 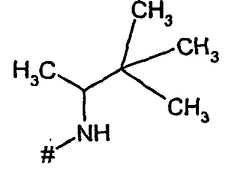 | -CH(OCH₃)-CH(CH₃)₂ | |
| 485 | 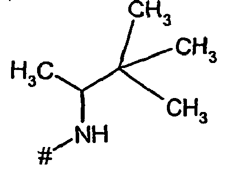 | -CH(CH₃)(OCH(CH₃)₂) | |
| 486 | 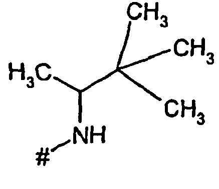 | -C(CH₃)(CF₃)-(OCH₃) | |
| 487 | 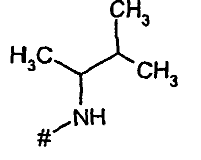 | -CH₂OH | |
| 488 | 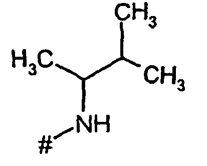 | -CH₂-O-CH₃ | |
| 489 | 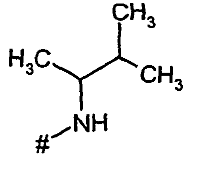 | Allyloxymethyl | |
| 490 | 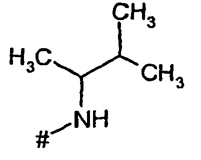 | -CH₂-O-C₂H₅ | |
| 491 | 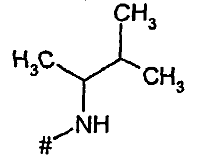 | 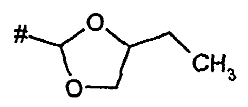 | |
| 492 | 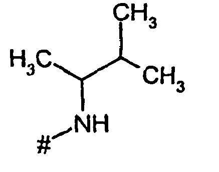 | 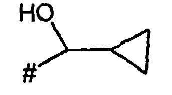 | |
| 493 | 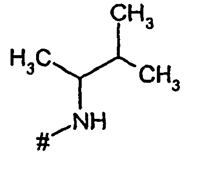 | 1-Hydroxyethyl | |
| 494 | 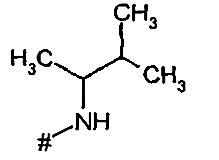 | 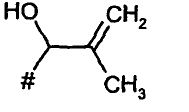 | |
| 495 | 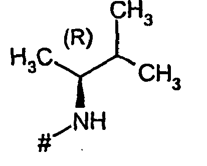 | 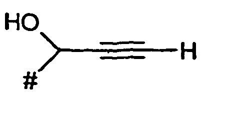 | |
| 496 | 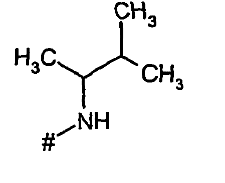 | 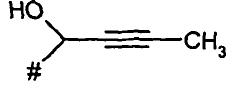 | |
| 497 | 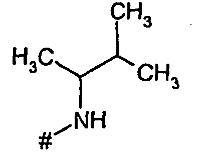 | 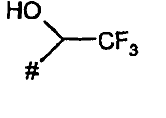 | |
| 498 | 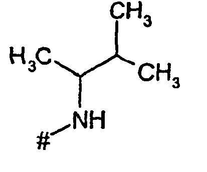 | -(CH(OH)-CH₂Cl | |
| 499 | 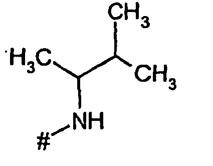 | -CH(OH)-CH(CH₃)₂ | |
| 500 | 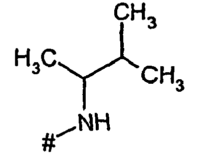 | -CH(OH)-C(CH₃)₃ | |
| 501 | 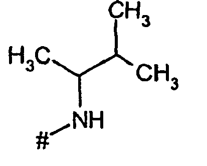 | -C(CH₃)(OH)-CH(CH₃)₂ | |
| 502 | 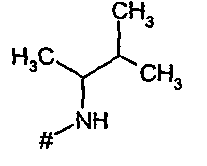 | -CH(OCH₃)-CH(CH₃)₂ | |
| 503 | 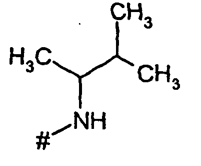 | -CH(CH₃)(OCH(CH₃)₂) | |
| 504 | 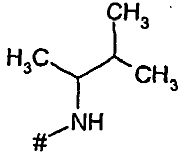 | -C(CH₃)(CF₃)-(OCH₃) | |
| 505 | 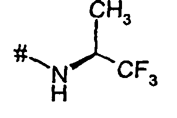 | -CH₂OH | |
| 506 | 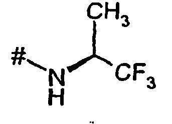 | -CH₂O-CH₃ | |
| 507 | 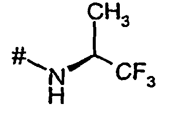 | Allyloxymethyl | |
| 508 | 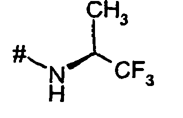 | -CH₂-O-C₂H₅ | |
| 509 | 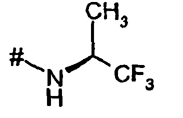 | 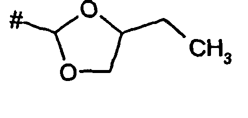 | |
| 510 | 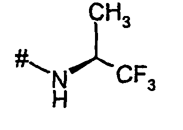 | 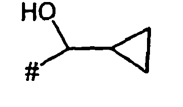 | |
| 511 | 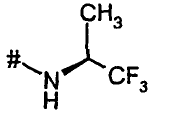 | 1-Hydroxyethyl | |
| 512 | 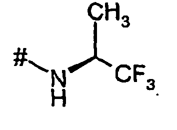 | 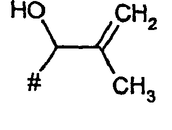 | |
| 513 | 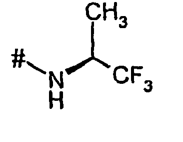 | 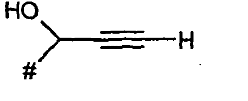 | |
| 514 | 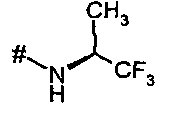 | 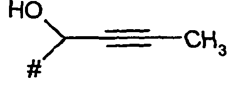 | |
| 515 | 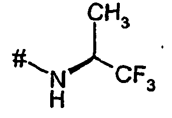 | 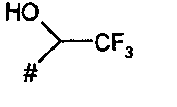 | |
| 516 | 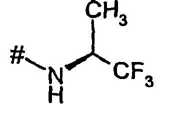 | -(CH(OH)-CH₂Cl | |
| 517 | 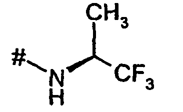 | -CH(OH)-CH(CH₃)₂ | |
| 518 | 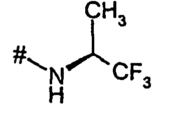 | -CH(OH)-C(CH₃)₃ | |
| 519 | 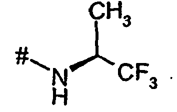 | -C(CH₃)(OH)-CH(CH₃)₂ | |
| 520 | 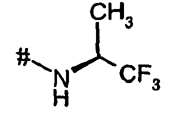 | -CH(OCH₃)-CH(CH₃)₂ | |
| 521 | 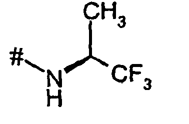 | -CH(CH₃)(OCH(CH₃)₂) | |
| 522 | 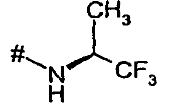 | -C(CH₃)(CF₃)-(OCH₃) | |
| 523 | 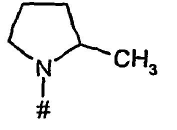 | -CH₂OH | |
| 524 | 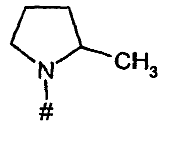 | -CH₂-O-CH₃ | |
| 525 | 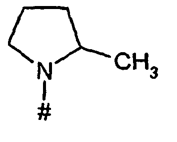 | Allyloxymethyl | |
| 526 | 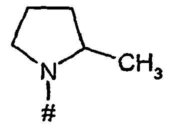 | -CH₂-O-C₂H₅ | |
| 527 | 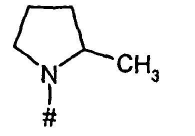 | 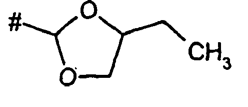 | |
| 528 | 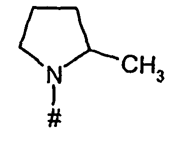 | 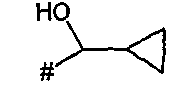 | |
| 529 | 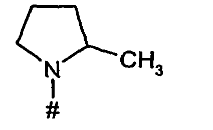 | 1-Hydroxyethyl | |
| 530 | 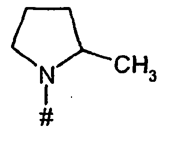 | 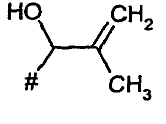 | |
| 531 | 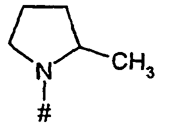 | 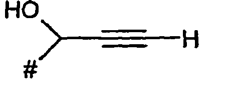 | |
| 532 | 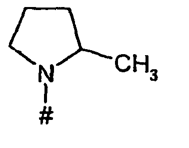 | 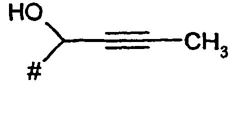 | |
| 533 | 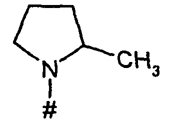 | 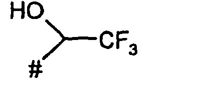 | |
| 534 | 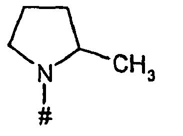 | -(CH(OH)-CH₂Cl | |
| 535 | 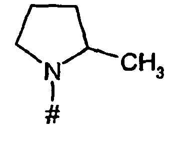 | -CH(OH)-CH(CH₃)₂ | |
| 536 | 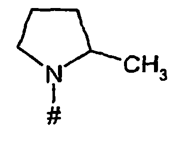 | CH(OH)-C(CH₃)₃ | |
| 537 | 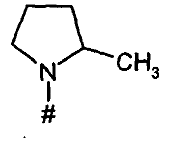 | -C(CH₃)(OH)-CH(CH₃)₂ | |
| 538 | 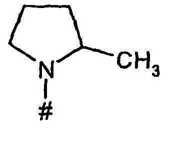 | -CH(OCH₃)-CH(CH₃)₂ | |
| 539 | 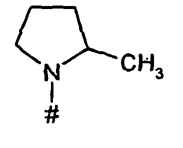 | -CH(CH₃)(OCH(CH₃)₂) | |
| 540 | 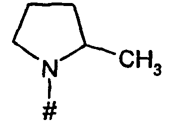 | -C(CH₃)(CF₃)-(OCH₃) | |

### Herstellung von Ausgangssubstanzen

### Beispiel 541

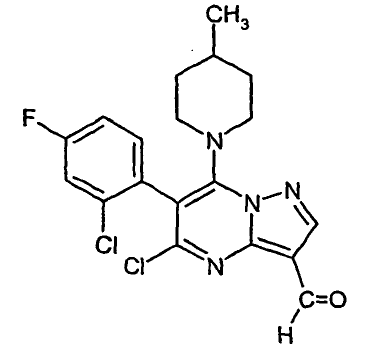

### Verfahren (e):

In ein Gemisch aus 37,2 mmol 5,7-Dihydroxy-6-(2-chlor-4-fluor-phenyl)-pyrazolo[1,5-a]pyrimidin und 372 mmol Phosphoroxychlorid werden bei 0°C unter Rühren 41 mmol N,N-Dimethylformamid eingetropft. Nach beendeter Zugabe wird zunächst 12 Stunden bei Raumtemperatur gerührt und dann 6 Stunden auf Rückflusstemperatur erhitzt. Dabei werden 37,2 mmol Phosphorpentachlorid portionsweise zugegeben. Nach dem anschließenden Abkühlen auf Raumtemperatur wird das Reaktionsgemisch auf Eiswasser gegeben. Das entstehende Gemisch wird dreimal mit Essigsäureethylester extrahiert. Die vereinigten organischen Phasen werden über Natriumsulfat getrocknet und dann durch Zugabe von Cyclohexan auf das doppelte Volumen gebracht. Die Lösung wird über Kieselgel filtriert und dann unter vermindertem Druck eingeengt. Man erhält auf diese Weise 3-Formyl-5,7-dichlor-6(2-chlor-4-fluor-phenyl)-pyrazolo[1,5-a]-pyrimidin in Form eines Rohproduktes, das ohne zusätzliche Reinigung für die weitere Synthese eingesetzt wird.

Ein Gemisch aus 2,2 mmol 3-Formyl-5,7-dichlor-6-(2-chlor-4-fluor-phenyl)-pyrazolo[1,5-a]-pyrimidin und 50 ml Dichlormethan wird bei Raumtemperatur unter Rühren mit 2,4 mmol 4-Methyl-piperidin und mit 2,4 mmol Triethylamin versetzt. Das Gemisch wird 15 Stunden bei Raumtemperatur gerührt und dann auf Wasser gegossen. Die organische Phase wird abgetrennt, und die wässrige Phase wird dreimal mit Essigsäureethylester extrahiert. Die vereinigten organischen Phase werden über Natriumsulfat getrocknet und dann unter vermindertem Druck eingeengt. Der verbleibende Rückstand wird mit Cyclohexan : Essigsäureethylester = 9 : 1 an Kieselgel chromatographiert. Man erhält auf diese Weise 3-Formyl-5-chlor-6-(2-chlor-4-fluorphenyl)-7-(4-methyl-piperidine)-pyrazolo[1,5-a]pyrimidin in Form eines gelben Öles, das allmählich kristallisiert.
log P_{(pH}= 2.3) = 4,53

### Beispiel 542

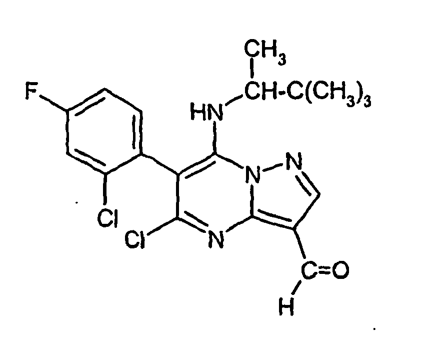

### Verfahren (c):

Eine Lösung von 11 mmol 3-Cyano-5-chlor-6-(2-chlor-4-fluor-phenyl)-7-(3,3-dimethyl-but-2-yl-amino)-pyrazolo[1,5-a]pyrimidin in 150 Dichlormethan wird bei -50°C unter Argonatmosphäre und unter Rühren mit 12,2 mmol Di-isobutyl-aluminiumhydrid (als 1-molare Lösung in Toluol) versetzt. Nach beendeter Zugabe wird zunächst noch 30 Minuten bei -50°C nachgerührt.

Dann wird bei 0°C mit gesättigter, wässriger Ammoniumchlorid Lösung versetzt und 2 Stunden bei 0°C gerührt. Anschließend wird 1-normale Salzsäure hinzugefügt und dann die organische Phase abgetrennt. Die wässrige Phase wird dreimal mit Dichlormethan extrahiert. Die vereinigten organischen Phase werden nacheinander mit gesättigter, wässriger NatriumhydrogencarbonatLösung und mit gesättigter, wässriger Kochsalz-Lösung gewaschen, dann über Natriumsulfat getrocknet und anschließend unter vermindertem Druck eingeengt. Der verbleibende Rückstand wird mit Methyl-tert-butyl-ether : Petrolether = 3 : 1 an Kieselgel chromatographiert. Man erhält auf diese Weise 6,4 mmol / 58 % der Theorie) an 3 Formyl-5-chlor-6-(2-chlor-4-fluor phenyl)-7-(3,3-dimethyl-but-2-yl-amino)-pyrazolo[1,5-a]pyrimidin.
log P = 4,43 / 4,47 (Atropisomere)

### Beispiel 543

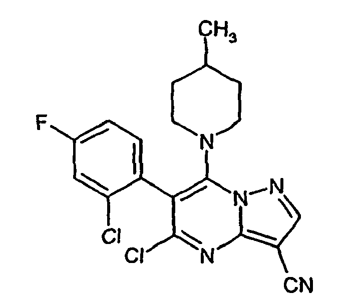

### Verfahren (f)

Eine Lösung von 5 mmol 3-Cyano-5,7-dichlor-6-(2-chlor-4-fluor-phenyl)-pyrazolo[1,5-a]pyrimidin in 10 ml Acetonitril wird bei Raumtemperatur unter Rühren in ein Gemisch aus 30 ml Acetonitril, 5 mmol Kaliumcarbonat und 5 mmol 4-Methyl-piperidin eingetropft. Das Reaktionsgemisch wird 15 Stunden bei Raumtemperatur gerührt und dann in Wasser eingerührt. Das entstehende Gemisch wird dreimal mit Essigsäureethylester extrahiert. Die vereinigten organischen Phasen werden über Natriumsulfat getrocknet und dann unter vermindertem Druck eingeengt. Man erhält auf diese Weise 4,28 mmol (86 % der Theorie) an 3-Cyano-5-chlor-6-(2-chlor-4-fluor-phenyl)-7-(4-methylpiperidino)-pyrazolo[1,5-a]pyrimidin.
Log P_{(pH = 2,3)} = 4,88

### Beispiel 544

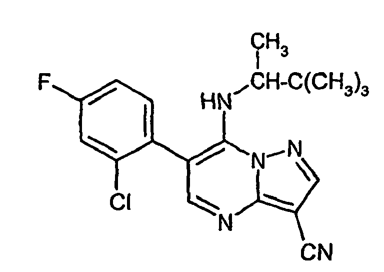

Die Herstellung der Verbindung der oben angegebenen Formel erfolgt nach der im Beispiel 6 angegebenen Methode.
HPLC: log P = 4,78

### Beispiel 545

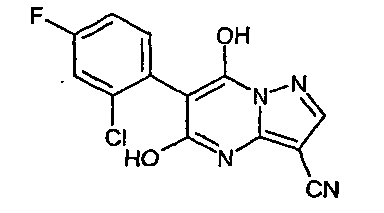

### Verfahren (h):

48 g (0,184 Mol) 2-Chlor-4-fluor-phenylmalonsäuredimethylester werden mit 19,91 g (0,184 Mol) 4-Cyano-5-aminopyrazol und mit 37,55 g (0,203 Mol) Tri-n-butylamin vermischt und 6 Stunden bei 180°C gerührt. Das bei der Reaktion entstehende Methanol wird kontinuierlich abdestilliert. Anschließend wird das Reaktionsgemisch auf Raumtemperatur abgekühlt. Bei 95°C und 1 mbar werden flüchtige Komponenten abdestilliert. Man erhält als Rückstand 6-(2-Chlor-4-fluor-phenyl)-5,7-dihydroxypyrazolo[1,5-a]pyrimidin-3-carbonitril in Form eines Rohproduktes, das ohne zusätzliche Reinigung für die weitere Synthese verwendet wird.

### Beispiel 546

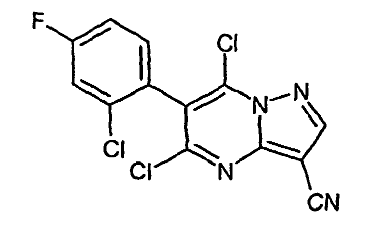

### Verfahren (g):

Das gemäß Beispiel 8 erhaltene 6-(2-Chlor-4-fluor-phenyl)-5,7-dihydroxy-pyrazolo[1,5-a]pyrimidin-3-carbonitril wird im Rohzustand in 367,3 g (2,395 Mol) Phosphoroxychlorid gelöst. Man gibt bei Raumtemperatur 31,95 g (0,153 Mol) Phosphorpentachlorid in Portionen dazu. Dann kocht man die Mischung 4 Stunden unter Rückfluss. Die flüchtigen Komponenten werden unter vermindertem Druck abdestilliert. Der Rückstand wird mit Dichlormethan versetzt und mit Wasser gewaschen. Die organische Phase wird über Natriumsulfat getrocknet und unter vermindertem Druck eingeengt. Der Rückstand wird mit 3 Teilen Cyclohexan und 1 Teil Essigsäureethylester als Laufmittel an Kieselgel chromatographiert. Man erhält 21 g 95,7 %iges 3-Cyano-5,7-dichlor-6-(2-chlor-4-fluor-phenyl)-pyrazolo[1 ,5-a]pyrimidin.
HPLC: logP = 3,48
¹H-NMR (DMSO-d6, Tetramethylsilan): δ = 7.44-7.52 (1H); 7.62-7.66 (1H); 7.71-7.77 (1H); 9.03 (1H) ppm.

### Verwendungsbeispiele

### Beispiel A

Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykotether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100% relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die in den Beispielen 1 und 2 aufgeführten erfindungsgemäßen Verbindungen bei einer Aufwandmenge von 100 g/ha einen Wirkungsgrad von über 90 %.

### Beispiel B

Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die in den Beispielen 1 und 2 aufgeführten erfindungsgemäßen Verbindungen bei einer Aufwandmenge von 500 g/ha einen Wirkungsgrad von über 90 %.

### Beispiel C

Podosphaera-Test (Apfel) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die erfindungsgemäßen Stoffe der Beispiele 1 und 2 bei einer Aufwandmenge von 100 g/ha einen Wirkungsgrad von über 90 %.

## Patentansprüche

1. Pyrazolopyrimidine der Formel 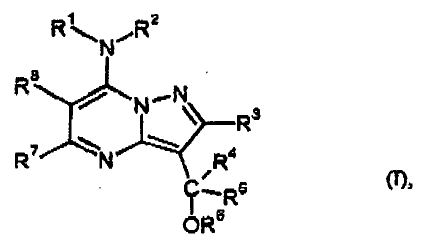 in welcher
R¹ für Alkyl mit 1 bis 6 Kolenstoffatomen steht, das einfach bis fünffach, gleichartig oder verschieden substituiert sein kann durch Halogen. Cyano, Hydroxy, Alkoxy mit 1 bis 4 Kohlenstoffatomen und/oder Cycloalkyl mit 3 bis 6 Kohlenstoffatomen, oder
R¹ für Alkenyl mit 2 bis 6 Kohlenstoffatomen steht, das einfach bis dreifach gleichartig oder verschieden substituiert sein kann durch Halogen, Cyano, Hydroxy, Alkoxy mit bis 4 Kohlensgoffatomen und/oder Cycloalkyl mit 3 bis 6 Kohlenstoffatomen, oder
R¹ für Alkinyl mit 3 bis 6 Kohlenstoffatomen steht, das einfach bis dreifach, gleichartig oder verschieden substituiert sein kann durch Halogen, Cyano, Alkoxy mit bis 4 Kohlenstoffatomen und/oder Cycloalkyl mit 3 bis 6 Kohlenstoffatomen oder
R¹ für Cycloalkyl mit 3 bis 6 Kohlenstoffatomen steht, das einfach bis dreifach, gleichartig oder verschieden substituiert sein kann durch Halogen und/oder Alkyl mit 1 I bis 4 Kohlenstoffatomen, oder
R¹ für gesättigtes oder ungesättigtes Heterocyclyl mit 5 oder 6 Ringgliedern und 1 bis 3 Heteroatomen, wie Stickstoff. Sauerstoff und/oder Schwefel, steht, wobei das Hetero-cyclyl einfach oder zweifach substituiert sein kann durch Halogen, Alkyl mit 1 bis 4 Kohlenstoffatomen. Cyano, Nitro und/oder Cycloalkyl mit 3 bis 6 Kohlenstoffatomen,
R² für Wasserstoff oder Alkyl mit 1 bis 4 Kohlenstoffatomen steht, oder
R¹ und R² gemeinsam mit dem Stickstoffatom, an das sie gebunden sind, für einen gesättigten. oder ungesättigten heterocyclischen Ring mit 3 bis 6 Ringgliedern stehen, wobei der Heterocyclus ein weiteres Stickstoff-, Sauerstoff oder Schwefelatom als Ringglied enthalten kann und wobei der Heterocyclus bis zu dreifach substituiert sein kann durch "Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
R³ für Wasserstoff. Fluor, Chlor, Brom, Iod, Alkyl mit 1 bis 4 Kohlenstoffatomen, Halogenalkyl mit 1 bis 4 Kohlenstoffatomen und 1 bis 9 Halogenatomen oder für Cycloalkyl mit 3 bis 6 Kohlenstoffatomen steht,
R⁴ für Wasserstoff, Alkyl mit 1 bis 4 Kohlenstoffatomen, Halogenalkyl mit 1 bis 4 Kohlenstoffatomen im Alkylteil, Cycloalkyl mit 3 bis 6 Kohlenstoffatomen, Alkoxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen, Alkinyl mit 2 bis 3 Kohlenstoffatomen oder Benzyl steht,
R⁵ für Wasserstoff, Alkyl mit 1 bis 4 Kohlenstoffatomen, Halogenalkyl mit 1 bis 4 Kohlenstoffatomen im Alkylteil, Cycloalkyl mit 3 bis 6 Koblenstoffatomen, Alloxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen, Alkinyl mit 2 bis 5 Kohlenstoffatomen oder Benzyl steht,
R⁶ für Wasserstoff. Alkyl mit 1 bis 4 Kohlenstoffatomen, Alkoxyalkyl mit 1 oder 2 Kohlenstoffatomen im Alkoxyteil und 1 bis 4 Kohlenstoffatomen im Alkylteil, Alkenyl mit 2 bis 5 Kohlenstoffatomen. Alkinyl mit 2 bis 5 Kohlenstoffatomen oder Benzyl steht, oder
R⁵ und -OR⁶ gemeinsam für einen Rest der Formel 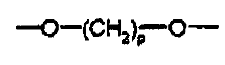 stehen,
worin
p für 2, 3 oder 4 steht und
1 oder 2 Wasserstoffatome ersetzt sein können durch Methyl. Ethyl, Hydroxy, Methoxy, Ethoxy, Hydroxymethyl, Methoxymethyl oder Ethoxymethyl,
R⁷ für Fluor, Chlor, Brom, CN, Methyl, Alkoxy mit 1 bis 4 Kohlenstoffatomen, Alkylthio mit 1 bis 4 Kohlenstoffatomen, Alkylsulfinyl mit 1 bis 4 Kohlenstoffatomen oder Alkylsulfonyl mit 1 bis 4 Kohlenstoffatomen steht, und
R⁸ für Phenyl steht, das einfach bis vierfach, gleichartig oder verschieden substituiert sein kann durch Halogen, Cyclo, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils garadkettiges oder verzweigtes Alkenyl oder Alkenyl mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Koblenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2.3-Position verknüpftes 1,3-Propandiyl, 1,4-Butandiyl, Methylendioxy (-O-CH₂-O-) oder 1,2-Ethylendioxy (-O-CH₂-CH₂O-), wobei diese Reste einfach bis mehrfach, gleichartig oder verschieden substituiert sein können durch Halogen. Alkyl mit 1 bis 4 Kohlenstoffatomen und/oder Halogenalkyl mit 1 bis 4 Kohlenstoffatomen und 1 bis 9 gleichen oder verschiedenen Halogenatomen.

2. Pyrazolopyrimidine der Formel (I) gemäß Anspruch 1, wobei
R¹ für einen Rest der Formel 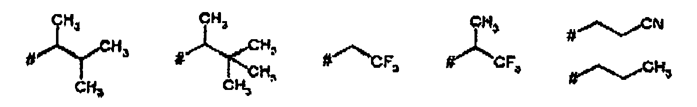 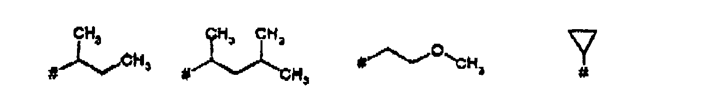 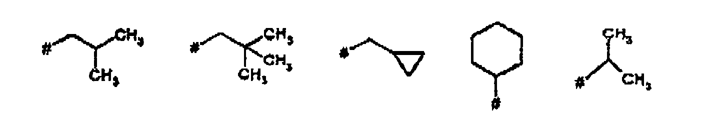 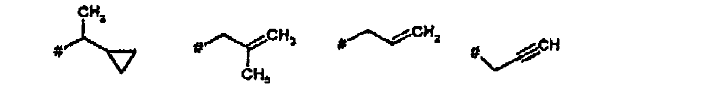 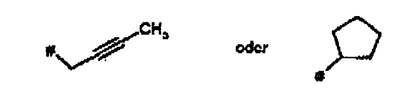 steht,
wobei # die Anknüpfungsstelle markiert und wobei für diejenigen Reste, die in optisch aktiver Form vorliegen können, jedes der möglichen Stereoisomeren oder auch Gemische davon vorliegen können,
R² für Wasserstoff, Methyl, Ethyl oder Propyl steht, oder
R¹ und R² gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl oder Tetrahydro-1(2H)-pyridazinyl stehen, wobei diese Reste durch 1 bis 3 Fluoratome, 1 bis 3 Methylgruppen und/oder Trifluormethyl substituiert sein können,
oder
R¹ und R² gemeinsam mit dem Stickstoffatom, an das sie gebunden sind, für einen Rost der Formel 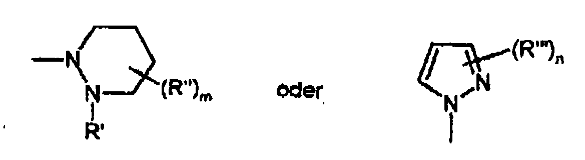 stehen
worin
R' für Wasserstoff oder Methyl steht,
R" für Methyl, Ethyl, Fluor, Chlor oder Trifluormethyl steht,
m für die zahlen 0, 1, 2 oder 3 steht, wobei R" für gleiche oder verschiedene Reste steht, wenn m für 2 oder 3 steht,
R"' für Methyl Ethyl, Fluor, Chlor oder Trifluormethyl steht
und
n für die Zahlen 0, 1, 2 oder 3 steht, wobei R'" für gleiche oder verschiedene Reste steht, wenn n für 2 oder 3 steht,
R³ für Wasserstoff Fluor, Chlor, Brom, Iod, Methyl, Ethyl Isopropyl, Cyclopropyl, Cyclobutyl. Cyclopentyl Cyclohexyl, Trifluormethyl. 1-Trifluormethyl-2,2,2-trifluorethyl oder Heptafluorisopropyl steht,
R⁴ für Wasserstoff Methyl, Ethyl, Propyl, Methoxymethyl, Methoxyethyl, Alkenyl mit 3 oder 4 Kohlenstoffatomen. Alkinyl mit 3 oder 4 Kohlenstoffatomen oder Benzyl steht,
R⁵ für Wasserstoff, Methyl, Ethyl, Propyl, Methoxymethyl, Methoxyethyl, Alkenyl mit 3 oder 4 Kohlenstoffatomen, Alkinyl mit 3 oder 4 Kohlenstoffatomen oder Benzyl steht,
R⁶ für Wasserstoff Methyl, Ethyl. Propyl, Methoxymethyl, Methoxyethyl, Alkenyl mit 3 oder 4 Kohtenstoffatomen, Alkinyl mit 3 oder 4 Kohlenstoffatomen oder Benzyl steht, oder
R⁵ und OR⁶ gemeinsam für einen Rest der Formel 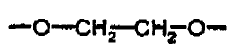 stehen, worin 1 oder 2 Wasserstoffatome ersetzt sein können durch Methyl, Ethyl, Hydroxy, Methoxy, Ethoxy, Hydroxymethyl, Methoxymethyl oder Ethoxymethyl,
R⁷ für Fluor, Chlor, Brom, Methoxy, Ethoxy, Methylthio, Methylsulfinyl oder Methylsulfonyl steht, und
R⁸ für Phenyl steht, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i·Propoxy, Methylthio, Ethylthio, n-oder i-Prapylthio, Methylsulfinyl, Ethylsulfinyl, Methylsulfonyl, Ethylsulfonyl, Allyloxy, Propargyloxy, Trifluormethyl, Trifluorethyl, Difluormethoxy. Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl. Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (O-CH₂-CH₂-O), wobei diese Reste einfach oder mehrfach gleichartig oder verschieden substituiert sein können durch Fluor, Chlor, Methyl, Ethyl, n-Propyl, i-Propyl und/oder Trifluormethyl.

3. Pyrazolopyrimidine der Formel (I) gemäß einem oder mehreren der Ansprüche bis 2, wobei
R⁷ für Fluor, Chlor, Brom, CN, Methyl, Methoxy oder Methylthio steht und
R⁸ für 2,4-, 2,5- oder 2,6-disubstituertes Phenyl, oder 2-substituiertes Phenyl oder für 2,4,6-trisubstituiertes Phenyl steht, wobei die Substituenten gewählt sind aus der Gruppe
Fluor, Chlor, Brom, Cyano, Nitro, Formyl, Methyl, Ethyl, n- oder i-Propyl, n-, i-, s- oder t-Bytyl, Allyl, Propargyl, Methoxy, Ethoxy, n- oder i-Propoxy, Methylthio, Ethylthio, n-oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl. Acetyloxy, Methoxycarbonyl. Ethoxycarbonyl, Hydroxymethyl,Hydroximinoçethyl, Methoximinomethyl, Ethoximinomethyl, Methoximinoethyl, Ethoximinoethyl, Cyclopropyl, Cyclobutyl, Cyclopentyl. Cyclohexyl,
in 2,3-Position verknüpftes 1,3-Propandiyl, Methylendioxy (-O-CH₂-O-) und 1,2-Ethylendioxy (O-CH₂-CH2-O), wobei diese Reste einfach oder mehrfach, gleichartig oder verschieden substituiert sein können durch Fluor, Chlor, Methyl, Ethyl, n-Propyl, i-Propyl und/oder Trifluormethyl.

4. Verfahren zur Herstellung von Pyrazolopyrimidinen der Formel (I) gemäß einem oder mehreren der Ansprüche 1 bis 3, **dadurch gekennzeichnet, dass** man
a) Pyrazolopyrimidine der Formel 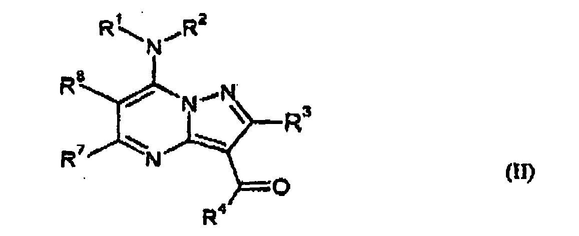 in welcher
R¹, R**²,** R³, R⁴, R⁷ und R⁸ die oben angegebenen Bedeutungen haben,
entweder
a) mit Diisobtuyl-aluminiumbydrid in Gegenwart von wässriger Ammoniumchlorid-Lösung sowie in Gegenwart eines organischen Verdünnungsmittels umsetzt,
oder mit Natriumborhydrid in Gegenwart eines Verdünnungsmittels umsetzt,
oder
β) mit Grignard-Verbindungen der Formel
R⁹-Mg-X (III)
in welcher
R⁹ für Alkyl, Alkoxyalkyl, Alkenyl, Alkinyl oder Benzyl steht und
X für Chlor, Brom oder Iod steht,
in Gegenwart eines Katalysators und in Gegenwart eines Verdünnungsmittels umsetzt,
und gegebenenfalls die nach der Variante (α) oder (β) erhaltenen Pyrazolopyrimidine der Formel 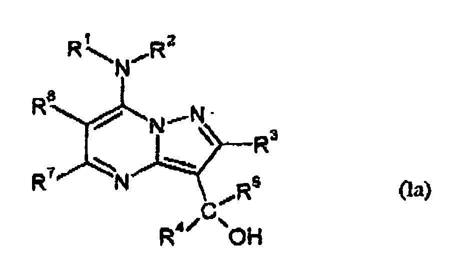 in welcher
R¹, R², R³, R⁴, R³, R⁷ und R⁸ die oben angegebenen Bedeutungen haben,
mit Verbindungen der Formel
R¹⁰-X¹ (IV)
in welcher
R¹⁰ für Alkyl, Alkoxyalkyl, Alkenyl, Alkinyl oder Benzyl steht, und
X¹ für Chlor, Brom lod oder den Rest R¹⁰O-SO₂-O- steht,
gegebenenfalls in Gegenwart einer Base und gegebenenfalls in Gegenwart eines Verdünnungsmittels umsetzt,
oder
b) Pyrazolopyrimidine der Formel 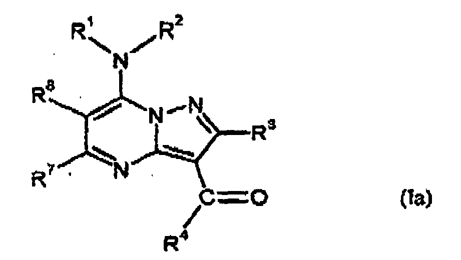
in welcher
R¹ R², R³, R⁴, R⁷ und R⁸ die oben angegebenen Bedeutungen haben,
mit Diolen der Formel
HO-(CH ₂)ₚ-O (V)
in welcher
p für ganze Zahlen von 1 bis 5 steht und
1 bis 3 Wasserstoffatome ersetzt sein können durch Methyl, Ethyl, Hydroxy, Methoxy, Ethoxy, Hydroxymethyl, Methoxymethyl oder Ethoxymethyl,
in Gegenwart eines Katalysators und gegebenenfalls in Gegenwart eines Verdünnungsmittels umsetzt.

5. Mittel zur Bekämpfung von unerwünschten Mikroorganismen, **gekennzeichnet durch** einen Gehalt an mindestens einem Pyrazolopyrimidin der Formel (1) gemäß einem oder mehreren der Ansprüche 1 bis 3 neben Streckmitteln und/oder oberflächenaktiven Stoffen.

6. Mittel gemäß Anspruch 5, enthaltend mindestens einen weiteren fungiziden oder insektiziden wirkstoff.

7. Verwendung von Pyrazolopyrimidinen der Formel (I) gemäß einem oder mehreren der Ansprüche 1 bis 3 zur Bekämpfung von unerwünschten Mikroorganismen im Pflanzen- und Materialschutz.

8. Verfahren zur Bekämpfung von unerwünschten Mikroorganismen im Pflanzen-und Materialschutz., **dadurch gekennzeichnet, dass** man Pyrazolopyrimidine der Formel (I) gemäß einem oder mehreren der Ansprüche 1 bis 3 auf die unerwünschten Mikroorganismen und/oder deren Lebensraum ausbringt.

9. Verfahren zur Herstellung von Mitteln zur Bekämpfung von unerwünschten Mikroorganismen, **dadurch gekennzeichnet dass** man Pyrazopyrimidine der Formel (I) gemäß einem oder mehreren der Ansprüche 1 bis 3 mit Streckmitteln und/oder oberflächenaktiven Stoffen vermischt.

## Claims

1. Pyrazolopyrimidines of the formula 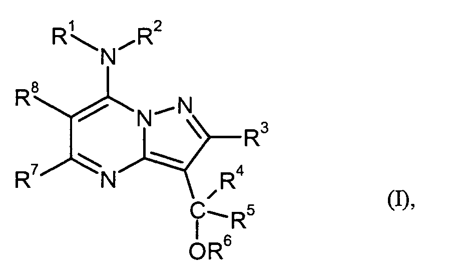 in which
R¹ represents alkyl having 1 to 6 carbon atoms which may be mono- to pentasubstituted by identical or different substituents from the group consisting of halogen, cyano, hydroxy, alkoxy having 1 to 4 carbon atoms and cycloalkyl having 3 to 6 carbon atoms, or
R¹ represents alkenyl having 2 to 6 carbon atoms which may be mono- to trisubstituted by identical or different substituents from the group consisting of halogen, cyano, hydroxy, alkoxy having 1 to 4 carbon atoms and cycloalkyl having 3 to 6 carbon atoms, or
R¹ represents alkynyl having 3 to 6 carbon atoms which may be mono- to trisubstituted by identical or different substituents from the group consisting of halogen, cyano, alkoxy having 1 to 4 carbon atoms and cycloalkyl having 3 to 6 carbon atoms, or
R¹ represents cycloalkyl having 3 to 6 carbon atoms which may be mono- to trisubstituted by identical or different substituents from the group consisting of halogen and alkyl having 1 to 4 carbon atoms, or
R¹ represents saturated or unsaturated heterocyclyl having 5 or 6 ring members and 1 to 3 heteroatoms, such as nitrogen, oxygen and/or sulfur, where the heterocyclyl may be mono- or disubstituted by halogen, alkyl having 1 to 4 carbon atoms, cyano, nitro and/or cycloalkyl having 3 to 6 carbon atoms,
R² represents hydrogen or alkyl having 1 to 4 carbon atoms, or
R¹ and R² together with the nitrogen atom to which they are attached represent a saturated or unsaturated heterocyclic ring having 3 to 6 ring members, where the heterocycle may contain a further nitrogen, oxygen or sulfur atom as ring member and where the heterocycle may be substituted up to three times by fluorine, chlorine, bromine, alkyl having 1 to 4 carbon atoms and/or haloalkyl having 1 to 4 carbon atoms and 1 to 9 fluorine and/or chlorine atoms,
R³ represents hydrogen, fluorine, chlorine, bromine, iodine, alkyl having 1 to 4 carbon atoms, haloalkyl having 1 to 4 carbon atoms and 1 to 9 halogen atoms or represents cycloalkyl having 3 to 6 carbon atoms,
R⁴ represents hydrogen, alkyl having 1 to 4 carbon atoms, haloalkyl having 1 to 4 carbon atoms in the alkyl moiety, cycloalkyl having 3 to 6 carbon atoms, alkoxyalkyl having 1 or 2 carbon atoms in the alkoxy moiety and 1 to 4 carbon atoms in the alkyl moiety, alkenyl having 2 to 5 carbon atoms, alkynyl having 2 to 5 carbon atoms or benzyl,
R⁵ represents hydrogen, alkyl having 1 to 4 carbon atoms, haloalkyl having 1 to 4 carbon atoms in the alkyl moiety, cycloalkyl having 3 to 6 carbon atoms, alkoxyalkyl having 1 or 2 carbon atoms in the alkoxy moiety and 1 to 4 carbon atoms in the alkyl moiety, alkenyl having 2 to 5 carbon atoms, alkynyl having 2 to 5 carbon atoms or benzyl,
R⁶ represents hydrogen, alkyl having 1 to 4 carbon atoms, alkoxyalkyl having 1 to 2 carbon atoms in the alkoxy moiety and 1 to 4 carbon atoms in the alkyl moiety, alkenyl having 2 to 5 carbon atoms, alkynyl having 2 to 5 carbon atoms or benzyl, or
R⁵ and -OR⁶ together represent a radical of the formula 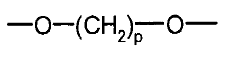 in which
p represents 2, 3 or 4 and
1 or 2 hydrogen atoms may be replaced by methyl, ethyl, hydroxy, methoxy, ethoxy, hydroxymethyl, methoxymethyl or ethoxymethyl,
R⁷ represents fluorine, chlorine, bromine, CN, methyl, alkoxy having 1 to 4 carbon atoms, alkylthio having 1 to 4 carbon atoms, alkylsulfinyl having 1 to 4 carbon atoms or alkylsulfonyl having 1 to 4 carbon atoms, and
R⁸ represents phenyl which may be mono- to tetrasubstituted by identical or different substituents from the group consisting of halogen, cyclo, nitro, amino, hydroxy, formyl, carboxy, carbamoyl, thiocarbamoyl;
in each case straight-chain or branched alkyl, alkoxy, alkylthio, alkylsulfinyl or alkylsulfonyl having in each case 1 to 6 carbon atoms;
in each case straight-chain or branched alkenyl or alkenyl having in each case 2 to 6 carbon atoms;
in each case straight-chain or branched haloalkyl, haloalkoxy, haloalkylthio, haloalkylsulfinyl or haloalkylsulfonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or alkoximinoalkyl having in each case 1 to 6 carbon atoms in the individual alkyl moieties;
cycloalkyl having 3 to 6 carbon atoms,
2,3-attached 1,3-propanediyl, 1,4-butanediyl, methylenedioxy (-O-CH₂-O-) or 1,2-ethylenedioxy (-O-CH₂-CH₂-O-), where these radicals may be mono- to polysubstituted by identical or different substituents from the group consisting of halogen, alkyl having 1 to 4 carbon atoms and haloalkyl having 1 to 4 carbon atoms and 1 to 9 identical or different halogen atoms.

2. Pyrazolopyrimidines of the formula (I) according to Claim 1, where
R¹ represents a radical of the formula 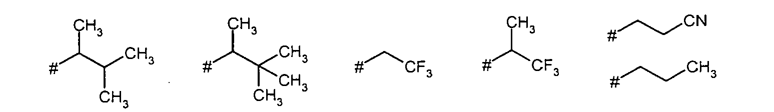 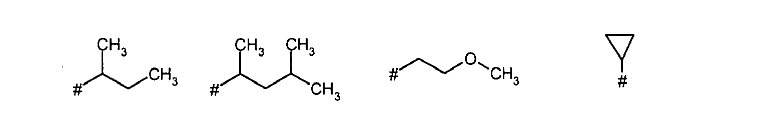 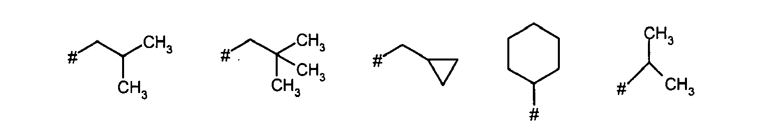 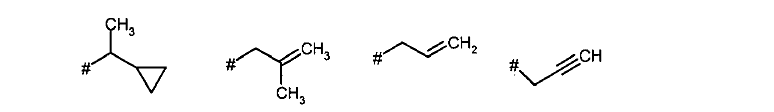 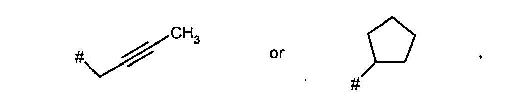 where # denotes the point of attachment and where in the case of radicals which may be present in optically active form each of the possible stereoisomers or else mixtures thereof may be present,
R² represents hydrogen, methyl, ethyl or propyl, or
R¹ and R² together with the nitrogen atom to which they are attached represent pyrrolidinyl, piperidinyl, morpholinyl, thiomorpholinyl, piperazinyl, 3,6-dihydro-1(2H)-piperidinyl or tetrahydro-1(2H)-pyridazinyl, where these radicals may be substituted by 1 to 3 fluorine atoms, 1 to 3 methyl groups and/or trifluoromethyl,
or
R¹ and R² together with the nitrogen atom to which they are attached represent a radical of the formula 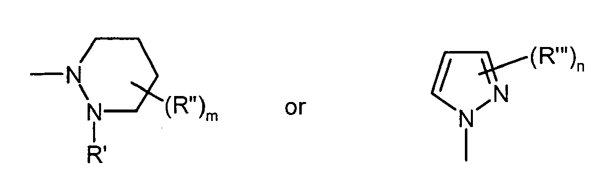 in which
R' represents hydrogen or methyl,
R" represents methyl, ethyl, fluorine, chlorine or trifluoromethyl,
m represents the number 0, 1, 2 or 3, where R" represents identical or different radicals if m represents 2 or 3,
R"' represents methyl, ethyl, fluorine, chlorine or trifluoromethyl and
n represents the number 0, 1, 2 or 3, where R"' represents identical or different radicals ifn represents 2 or 3,
R³ represents hydrogen, fluorine, chlorine, bromine, iodine, methyl, ethyl, isopropyl, cyclopropyl, cyclobutyl, cyclopentyl, cyclohexyl, trifluoromethyl, 1-trifluorornethyl-2,2,2-trifluoroethyl or heptafluoroisopropyl,
R⁴ represents hydrogen, methyl, ethyl, propyl, methoxymethyl, methoxyethyl, alkenyl having 3 or 4 carbon atoms, alkynyl having 3 or 4 carbon atoms or benzyl,
R⁵ represents hydrogen, methyl, ethyl, propyl, methoxymethyl, methoxyethyl, alkenyl having 3 or 4 carbon atoms, alkynyl having 3 or 4 carbon atoms or benzyl,
R⁶ represents hydrogen, methyl, ethyl, propyl, methoxymethyl, methoxyethyl, alkenyl having 3 or 4 carbon atoms, alkynyl having 3 or 4 carbon atoms or benzyl, or
R5 and -OR⁶ together represent a radical of the formula 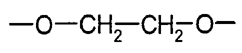 in which 1 or 2 hydrogen atoms may be replaced by methyl, ethyl, hydroxy, methoxy, ethoxy, hydroxymethyl, methoxymethyl or ethoxymethyl,
R⁷ represents fluorine, chlorine, bromine, methoxy, ethoxy, methylthio, methylsulfinyl or methylsulfonyl, and
R⁸ represents phenyl which may be mono- to trisubstituted by identical or different substituents from the group consisting of
fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulfinyl, ethylsulfinyl, methylsulfonyl, ethylsulfonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl, trifluoromethylsulfonyl, trichloroethynyloxy, trifluoroethynyloxy, chloroallyloxy, iodopropargyloxy, methylamino, ethylamino, n- or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl,
2,3-attached 1,3-propanediyl, methylenedioxy (-O-CH₂-O-) or 1,2-ethylenedioxy (O-CH₂-CH₂-O), where these radicals may be mono- or polysubstituted by identical or different substituents from the group consisting of fluorine, chlorine, methyl, ethyl, n-propyl, i-propyl and trifluoromethyl.

3. Pyrazolopyrimidines of the formula (I) according to one or more of Claims 1 or 2, where
R⁷ represents fluorine, chlorine, bromine, CN, methyl, methoxy or methylthio and
R⁸ represents 2,4-, 2,5- or 2,6-disubstituted phenyl or 2-substituted phenyl or represents 2,4,6-trisubstituted phenyl, where the substituents are selected from the group consisting of
fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulfinyl, ethylsulfinyl, methylsulfonyl, ethylsulfonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl, trifluoromethylsulfonyl, trichloroethynyloxy, trifluoroethynyloxy, chloroallyloxy, iodopropargyloxy, methylamino, ethylamino, n- or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl,
2,3-attached 1,3-propanediyl, methylenedioxy (-O-CH₂-O-) or 1,2-ethylenedioxy (O-CH₂-CH₂-O), where these radicals may be mono- or polysubstituted by identical or different substituents from the group consisting of fluorine, chlorine, methyl, ethyl, n-propyl, i-propyl and trifluoromethyl.

4. Process for preparing pyrazolopyrimidines of the formula (I) according to one or more of Claims 1 to 3, **characterized in that**
a) pyrazolopyrimidines of the formula 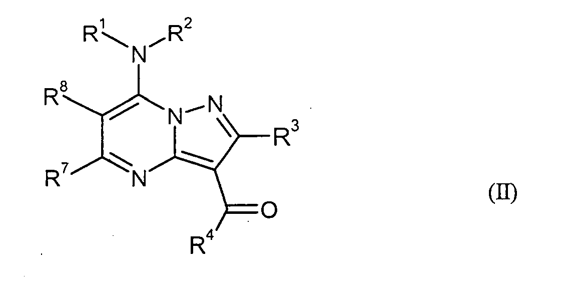 in which
R¹, R², R³, R⁴, R⁷ and R⁸ are as defined above
are either
α) reacted with diisobutylaluminum hydride in the presence of aqueous ammonium chloride solution and in the presence of an organic diluent,
or reacted with sodium borohydride in the presence of a diluent,
or
β) reacted with Grignard compounds of the formula
R⁹-Mg-X (III)
in which
R⁹ represents alkyl, alkoxyalkyl, alkenyl, alkynyl or benzyl and
X represents chlorine, bromine or iodine,
in the presence of a catalyst and in the presence of a diluent,
and the pyrazolopyrimidines, obtained according to variant (α) or (β), of the formula 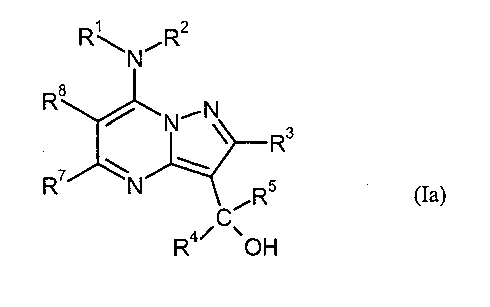
in which
R¹, R², R³, R⁴, R⁵, R⁷ and R⁸ are as defined above
are, if appropriate, reacted with compounds of the formula
R¹⁰-X¹ (IV)
in which
R¹⁰ represents alkyl, alkoxyalkyl, alkenyl, alkynyl or benzyl and
X¹ represents chlorine, bromine, iodine or the radical R¹⁰O-SO₂-O-,
if appropriate in the presence of a base and if appropriate in the presence of a diluent,
or
b) pyrazolopyrimidines of the formula 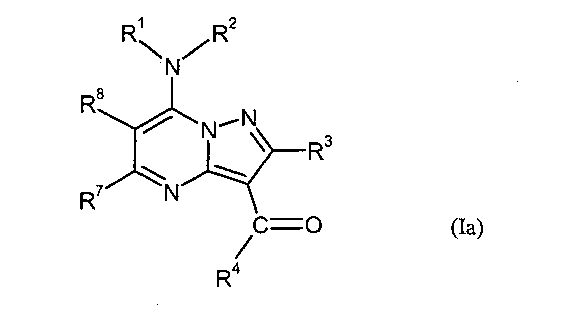
in which
R¹, R², R³, R⁴, R⁷ and R⁸ are as defined above,
are reacted with diols of the formula
HO-(CH₂)ₚ-O (V)
in which
p represents integers from 1 to 5 and
1 to 3 hydrogen atoms may be replaced by methyl, ethyl, hydroxy, methoxy, ethoxy, hydroxymethyl, methoxymethyl or ethoxymethyl,
in the presence of a catalyst and, if appropriate, in the presence of a diluent.

5. Composition for controlling unwanted microorganisms, **characterized in that** it comprises at least one pyrazolopyrimidine of the formula (I) according to one or more of Claims 1 to 3, in addition to extenders and/or surfactants.

6. Composition according to Claim 5, comprising at least one further fungicidally or insecticidally active component.

7. Use of pyrazolopyrimidines of the formula (I) according to one or more of Claims 1 to 3 for controlling unwanted microorganisms in the protection of plants and materials.

8. Method for controlling unwanted microorganisms in the protection of plants and materials, **characterized in that** pyrazolopyrimidines of the formula (I) according to one or more of Claims 1 to 3 are applied to the unwanted microorganisms and/or their habitats.

9. Process for preparing compositions for controlling unwanted microorganisms, **characterized in that** pyrazolopyrimidines of the formula (I) according to one or more of Claims 1 to 3 are mixed with extenders and/or surfactants.

## Revendications

1. Pyrazolopyrimidines de formule 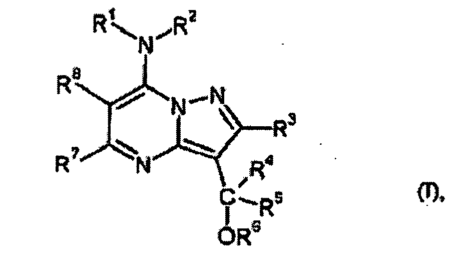 dans laquelle
R¹ représente un reste alkyle ayant 1 à 6 atomes de carbone, qui peut être substitué une à cinq fois identiques ou différentes par un radical halogéno, cyano, hydroxy, alkoxy ayant 1 à 4 atomes de carbone et/ou cycloalkyle ayant 3 à 6 atomes de carbone, ou bien
R¹ représente un reste alcényle ayant 2 à 6 atomes de carbone, qui peut être substitué une à trois fois identiques ou différentes par un radical halogéno, cyano, hydroxy, alkoxy ayant 1 à 4 atomes de carbone et/ou cycloalkyle ayant 3 à 6 atomes de carbone, ou bien
R¹ représente un reste alcynyle ayant 3 à 6 atomes de carbone, qui peut être substitué une à trois fois identiques ou différentes par un radical halogéno, cyano, alkoxy ayant 1 à 4 atomes de carbone et/ou cycloalkyle ayant 3 à 6 atomes de carbone, ou bien
R¹ représente un reste cycloalkyle ayant 3 à 6 atomes de carbone, qui peut être substitué une à trois fois identiques ou différentes par un radical halogéno et/ou alkyle ayant 1 à 4 atomes de carbone, ou bien
R¹ représente un reste hétérocyclyle saturé ou non saturé à noyau de 5 ou 6 chaînons et ayant 1 à 3 hétéroatomes tels qu'azote, oxygène et/ou soufre, le reste hétérocyclyle pouvant être substitué une ou deux fois par un radical halogéno, alkyle ayant 1 à 4 atomes de carbone, cyano, nitro et/ou cycloalkyle ayant 3 à 6 atomes de carbone,
R² représente l'hydrogène ou un reste alkyle ayant. 1. à 4 atomes de carbone, où bien
R¹ et R² forment, conjointement avec l'atome d'azote auquel ils sont liés, un noyau hétérocyclique saturé ou non saturé ayant 3 à 6 chaînons, l'hétérocycle pouvant contenir comme chaînon un autre atome d'azote, d'oxygène ou de soufre et l'hétérocycle pouvant être substitué jusqu'à trois fois par un radical fluoro, chloro, bromo, alkyle ayant 1 à 4 atomes de carbone et/ou halogénalkyle ayant 1 à 4 atomes de carbone et 1 à 9 atomes de fluor et/ou de chlore,
R³ représente l'hydrogène, le fluor, le chlore, le brome, l'iode, un reste alkyle ayant 1 à 4 atomes de carbone, halogénalkyle ayant 1 à 4 atomes de carbone et 1 à 9 atomes d'halogène ou un reste cycloalkyle ayant 3 à 6 atomes de carbone,
R⁴ représente l'hydrogène, un reste alkyle ayant 1 à 4 atomes de carbone, halogénalkyle ayant 1 à 4 atomes de carbone dans la partie alkyle, cycloalkyle ayant 3 à 6 atomes de carbone, alkoxyalkyle ayant 1 ou 2 atomes de carbone dans la partie alkoxy et 1 à 4 atomes de carbone dans la partie alkyle, alcényle ayant 2 à 5 atomes de carbone, alcynyle ayant 2 à 5 atomes de carbone, ou benzyle,
R⁵ représente l'hydrogène, un reste alkyle ayant 1 à 4 atomes de carbone, halogénalkyle ayant 1 à 4 atomes de carbone dans la partie alkyle, cycloalkyle ayant 3 à 6 atomes de carbone, alkoxyalkyle ayant 1 ou 2 atomes de carbone dans la partie alkoxy et 1 à 4 atomes de carbone dans la partie alkyle, alcényle ayant 2 à 5 atomes de carbone, alcynyle ayant 2 à 5 atomes de carbone, ou benzyle,
R⁶ représente l'hydrogène, un reste alkyle ayant 1 à 4 atomes de carbone, alkoxyalkyle ayant 1 ou 2 atomes de carbone dans la partie alkoxy et 1 à 4 atomes de carbone dans la partie alkyle, alcényle ayant 2 à 5 atomes de carbone, alcynyle ayant 2 à 5 atomes de carbone, ou benzyle, ou bien
R⁵ et -OR⁶ forment ensemble un reste de formule 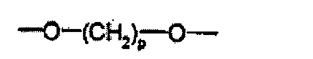 dans laquelle
p a la valeur 2, 3 ou 4 et
1 ou 2 atomes d'hydrogène peuvent être remplacés par un radical méthyle, éthyle, hydroxy, méthoxy, éthoxy, hydroxyméthyle, méthoxyméthyle ou éthoxyméthyle,
R⁷ représente le fluor, le chlore, le brome, un groupe CN, un reste méthyle, alkoxy ayant 1 à 4 atomes de carbone, alkylthio ayant 1 à 4 atomes de carbone, alkylsulfinyle ayant 1 à 4 atomes de carbone ou alkylsulfonyle ayant 1 à 4 atomes de carbone, et
R8 représente un reste phényle qui peut être substitué une à quatre fois identiques ou différentes par un radical halogéno, cyclo, nitro, amino, hydroxy, formyle, carboxy, carbamoyle, thiocarbamoyle;
un reste alkyle, alkoxy, alkylthio, alkylsulfinyle ou alkylsulfonyle, chacun linéaire ou ramifié, ayant chacun 1 à 6 atomes de carbone;
un reste alcényle ou alcényle, chacun linéaire ou ramifié, ayant chacun 2 à 6 atomes de carbone ;
un reste halogénalkyle, halogénalkoxy, halogénalkylthio, halogénalkylsulfinyle ou halogénalkylsulfonyle, chacun linéaire ou ramifié, ayant chacun 1 à 6 atomes de carbone et 1 à 13 atomes d'halogènes identiques ou différents ;
un reste halogénalcényle ou halogénalcényloxy, chacun linéaire ou ramifié, ayant chacun 2 à 6 atomes de carbone et 1 à 11 atomes d'halogènes identiques ou différents ;
un reste' alkylamino, dialkylamino, alkylcarbonyle, alkylcarbonyloxy, alkoxycarbonyle, alkylsulfonyloxy, hydroximinoalkyle ou alkoximinoalkyle, chacun linéaire ou ramifié, ayant chacun 1 à 6 atomes de carbone dans les parties alkyle individuelles ;
un reste cycloalkyle ayant 3 à 6 atomes de carbone,
un reste 1,3-propanediyle lié en positions 2,3, 1,4-butane-diyle, méthylènedioxy (-O-CH₂-O-) ou 1,2-éthylènedioxy (-O-CH-CH₂-O-), ces restes pouvant être substitués une à plusieurs fois identiques ou différentes par un radical halogéno, alkyle ayant 1 à 4 atomes de carbone et/ou halogénalkyle ayant 1 à 4 atomes de carbone et 1 à 9 atomes d'halogènes identiques ou différents.

2. Pyrazolopyrimidines de formule (I) suivant la revendication 1, formule dans laquelle
R1 représente un reste de formule 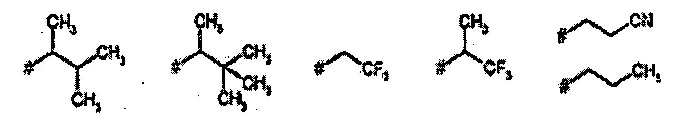 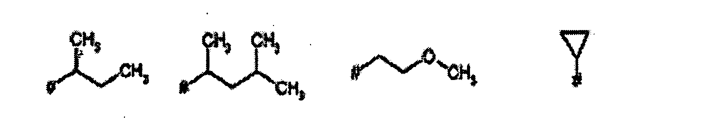 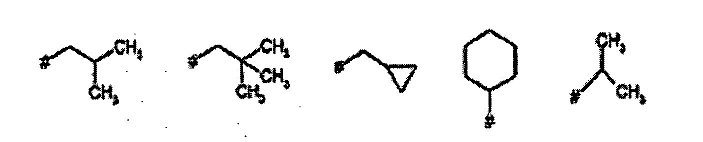 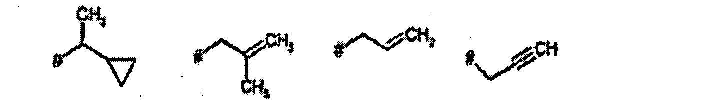 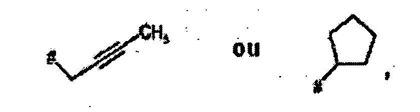 où le signe # indique la position de liaisons et, pour les restes qui peuvent se présenter sous une forme optiquement active, il peut exister chacun des stéréoisomères possibles ou aussi leurs mélanges,
R² représente l'hydrogène, un reste méthyle, éthyle ou propyle, ou bien
R¹ et R² forment, conjointement avec l'atome d'azote auquel ils sont liés, un reste pyrrolidinyle, pipéridinyle,: morpholinyle, thiomorpholinyle, pipérazinyle, 3,6-dihydro-1(2H)-pipéridinyle ou tétrahydro-1(2H)-pyridazinyle, ces restes pouvant être substitués par 1 à 3 atomes de fluor, 1 à 3 groupes méthyle et/ou un groupe trifluorométhyle,
ou bien
R¹ et R² forment, conjointement avec l'atome d'azote auquel ils sont liés, un reste de formule 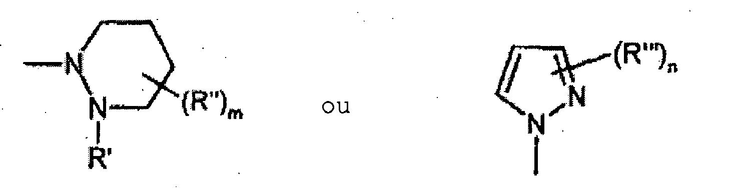 dans laquelle
R' représente l'hydrogène ou un reste méthyle,
R" représente un reste méthyle, éthyle, le fluor, le chlore ou un reste trifluorométhyle,
m représente les nombres 0, 1, 2 ou 3, R" représentant des restes identiques ou différents lorsque m a la valeur 2 ou 3,
R'" désigne un reste méthyle, éthyle, le fluor, le chlore ou un reste trifluorométhyle
et
n représente les nombres 0, 1, 2 ou 3, R'" représentant des restes identiques ou différents lorsque n a la valeur 2 ou 3,
R³ représente l'hydrogène, le fluor, le chlore, le brome, l'iode, un reste méthyle, éthyle, isopropyle, cyclopropyle, cyclobutyle, cyclopentyle, cyclohexyle, trifluorométhyle, 1-trifluorométhyl-2,2,2-trifluoréthyle ou heptafluorisopropyle,
R⁴ représente l'hydrogène, un reste méthyle, éthyle, propyle, méthoxyméthyle, méthoxyéthyle, alcényle ayant 3 ou 4 atomes de carbone, alcynyle ayant 3 ou 4 atomes de carbone, ou benzyle,
R⁵ représente l'hydrogène, un reste méthyle, éthyle, propyle, méthoxyméthyle, méthoxyéthyle, alcényle ayant 3 ou 4 atomes de carbone, alcynyle ayant 3 ou 4 atomes de carbone, ou benzyle,
R⁶ représente l'hydrogène, un reste méthyle, éthyle, propyle, méthoxyméthyle, méthoxyéthyle, alcényle ayant 3 ou 4 atomes de carbone, alcynyle ayant 3 ou 4 atomes de carbone, ou benzyle,
ou bien
R⁵ et -OR⁶ forment ensemble un reste de formule -O-CH₂=CH₂-O-, où 1 ou 2 atomes d'hydrogène peuvent être remplacés par un radical méthyle, éthyle, hydroxy, méthoxy, éthoxy, hydroxyméthyle, méthoxyméthyle ou éthoxyméthyle,
R⁷ représente le fluor, le chlore, le brome, un reste méthoxy, éthoxy, méthylthio, méthylsulfinyle ou méthylsulfonyle, et
R⁸ représente un reste phényle, qui peut être substitué une à trois fois identiques ou différentes par un radical
fluoro, chloro, bromo, cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, trichloréthynyloxy, trifluoréthynyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
1,3-propanediyle lié en positions 2,3, méthylènedioxy (-O-CH₂-O-) ou 1, 2-éthylènédioxy (O-CH₂-CH₂-O), ces restes pouvant être substitués une ou plusieurs fois
identiques ou différentes par un radical fluoro, chloro, méthyle, éthyle, n-propyle, isopropyle et/ou trifluorométhyle.

3. Pyrazolopyrimidines de formule (I) suivant l'une ou plusieurs des revendications 1 et 2, formule dans laquelle
R⁷ représente le fluor, le chlore, le brome, un groupe CN, un reste méthyle, méthoxy ou méthylthio et
R⁸ représente un reste phényle disubstitué en positions 2,4, 2,5 ou 2,6 ou un reste phényle substitué en position 2 ou un reste phényle trisubstitué en positions 2,4,6, les substituants étant choisis dans le groupe des substituants
fluoro, chloro, bromo, cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonylé, trichloréthynyloxy, trifluoréthynyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonylé, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
1,3-propanediyle lié en positions 2,3, méthylènedioxy (-O-CH₂-O-) et 1,2-éthylènedioxy (O-CH₂-CH₂-O), ces restes pouvant être substitués une ou plusieurs fois identiques ou différentes par un radical fluoro,
chloro, méthyle, éthyle, n-propyle, isopropyle et/ou trifluorométhyle.

4. Procédé de production de pyrazolopyrimidines de formule (I) suivant une ou plusieurs des revendications 1 à 3, **caractérisé en ce que**
a) on fait réagir des pyrazolopyrimidines de formule 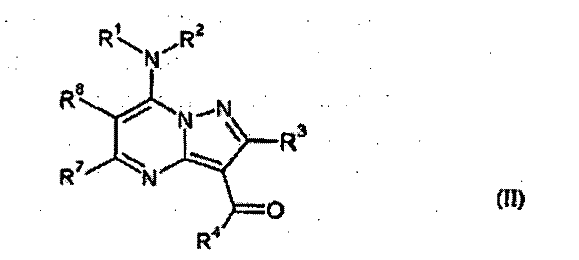 dans laquelle
R¹, R², R³, R⁴, R⁷ et R⁸ ont les définitions indiquées ci-dessus,
soit
α) avec l'hydrure de diisobutylaluminium en présence d'une solution aqueuse de chlorure d'ammonium ainsi qu'en présence d'un diluant organique, ou bien avec le borohydrure de sodium en présence d'un diluant
soit
β) avec des composés de Grignard de formule
R⁹-Mg-X (III)
dans laquelle
R⁹ représente un reste alkyle, alkoxyalkyle, alcényle, alcynyle ou benzyle et
X représente le chlore, le brome ou l'iode,
en présence d'un catalyseur et en présence d'un diluant, et le cas échéant, on fait réagir les pyrazolopyrimidines obtenues selon la variante (α) ou (β), de formule 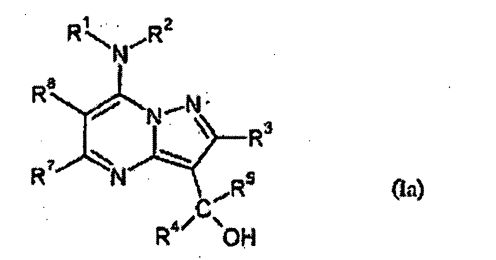
dans laquelle
R¹, R², R³, R⁴, R⁵, R⁷ et R⁸ ont les définitions indiquées ci-dessus,
avec des composés de formule
R¹⁰-X¹ (IV)
dans laquelle
R¹⁰ est un reste alkyle, alkoxyalkyle, alcényle, alcynyle ou benzyle, et
X¹ représente le chlore, le brome, l'iode ou le reste R¹⁰ O-SO₂-O-,
le cas échéant en présence d'une base et, éventuellement, en présence d'un diluant,
ou bien
b) on fait réagir des pyrazolopyrimidines de formule 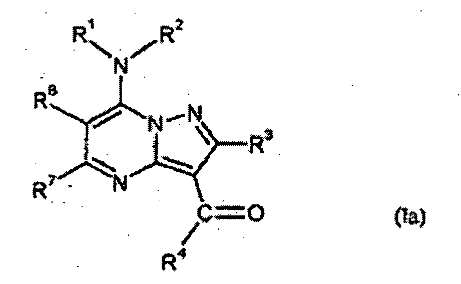
dans laquelle
R¹, R², R³, R⁴, R⁷ et R⁸ ont les définitions indiquées ci-dessus,
avec des diols de formule
HO- (CH₂)ₚ-O (V)
dans laquelle
p représente des nombres entiers de 1 à 5, et 1 à 3 atomes d'hydrogène peuvent être remplacés par un radical méthyle, éthyle, hydroxy, méthoxy, éthoxy, hydroxyméthyle, méthoxyméthyle ou éthoxyméthyle,
en présence d'un catalyseur et, le cas échéant, en présence d'un diluant

5. Composition destinée à combattre des micro-organismes indésirables, **caractérisée par** une teneur en au moins une pyrazolopyrimidine de formule (I) suivant l'une ou plusieurs des revendications 1 à 3, en plus de diluants et/ou d'agents tensioactifs.

6. Composition suivant la revendication 5, contenant au moins une autre substance active fongicide ou insecticide.

7. Utilisation de pyrazolopyrimidines de formule (I) suivant l'une ou plusieurs des revendications 1 à 3 pour combattre des micro-organismes indésirables dans la protection des plantes et des matériaux.

8. Procédé de lutte contre des micro-organismes indésirables dans la protection de plantes et de matériaux, **caractérisé en qu'**on épand des pyrazolopyrimidines de formule (I) suivant l'une ou plusieurs des revendications 1 à 3 sur les micro-organismes indésirables et/ou sur leur milieu.

9. Procédé de préparation de compositions destinées à combattre des micro-organismes indésirables, **caractérisé en ce qu'**on mélange des pyrazolopyrimidines de formule (I) suivant l'une ou plusieurs des revendications 1 à 3 avec des diluants et/ou des agents tensioactifs.
